(19) Europäisches Patentamt
European Patent Office
Office européen des brevets

(11) EP 3 766 968 A1

(12) EUROPEAN PATENT APPLICATION

(43) Date of publication:
**20.01.2021 Bulletin 2021/03**

(51) Int Cl.:
***C12N 9/22*** *(2006.01)* ***C07K 14/005*** *(2006.01)*
***C12N 15/63*** *(2006.01)* ***A61K 38/46*** *(2006.01)*

(21) Application number: **19186590.6**

(22) Date of filing: **16.07.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Deutsches Krebsforschungszentrum 69120 Heidelberg (DE)**
- **Universität Heidelberg 69117 Heidelberg (DE)**
- **Berliner Institut für Gesundheitsforschung Institute of Health - BIH 10117 Berlin (DE)**

(72) Inventors:
- **NIOPEK, Dominik 69121 Heidelberg (DE)**
- **ASCHENBRENNER, Sabine 69120 Heidelberg (DE)**
- **HOFFMANN, Mareike 69120 Heidelberg (DE)**
- **KALLENBERGER, Stefan 69120 Heidelberg (DE)**
- **EILS, Roland 12555 Berlin (DE)**
- **HUCK, Adrian 77723 Gengenbach (DE)**

(74) Representative: **Altmann Stößel Dick Patentanwälte PartG mbB Dudenstrasse 46 68167 Mannheim (DE)**

(54) **IMPROVING CAS NUCLEASE TARGET SPECIFICITY**

(57) The present invention relates to a method for modifying a target site in a host cell comprising contacting said host cell with a Cas nuclease, wherein said host cell is further contacted with a low-affinity Cas inhibitor and/or a sub-inhibitory concentration of a Cas inhibitor; and to a method for improving specificity of a Cas nuclease, comprising a) providing a Cas nuclease; and b) contacting said Cas nuclease with a low-affinity Cas inhibitor or a sub-inhibitory concentration of a Cas inhibitor; and c) thereby improving specificity of said Cas enzyme. Further, the present invention relates to compositions, polypeptides, uses and methods related thereto.

EP 3 766 968 A1

SEQUENCE LISTING

<110> Deutsches Krebsforschungszentrum
Universität Heidelberg
Berliner Institut für Gesundheitsforschung Institute of Health \226
BIH

<120> Improving Cas Nuclease Target Specificity

<130> DK15992EP

<160> 65

<170> PatentIn version 3.5

<210> 1
<211> 1367
<212> PRT
<213> Artificial Sequence

<220>
<223> Cas9

<400> 1

```
Asp Lys Lys Tyr Ser Ile Gly Leu Asp Ile Gly Thr Asn Ser Val Gly
1               5                   10                  15

Trp Ala Val Ile Thr Asp Glu Tyr Lys Val Pro Ser Lys Lys Phe Lys
            20                  25                  30

Val Leu Gly Asn Thr Asp Arg His Ser Ile Lys Lys Asn Leu Ile Gly
        35                  40                  45

Ala Leu Leu Phe Asp Ser Gly Glu Thr Ala Glu Ala Thr Arg Leu Lys
    50                  55                  60

Arg Thr Ala Arg Arg Arg Tyr Thr Arg Arg Lys Asn Arg Ile Cys Tyr
65                  70                  75                  80

Leu Gln Glu Ile Phe Ser Asn Glu Met Ala Lys Val Asp Asp Ser Phe
                85                  90                  95

Phe His Arg Leu Glu Glu Ser Phe Leu Val Glu Glu Asp Lys Lys His
            100                 105                 110

Glu Arg His Pro Ile Phe Gly Asn Ile Val Asp Glu Val Ala Tyr His
        115                 120                 125

Glu Lys Tyr Pro Thr Ile Tyr His Leu Arg Lys Lys Leu Val Asp Ser
    130                 135                 140

Thr Asp Lys Ala Asp Leu Arg Leu Ile Tyr Leu Ala Leu Ala His Met
145                 150                 155                 160
```

```
Ile Lys Phe Arg Gly His Phe Leu Ile Glu Gly Asp Leu Asn Pro Asp
                165                 170                 175

Asn Ser Asp Val Asp Lys Leu Phe Ile Gln Leu Val Gln Thr Tyr Asn
            180                 185                 190

Gln Leu Phe Glu Glu Asn Pro Ile Asn Ala Ser Gly Val Asp Ala Lys
        195                 200                 205

Ala Ile Leu Ser Ala Arg Leu Ser Lys Ser Arg Arg Leu Glu Asn Leu
    210                 215                 220

Ile Ala Gln Leu Pro Gly Glu Lys Lys Asn Gly Leu Phe Gly Asn Leu
225                 230                 235                 240

Ile Ala Leu Ser Leu Gly Leu Thr Pro Asn Phe Lys Ser Asn Phe Asp
                245                 250                 255

Leu Ala Glu Asp Ala Lys Leu Gln Leu Ser Lys Asp Thr Tyr Asp Asp
            260                 265                 270

Asp Leu Asp Asn Leu Leu Ala Gln Ile Gly Asp Gln Tyr Ala Asp Leu
        275                 280                 285

Phe Leu Ala Ala Lys Asn Leu Ser Asp Ala Ile Leu Leu Ser Asp Ile
    290                 295                 300

Leu Arg Val Asn Thr Glu Ile Thr Lys Ala Pro Leu Ser Ala Ser Met
305                 310                 315                 320

Ile Lys Arg Tyr Asp Glu His His Gln Asp Leu Thr Leu Leu Lys Ala
                325                 330                 335

Leu Val Arg Gln Gln Leu Pro Glu Lys Tyr Lys Glu Ile Phe Phe Asp
            340                 345                 350

Gln Ser Lys Asn Gly Tyr Ala Gly Tyr Ile Asp Gly Gly Ala Ser Gln
        355                 360                 365

Glu Glu Phe Tyr Lys Phe Ile Lys Pro Ile Leu Glu Lys Met Asp Gly
    370                 375                 380

Thr Glu Glu Leu Leu Val Lys Leu Asn Arg Glu Asp Leu Leu Arg Lys
385                 390                 395                 400

Gln Arg Thr Phe Asp Asn Gly Ser Ile Pro His Gln Ile His Leu Gly
```

405 410 415

Glu Leu His Ala Ile Leu Arg Arg Gln Glu Asp Phe Tyr Pro Phe Leu
420 425 430

Lys Asp Asn Arg Glu Lys Ile Glu Lys Ile Leu Thr Phe Arg Ile Pro
435 440 445

Tyr Tyr Val Gly Pro Leu Ala Arg Gly Asn Ser Arg Phe Ala Trp Met
450 455 460

Thr Arg Lys Ser Glu Glu Thr Ile Thr Pro Trp Asn Phe Glu Glu Val
465 470 475 480

Val Asp Lys Gly Ala Ser Ala Gln Ser Phe Ile Glu Arg Met Thr Asn
485 490 495

Phe Asp Lys Asn Leu Pro Asn Glu Lys Val Leu Pro Lys His Ser Leu
500 505 510

Leu Tyr Glu Tyr Phe Thr Val Tyr Asn Glu Leu Thr Lys Val Lys Tyr
515 520 525

Val Thr Glu Gly Met Arg Lys Pro Ala Phe Leu Ser Gly Glu Gln Lys
530 535 540

Lys Ala Ile Val Asp Leu Leu Phe Lys Thr Asn Arg Lys Val Thr Val
545 550 555 560

Lys Gln Leu Lys Glu Asp Tyr Phe Lys Lys Ile Glu Cys Phe Asp Ser
565 570 575

Val Glu Ile Ser Gly Val Glu Asp Arg Phe Asn Ala Ser Leu Gly Thr
580 585 590

Tyr His Asp Leu Leu Lys Ile Ile Lys Asp Lys Asp Phe Leu Asp Asn
595 600 605

Glu Glu Asn Glu Asp Ile Leu Glu Asp Ile Val Leu Thr Leu Thr Leu
610 615 620

Phe Glu Asp Arg Glu Met Ile Glu Glu Arg Leu Lys Thr Tyr Ala His
625 630 635 640

Leu Phe Asp Asp Lys Val Met Lys Gln Leu Lys Arg Arg Arg Tyr Thr
645 650 655

```
Gly Trp Gly Arg Leu Ser Arg Lys Leu Ile Asn Gly Ile Arg Asp Lys
            660                 665                 670

Gln Ser Gly Lys Thr Ile Leu Asp Phe Leu Lys Ser Asp Gly Phe Ala
        675                 680                 685

Asn Arg Asn Phe Met Gln Leu Ile His Asp Asp Ser Leu Thr Phe Lys
    690                 695                 700

Glu Asp Ile Gln Lys Ala Gln Val Ser Gly Gln Gly Asp Ser Leu His
705                 710                 715                 720

Glu His Ile Ala Asn Leu Ala Gly Ser Pro Ala Ile Lys Lys Gly Ile
                725                 730                 735

Leu Gln Thr Val Lys Val Val Asp Glu Leu Val Lys Val Met Gly Arg
            740                 745                 750

His Lys Pro Glu Asn Ile Val Ile Glu Met Ala Arg Glu Asn Gln Thr
        755                 760                 765

Thr Gln Lys Gly Gln Lys Asn Ser Arg Glu Arg Met Lys Arg Ile Glu
    770                 775                 780

Glu Gly Ile Lys Glu Leu Gly Ser Gln Ile Leu Lys Glu His Pro Val
785                 790                 795                 800

Glu Asn Thr Gln Leu Gln Asn Glu Lys Leu Tyr Leu Tyr Tyr Leu Gln
                805                 810                 815

Asn Gly Arg Asp Met Tyr Val Asp Gln Glu Leu Asp Ile Asn Arg Leu
            820                 825                 830

Ser Asp Tyr Asp Val Asp His Ile Val Pro Gln Ser Phe Leu Lys Asp
        835                 840                 845

Asp Ser Ile Asp Asn Lys Val Leu Thr Arg Ser Asp Lys Asn Arg Gly
    850                 855                 860

Lys Ser Asp Asn Val Pro Ser Glu Glu Val Val Lys Lys Met Lys Asn
865                 870                 875                 880

Tyr Trp Arg Gln Leu Leu Asn Ala Lys Leu Ile Thr Gln Arg Lys Phe
                885                 890                 895

Asp Asn Leu Thr Lys Ala Glu Arg Gly Gly Leu Ser Glu Leu Asp Lys
            900                 905                 910
```

```
Ala Gly Phe Ile Lys Arg Gln Leu Val Glu Thr Arg Gln Ile Thr Lys
        915                 920                 925

His Val Ala Gln Ile Leu Asp Ser Arg Met Asn Thr Lys Tyr Asp Glu
    930                 935                 940

Asn Asp Lys Leu Ile Arg Glu Val Lys Val Ile Thr Leu Lys Ser Lys
945                 950                 955                 960

Leu Val Ser Asp Phe Arg Lys Asp Phe Gln Phe Tyr Lys Val Arg Glu
                965                 970                 975

Ile Asn Asn Tyr His His Ala His Asp Ala Tyr Leu Asn Ala Val Val
            980                 985                 990

Gly Thr Ala Leu Ile Lys Lys Tyr  Pro Lys Leu Glu Ser  Glu Phe Val
        995                 1000                1005

Tyr Gly  Asp Tyr Lys Val Tyr  Asp Val Arg Lys Met  Ile Ala Lys
    1010                 1015                 1020

Ser Glu  Gln Glu Ile Gly Lys  Ala Thr Ala Lys Tyr  Phe Phe Tyr
    1025                 1030                 1035

Ser Asn  Ile Met Asn Phe Phe  Lys Thr Glu Ile Thr  Leu Ala Asn
    1040                 1045                 1050

Gly Glu  Ile Arg Lys Arg Pro  Leu Ile Glu Thr Asn  Gly Glu Thr
    1055                 1060                 1065

Gly Glu  Ile Val Trp Asp Lys  Gly Arg Asp Phe Ala  Thr Val Arg
    1070                 1075                 1080

Lys Val  Leu Ser Met Pro Gln  Val Asn Ile Val Lys  Lys Thr Glu
    1085                 1090                 1095

Val Gln  Thr Gly Gly Phe Ser  Lys Glu Ser Ile Leu  Pro Lys Arg
    1100                 1105                 1110

Asn Ser  Asp Lys Leu Ile Ala  Arg Lys Lys Asp Trp  Asp Pro Lys
    1115                 1120                 1125

Lys Tyr  Gly Gly Phe Asp Ser  Pro Thr Val Ala Tyr  Ser Val Leu
    1130                 1135                 1140

Val Val  Ala Lys Val Glu Lys  Gly Lys Ser Lys Lys  Leu Lys Ser
    1145                 1150                 1155
```

```
Val Lys Glu Leu Leu Gly Ile Thr Ile Met Glu Arg Ser Ser Phe
    1160                1165                1170

Glu Lys Asn Pro Ile Asp Phe Leu Glu Ala Lys Gly Tyr Lys Glu
    1175                1180                1185

Val Lys Lys Asp Leu Ile Ile Lys Leu Pro Lys Tyr Ser Leu Phe
    1190                1195                1200

Glu Leu Glu Asn Gly Arg Lys Arg Met Leu Ala Ser Ala Gly Glu
    1205                1210                1215

Leu Gln Lys Gly Asn Glu Leu Ala Leu Pro Ser Lys Tyr Val Asn
    1220                1225                1230

Phe Leu Tyr Leu Ala Ser His Tyr Glu Lys Leu Lys Gly Ser Pro
    1235                1240                1245

Glu Asp Asn Glu Gln Lys Gln Leu Phe Val Glu Gln His Lys His
    1250                1255                1260

Tyr Leu Asp Glu Ile Ile Glu Gln Ile Ser Glu Phe Ser Lys Arg
    1265                1270                1275

Val Ile Leu Ala Asp Ala Asn Leu Asp Lys Val Leu Ser Ala Tyr
    1280                1285                1290

Asn Lys His Arg Asp Lys Pro Ile Arg Glu Gln Ala Glu Asn Ile
    1295                1300                1305

Ile His Leu Phe Thr Leu Thr Asn Leu Gly Ala Pro Ala Ala Phe
    1310                1315                1320

Lys Tyr Phe Asp Thr Thr Ile Asp Arg Lys Arg Tyr Thr Ser Thr
    1325                1330                1335

Lys Glu Val Leu Asp Ala Thr Leu Ile His Gln Ser Ile Thr Gly
    1340                1345                1350

Leu Tyr Glu Thr Arg Ile Asp Leu Ser Gln Leu Gly Gly Asp
    1355                1360                1365
```

<210> 2
<211> 4101
<212> DNA
<213> Artificial Sequence

<220>
<223> Cas9 encoding

<400> 2
gacaagaagt acagcatcgg cctggacatc ggcaccaact ctgtgggctg ggccgtgatc 60

accgacgagt acaaggtgcc cagcaagaaa ttcaaggtgc tgggcaacac cgaccggcac 120

agcatcaaga agaacctgat cggagccctg ctgttcgaca gcggcgaaac agccgaggcc 180

acccggctga agagaaccgc cagaagaaga tacaccagac ggaagaaccg gatctgctat 240

ctgcaagaga tcttcagcaa cgagatggcc aaggtggacg acagcttctt ccacagactg 300

gaagagtcct tcctggtgga agaggataag aagcacgagc ggcaccccat cttcggcaac 360

atcgtggacg aggtggccta ccacgagaag taccccacca tctaccacct gagaaagaaa 420

ctggtggaca gcaccgacaa ggccgacctg cggctgatct atctggccct ggcccacatg 480

atcaagttcc ggggccactt cctgatcgag ggcgacctga accccgacaa cagcgacgtg 540

gacaagctgt tcatccagct ggtgcagacc tacaaccagc tgttcgagga aaaccccatc 600

aacgccagcg gcgtggacgc caaggccatc ctgtctgcca gactgagcaa gagcagacgg 660

ctggaaaatc tgatcgccca gctgcccggc gagaagaaga atggcctgtt cggaaacctg 720

attgccctga gcctgggcct gacccccaac ttcaagagca acttcgacct ggccgaggat 780

gccaaactgc agctgagcaa ggacacctac gacgacgacc tggacaacct gctggcccag 840

atcggcgacc agtacgccga cctgtttctg gccgccaaga acctgtccga cgccatcctg 900

ctgagcgaca tcctgagagt gaacaccgag atcaccaagg ccccccctgag cgcctctatg 960

atcaagagat acgacgagca ccaccaggac ctgaccctgc tgaaagctct cgtgcggcag 1020

cagctgcctg agaagtacaa agagattttc ttcgaccaga gcaagaacgg ctacgccggc 1080

tacattgacg gcggagccag ccaggaagag ttctacaagt tcatcaagcc catcctggaa 1140

aagatggacg gcaccgagga actgctcgtg aagctgaaca gagaggacct gctgcggaag 1200

cagcggacct tcgacaacgg cagcatcccc caccagatcc acctgggaga gctgcacgcc 1260

attctgcggc ggcaggaaga tttttaccca ttcctgaagg acaaccggga aaagatcgag 1320

aagatcctga ccttccgcat cccctactac gtgggccctc tggccagggg aaacagcaga 1380

ttcgcctgga tgaccagaaa gagcgaggaa accatcaccc cctggaactt cgaggaagtg 1440

gtggacaagg gcgcttccgc ccagagcttc atcgagcgga tgaccaactt cgataagaac 1500

ctgcccaacg agaaggtgct gcccaagcac agcctgctgt acgagtactt caccgtgtat 1560

aacgagctga ccaaagtgaa atacgtgacc gagggaatga gaaagcccgc cttcctgagc 1620

ggcgagcaga aaaaggccat cgtggacctg ctgttcaaga ccaaccggaa agtgaccgtg 1680

aagcagctga aagaggacta cttcaagaaa atcgagtgct tcgactccgt ggaaatctcc 1740

ggcgtggaag atcggttcaa cgcctccctg ggcacatacc acgatctgct gaaaattatc 1800

aaggacaagg acttcctgga caatgaggaa aacgaggaca ttctggaaga tatcgtgctg 1860
accctgacac tgtttgagga cagagagatg atcgaggaac ggctgaaaac ctatgcccac 1920
ctgttcgacg acaaagtgat gaagcagctg aagcggcgga gatacaccgg ctggggcagg 1980
ctgagccgga agctgatcaa cggcatccgg gacaagcagt ccggcaagac aatcctggat 2040
ttcctgaagt ccgacggctt cgccaacaga aacttcatgc agctgatcca cgacgacagc 2100
ctgaccttta aagaggacat ccagaaagcc caggtgtccg gccagggcga tagcctgcac 2160
gagcacattg ccaatctggc cggcagcccc gccattaaga agggcatcct gcagacagtg 2220
aaggtggtgg acgagctcgt gaaagtgatg ggccggcaca agcccgagaa catcgtgatc 2280
gaaatggcca gagagaacca gaccacccag aagggacaga agaacagccg cgagagaatg 2340
aagcggatcg aagagggcat caaagagctg ggcagccaga tcctgaaaga acaccccgtg 2400
gaaaacaccc agctgcagaa cgagaagctg tacctgtact acctgcagaa tgggcgggat 2460
atgtacgtgg accaggaact ggacatcaac cggctgtccg actacgatgt ggaccatatc 2520
gtgcctcaga gctttctgaa ggacgactcc atcgacaaca aggtgctgac cagaagcgac 2580
aagaaccggg gcaagagcga caacgtgccc tccgaagagg tcgtgaagaa gatgaagaac 2640
tactggcggc agctgctgaa cgccaagctg attacccaga gaaagttcga caatctgacc 2700
aaggccgaga gaggcggcct gagcgaactg gataaggccg gcttcatcaa gagacagctg 2760
gtggaaaccc ggcagatcac aaagcacgtg gcacagatcc tggactcccg gatgaacact 2820
aagtacgacg agaatgacaa gctgatccgg gaagtgaaag tgatcaccct gaagtccaag 2880
ctggtgtccg atttccggaa ggatttccag ttttacaaag tgcgcgagat caacaactac 2940
caccacgccc acgacgccta cctgaacgcc gtcgtgggaa ccgccctgat caaaaagtac 3000
cctaagctgg aaagcgagtt cgtgtacggc gactacaagg tgtacgacgt gcggaagatg 3060
atcgccaaga gcgagcagga aatcggcaag gctaccgcca agtacttctt ctacagcaac 3120
atcatgaact ttttcaagac cgagattacc ctggccaacg gcgagatccg gaagcggcct 3180
ctgatcgaga caaacggcga aaccggggag atcgtgtggg ataagggccg ggattttgcc 3240
accgtgcgga aagtgctgag catgccccaa gtgaatatcg tgaaaaagac cgaggtgcag 3300
acaggcggct tcagcaaaga gtctatcctg cccaagagga acagcgataa gctgatcgcc 3360
agaaagaagg actgggaccc taagaagtac ggcggcttcg acagccccac cgtggcctat 3420
tctgtgctgg tggtggccaa agtggaaaag ggcaagtcca agaaactgaa gagtgtgaaa 3480
gagctgctgg ggatcaccat catggaaaga agcagcttcg agaagaatcc catcgacttt 3540
ctggaagcca agggctacaa agaagtgaaa aaggacctga tcatcaagct gcctaagtac 3600
tccctgttcg agctggaaaa cggccggaag agaatgctgg cctctgccgg cgaactgcag 3660

```
aagggaaacg aactggccct gccctccaaa tatgtgaact tcctgtacct ggccagccac     3720

tatgagaagc tgaagggctc cccgaggat aatgagcaga aacagctgtt tgtggaacag      3780

cacaagcact acctggacga gatcatcgag cagatcagcg agttctccaa gagagtgatc     3840

ctggccgacg ctaatctgga caaagtgctg tccgcctaca acaagcaccg ggataagccc     3900

atcagagagc aggccgagaa tatcatccac ctgtttaccc tgaccaatct gggagcccct     3960

gccgccttca agtactttga caccaccatc gaccggaaga ggtacaccag caccaaagag     4020

gtgctggacg ccaccctgat ccaccagagc atcaccggcc tgtacgagac acggatcgac     4080

ctgtctcagc tgggaggcga c                                               4101


<210>  3
<211>  87
<212>  PRT
<213>  Artificial Sequence

<220>
<223>  AcrIIA4

<400>  3

Met Asn Ile Asn Asp Leu Ile Arg Glu Ile Lys Asn Lys Asp Tyr Thr
1               5                   10                  15


Val Lys Leu Ser Gly Thr Asp Ser Asn Ser Ile Thr Gln Leu Ile Ile
            20                  25                  30


Arg Val Asn Asn Asp Gly Asn Glu Tyr Val Ile Ser Glu Ser Glu Asn
        35                  40                  45


Glu Ser Ile Val Glu Lys Phe Ile Ser Ala Phe Lys Asn Gly Trp Asn
    50                  55                  60


Gln Glu Tyr Glu Asp Glu Glu Glu Phe Tyr Asn Asp Met Gln Thr Ile
65                  70                  75                  80


Thr Leu Lys Ser Glu Leu Asn
                85


<210>  4
<211>  261
<212>  DNA
<213>  Artificial Sequence

<220>
<223>  AcrIIA4 encoding

<400>  4
atgaacatta acgacctgat tcgggaaata aagaacaagg actacacggt caagctgtca       60

ggcaccgact ccaactctat aacacagctc attataaggg ttaataatga tggcaacgaa      120
```

tatgtcatct ctgaatccga gaatgaatca atcgtggaga aattcatcag tgcatttaaa 180

aacggctgga accaggagta cgaagatgag gaagagttct acaatgatat gcaaactatc 240

acactgaaat ctgagctgaa c 261

<210> 5
<211> 87
<212> PRT
<213> Artificial Sequence

<220>
<223> AcrIIA4 N39A

<400> 5

Met Asn Ile Asn Asp Leu Ile Arg Glu Ile Lys Asn Lys Asp Tyr Thr
1 5 10 15

Val Lys Leu Ser Gly Thr Asp Ser Asn Ser Ile Thr Gln Leu Ile Ile
20 25 30

Arg Val Asn Asn Asp Gly Ala Glu Tyr Val Ile Ser Glu Ser Glu Asn
35 40 45

Glu Ser Ile Val Glu Lys Phe Ile Ser Ala Phe Lys Asn Gly Trp Asn
50 55 60

Gln Glu Tyr Glu Asp Glu Glu Glu Phe Tyr Asn Asp Met Gln Thr Ile
65 70 75 80

Thr Leu Lys Ser Glu Leu Asn
85

<210> 6
<211> 261
<212> DNA
<213> Artificial Sequence

<220>
<223> AcrIIA4 N39A encoding

<400> 6
atgaacatta acgacctgat tcgggaaata aagaacaagg actacacggt caagctgtca 60

ggcaccgact ccaactctat aacacagctc attataaggg ttaataatga tggcgccgaa 120

tatgtcatct ctgaatccga gaatgaatca atcgtggaga aattcatcag tgcatttaaa 180

aacggctgga accaggagta cgaagatgag gaagagttct acaatgatat gcaaactatc 240

acactgaaat ctgagctgaa c 261

<210> 7

<211> 87
<212> PRT
<213> Artificial Sequence

<220>
<223> AcrIIA4 D14A/G38A

<400> 7

Met Asn Ile Asn Asp Leu Ile Arg Glu Ile Lys Asn Lys Ala Tyr Thr
1 5 10 15

Val Lys Leu Ser Gly Thr Asp Ser Asn Ser Ile Thr Gln Leu Ile Ile
20 25 30

Arg Val Asn Asn Asp Ala Asn Glu Tyr Val Ile Ser Glu Ser Glu Asn
35 40 45

Glu Ser Ile Val Glu Lys Phe Ile Ser Ala Phe Lys Asn Gly Trp Asn
50 55 60

Gln Glu Tyr Glu Asp Glu Glu Glu Phe Tyr Asn Asp Met Gln Thr Ile
65 70 75 80

Thr Leu Lys Ser Glu Leu Asn
85

<210> 8
<211> 261
<212> DNA
<213> Artificial Sequence

<220>
<223> AcrIIA4 D14A/G38A encoding

<400> 8
atgaacatta acgacctgat tcgggaaata aagaacaagg cctacacggt caagctgtca 60

ggcaccgact ccaactctat aacacagctc attataaggg ttaataatga tgccaacgaa 120

tatgtcatct ctgaatccga gaatgaatca atcgtggaga aattcatcag tgcatttaaa 180

aacggctgga accaggagta cgaagatgag gaagagttct acaatgatat gcaaactatc 240

acactgaaat ctgagctgaa c 261

<210> 9
<211> 227
<212> PRT
<213> Artificial Sequence

<220>
<223> AcrIIA4 del N64/Q65/E66 + LOV

<400> 9

```
Met Asn Ile Asn Asp Leu Ile Arg Glu Ile Lys Asn Lys Asp Tyr Thr
1               5                   10                  15

Val Lys Leu Ser Gly Thr Asp Ser Asn Ser Ile Thr Gln Leu Ile Ile
            20                  25                  30

Arg Val Asn Asn Asp Gly Asn Glu Tyr Val Ile Ser Glu Ser Glu Asn
        35                  40                  45

Glu Ser Ile Val Glu Lys Phe Ile Ser Ala Phe Lys Asn Gly Trp Leu
    50                  55                  60

Ala Ala Ala Leu Glu Arg Ile Glu Lys Asn Phe Val Ile Thr Asp Pro
65                  70                  75                  80

Arg Leu Pro Asp Asn Pro Ile Ile Phe Ala Ser Asp Ser Phe Leu Gln
                85                  90                  95

Leu Thr Glu Tyr Ser Arg Glu Glu Ile Leu Gly Arg Asn Cys Arg Phe
            100                 105                 110

Leu Gln Gly Pro Glu Thr Asp Arg Ala Thr Val Arg Lys Ile Arg Asp
        115                 120                 125

Ala Ile Asp Asn Gln Thr Glu Val Thr Val Gln Leu Ile Asn Tyr Thr
    130                 135                 140

Lys Ser Gly Lys Lys Phe Trp Asn Leu Phe His Leu Gln Pro Met Arg
145                 150                 155                 160

Asp Gln Lys Gly Asp Val Gln Tyr Phe Ile Gly Val Gln Leu Asp Gly
                165                 170                 175

Thr Glu His Val Arg Asp Ala Ala Glu Arg Glu Ala Val Met Leu Ile
            180                 185                 190

Lys Lys Thr Ala Glu Glu Ile Asp Glu Ala Ala Lys Glu Leu Tyr Glu
        195                 200                 205

Asp Glu Glu Glu Phe Tyr Asn Asp Met Gln Thr Ile Thr Leu Lys Ser
    210                 215                 220

Glu Leu Asn
225
```

<210> 10
<211> 681
<212> DNA

<213> Artificial Sequence

<220>
<223> AcrIIA4 del N64/Q65/E66 + LOV encoding

<400> 10
atgaacatta acgacctgat tcgggaaata aagaacaagg actacacggt caagctgtca 60

ggcaccgact ccaactctat aacacagctc attataaggg ttaataatga tggcaacgaa 120

tatgtcatct ctgaatccga gaatgaatca atcgtggaga aattcatcag tgcatttaaa 180

aacggctggc ttgctgcagc gttggagcgg attgagaaga atttcgtcat aactgatccc 240

aggttgcctg ataatcctat aatattcgcc tccgatagtt ttcttcaact tacggagtac 300

tccagagagg agatactggg aaggaattgc agattcctcc aaggtccaga aactgataga 360

gctactgtga ggaagatacg ggatgccatt gacaatcaaa ctgaagtgac agtgcaactt 420

atcaattaca ccaagtcagg gaaaaaattc tggaacctgt tccatttgca acccatgagg 480

gaccaaaagg gcgatgtgca atatttcata ggcgttcaac ttgatggtac tgaacacgtc 540

agagatgcgg cggagcgaga agcagtgatg ctgattaaga aaacagcaga ggagatcgac 600

gaagcggcga aagagttgta cgaagatgag gaagagttct acaatgatat gcaaactatc 660

acactgaaat ctgagctgaa c 681

<210> 11
<211> 143
<212> PRT
<213> Artificial Sequence

<220>
<223> LOV2 domain

<400> 11

Leu Ala Thr Thr Leu Glu Arg Ile Glu Lys Asn Phe Val Ile Thr Asp
1 5 10 15

Pro Arg Leu Pro Asp Asn Pro Ile Ile Phe Ala Ser Asp Ser Phe Leu
20 25 30

Gln Leu Thr Glu Tyr Ser Arg Glu Glu Ile Leu Gly Arg Asn Cys Arg
35 40 45

Phe Leu Gln Gly Pro Glu Thr Asp Arg Ala Thr Val Arg Lys Ile Arg
50 55 60

Asp Ala Ile Asp Asn Gln Thr Glu Val Thr Val Gln Leu Ile Asn Tyr
65 70 75 80

Thr Lys Ser Gly Lys Lys Phe Trp Asn Leu Phe His Leu Gln Pro Met
85 90 95

Arg Asp Gln Lys Gly Asp Val Gln Tyr Phe Ile Gly Val Gln Leu Asp
100 105 110

Gly Thr Glu His Val Arg Asp Ala Ala Glu Arg Glu Gly Val Met Leu
115 120 125

Ile Lys Lys Thr Ala Glu Asn Ile Asp Glu Ala Ala Lys Glu Leu
130 135 140

<210> 12
<211> 429
<212> DNA
<213> Artificial Sequence

<220>
<223> LOV2 domain encoding

<400> 12
cttgctacaa ctttggagcg gattgagaag aatttcgtca taactgatcc caggttgcct 60

gataatccta taatattcgc ctccgatagt tttcttcaac ttacggagta ctccagagag 120

gagatactgg gaaggaattg cagattcctc caaggtccag aaactgatag agctactgtg 180

aggaagatac gggatgccat tgacaatcaa actgaagtga cagtgcaact tatcaattac 240

accaagtcag ggaaaaaatt ctggaacctg ttccatttgc aacccatgag ggaccaaaag 300

ggcgatgtgc aatatttcat aggcgttcaa cttgatggta ctgaacacgt cagagatgcg 360

gcggagcgag aaggagtgat gctgattaag aaaacagcag agaatatcga cgaagcggcg 420

aaagagttg 429

<210> 13
<211> 4
<212> PRT
<213> Artificial Sequence

<220>
<223> GGSG linker

<400> 13

Gly Gly Ser Gly
1

<210> 14
<211> 40
<212> PRT
<213> Artificial Sequence

<220>
<223> GGSG linker long

<400> 14

Gly Gly Ser Gly Gly Gly Ser Gly Gly Gly Ser Gly Gly Gly Ser Gly
1               5                   10                  15

Gly Gly Ser Gly Gly Gly Ser Gly Gly Gly Ser Gly Gly Gly Ser Gly
            20                  25                  30

Gly Gly Ser Gly Gly Gly Ser Gly
        35                  40

<210>  15
<211>  1552
<212>  PRT
<213>  Artificial Sequence

<220>
<223>  CasCAID#1 (NLS-Cas9-NLS fused to wild-type AcrIIA4)

<400>  15

Met Asp Tyr Lys Asp His Asp Gly Asp Tyr Lys Asp His Asp Ile Asp
1               5                   10                  15

Tyr Lys Asp Asp Asp Asp Lys Met Ala Pro Lys Lys Lys Arg Lys Val
            20                  25                  30

Gly Ile His Gly Val Pro Ala Ala Asp Lys Lys Tyr Ser Ile Gly Leu
        35                  40                  45

Asp Ile Gly Thr Asn Ser Val Gly Trp Ala Val Ile Thr Asp Glu Tyr
    50                  55                  60

Lys Val Pro Ser Lys Lys Phe Lys Val Leu Gly Asn Thr Asp Arg His
65                  70                  75                  80

Ser Ile Lys Lys Asn Leu Ile Gly Ala Leu Leu Phe Asp Ser Gly Glu
                85                  90                  95

Thr Ala Glu Ala Thr Arg Leu Lys Arg Thr Ala Arg Arg Arg Tyr Thr
            100                 105                 110

Arg Arg Lys Asn Arg Ile Cys Tyr Leu Gln Glu Ile Phe Ser Asn Glu
        115                 120                 125

Met Ala Lys Val Asp Asp Ser Phe Phe His Arg Leu Glu Glu Ser Phe
    130                 135                 140

Leu Val Glu Glu Asp Lys Lys His Glu Arg His Pro Ile Phe Gly Asn
145                 150                 155                 160

```
Ile Val Asp Glu Val Ala Tyr His Glu Lys Tyr Pro Thr Ile Tyr His
                165                 170                 175

Leu Arg Lys Lys Leu Val Asp Ser Thr Asp Lys Ala Asp Leu Arg Leu
            180                 185                 190

Ile Tyr Leu Ala Leu Ala His Met Ile Lys Phe Arg Gly His Phe Leu
        195                 200                 205

Ile Glu Gly Asp Leu Asn Pro Asp Asn Ser Asp Val Asp Lys Leu Phe
    210                 215                 220

Ile Gln Leu Val Gln Thr Tyr Asn Gln Leu Phe Glu Glu Asn Pro Ile
225                 230                 235                 240

Asn Ala Ser Gly Val Asp Ala Lys Ala Ile Leu Ser Ala Arg Leu Ser
                245                 250                 255

Lys Ser Arg Arg Leu Glu Asn Leu Ile Ala Gln Leu Pro Gly Glu Lys
            260                 265                 270

Lys Asn Gly Leu Phe Gly Asn Leu Ile Ala Leu Ser Leu Gly Leu Thr
        275                 280                 285

Pro Asn Phe Lys Ser Asn Phe Asp Leu Ala Glu Asp Ala Lys Leu Gln
    290                 295                 300

Leu Ser Lys Asp Thr Tyr Asp Asp Asp Leu Asp Asn Leu Leu Ala Gln
305                 310                 315                 320

Ile Gly Asp Gln Tyr Ala Asp Leu Phe Leu Ala Ala Lys Asn Leu Ser
                325                 330                 335

Asp Ala Ile Leu Leu Ser Asp Ile Leu Arg Val Asn Thr Glu Ile Thr
            340                 345                 350

Lys Ala Pro Leu Ser Ala Ser Met Ile Lys Arg Tyr Asp Glu His His
        355                 360                 365

Gln Asp Leu Thr Leu Leu Lys Ala Leu Val Arg Gln Gln Leu Pro Glu
    370                 375                 380

Lys Tyr Lys Glu Ile Phe Phe Asp Gln Ser Lys Asn Gly Tyr Ala Gly
385                 390                 395                 400

Tyr Ile Asp Gly Gly Ala Ser Gln Glu Glu Phe Tyr Lys Phe Ile Lys
                405                 410                 415
```

Pro Ile Leu Glu Lys Met Asp Gly Thr Glu Glu Leu Leu Val Lys Leu
420 425 430

Asn Arg Glu Asp Leu Leu Arg Lys Gln Arg Thr Phe Asp Asn Gly Ser
435 440 445

Ile Pro His Gln Ile His Leu Gly Glu Leu His Ala Ile Leu Arg Arg
450 455 460

Gln Glu Asp Phe Tyr Pro Phe Leu Lys Asp Asn Arg Glu Lys Ile Glu
465 470 475 480

Lys Ile Leu Thr Phe Arg Ile Pro Tyr Tyr Val Gly Pro Leu Ala Arg
485 490 495

Gly Asn Ser Arg Phe Ala Trp Met Thr Arg Lys Ser Glu Glu Thr Ile
500 505 510

Thr Pro Trp Asn Phe Glu Glu Val Val Asp Lys Gly Ala Ser Ala Gln
515 520 525

Ser Phe Ile Glu Arg Met Thr Asn Phe Asp Lys Asn Leu Pro Asn Glu
530 535 540

Lys Val Leu Pro Lys His Ser Leu Leu Tyr Glu Tyr Phe Thr Val Tyr
545 550 555 560

Asn Glu Leu Thr Lys Val Lys Tyr Val Thr Glu Gly Met Arg Lys Pro
565 570 575

Ala Phe Leu Ser Gly Glu Gln Lys Lys Ala Ile Val Asp Leu Leu Phe
580 585 590

Lys Thr Asn Arg Lys Val Thr Val Lys Gln Leu Lys Glu Asp Tyr Phe
595 600 605

Lys Lys Ile Glu Cys Phe Asp Ser Val Glu Ile Ser Gly Val Glu Asp
610 615 620

Arg Phe Asn Ala Ser Leu Gly Thr Tyr His Asp Leu Leu Lys Ile Ile
625 630 635 640

Lys Asp Lys Asp Phe Leu Asp Asn Glu Glu Asn Glu Asp Ile Leu Glu
645 650 655

Asp Ile Val Leu Thr Leu Thr Leu Phe Glu Asp Arg Glu Met Ile Glu
660 665 670

```
Glu Arg Leu Lys Thr Tyr Ala His Leu Phe Asp Asp Lys Val Met Lys
        675                 680                 685

Gln Leu Lys Arg Arg Arg Tyr Thr Gly Trp Gly Arg Leu Ser Arg Lys
    690                 695                 700

Leu Ile Asn Gly Ile Arg Asp Lys Gln Ser Gly Lys Thr Ile Leu Asp
705                 710                 715                 720

Phe Leu Lys Ser Asp Gly Phe Ala Asn Arg Asn Phe Met Gln Leu Ile
                725                 730                 735

His Asp Asp Ser Leu Thr Phe Lys Glu Asp Ile Gln Lys Ala Gln Val
            740                 745                 750

Ser Gly Gln Gly Asp Ser Leu His Glu His Ile Ala Asn Leu Ala Gly
        755                 760                 765

Ser Pro Ala Ile Lys Lys Gly Ile Leu Gln Thr Val Lys Val Val Asp
    770                 775                 780

Glu Leu Val Lys Val Met Gly Arg His Lys Pro Glu Asn Ile Val Ile
785                 790                 795                 800

Glu Met Ala Arg Glu Asn Gln Thr Thr Gln Lys Gly Gln Lys Asn Ser
                805                 810                 815

Arg Glu Arg Met Lys Arg Ile Glu Glu Gly Ile Lys Glu Leu Gly Ser
            820                 825                 830

Gln Ile Leu Lys Glu His Pro Val Glu Asn Thr Gln Leu Gln Asn Glu
        835                 840                 845

Lys Leu Tyr Leu Tyr Tyr Leu Gln Asn Gly Arg Asp Met Tyr Val Asp
    850                 855                 860

Gln Glu Leu Asp Ile Asn Arg Leu Ser Asp Tyr Asp Val Asp His Ile
865                 870                 875                 880

Val Pro Gln Ser Phe Leu Lys Asp Asp Ser Ile Asp Asn Lys Val Leu
                885                 890                 895

Thr Arg Ser Asp Lys Asn Arg Gly Lys Ser Asp Asn Val Pro Ser Glu
            900                 905                 910

Glu Val Val Lys Lys Met Lys Asn Tyr Trp Arg Gln Leu Leu Asn Ala
```

915 920 925

Lys Leu Ile Thr Gln Arg Lys Phe Asp Asn Leu Thr Lys Ala Glu Arg
930 935 940

Gly Gly Leu Ser Glu Leu Asp Lys Ala Gly Phe Ile Lys Arg Gln Leu
945 950 955 960

Val Glu Thr Arg Gln Ile Thr Lys His Val Ala Gln Ile Leu Asp Ser
965 970 975

Arg Met Asn Thr Lys Tyr Asp Glu Asn Asp Lys Leu Ile Arg Glu Val
980 985 990

Lys Val Ile Thr Leu Lys Ser Lys Leu Val Ser Asp Phe Arg Lys Asp
995 1000 1005

Phe Gln Phe Tyr Lys Val Arg Glu Ile Asn Asn Tyr His His Ala
1010 1015 1020

His Asp Ala Tyr Leu Asn Ala Val Val Gly Thr Ala Leu Ile Lys
1025 1030 1035

Lys Tyr Pro Lys Leu Glu Ser Glu Phe Val Tyr Gly Asp Tyr Lys
1040 1045 1050

Val Tyr Asp Val Arg Lys Met Ile Ala Lys Ser Glu Gln Glu Ile
1055 1060 1065

Gly Lys Ala Thr Ala Lys Tyr Phe Phe Tyr Ser Asn Ile Met Asn
1070 1075 1080

Phe Phe Lys Thr Glu Ile Thr Leu Ala Asn Gly Glu Ile Arg Lys
1085 1090 1095

Arg Pro Leu Ile Glu Thr Asn Gly Glu Thr Gly Glu Ile Val Trp
1100 1105 1110

Asp Lys Gly Arg Asp Phe Ala Thr Val Arg Lys Val Leu Ser Met
1115 1120 1125

Pro Gln Val Asn Ile Val Lys Lys Thr Glu Val Gln Thr Gly Gly
1130 1135 1140

Phe Ser Lys Glu Ser Ile Leu Pro Lys Arg Asn Ser Asp Lys Leu
1145 1150 1155

Ile Ala Arg Lys Lys Asp Trp Asp Pro Lys Lys Tyr Gly Gly Phe
1160 1165 1170

Asp Ser Pro Thr Val Ala Tyr Ser Val Leu Val Val Ala Lys Val
1175 1180 1185

Glu Lys Gly Lys Ser Lys Lys Leu Lys Ser Val Lys Glu Leu Leu
1190 1195 1200

Gly Ile Thr Ile Met Glu Arg Ser Ser Phe Glu Lys Asn Pro Ile
1205 1210 1215

Asp Phe Leu Glu Ala Lys Gly Tyr Lys Glu Val Lys Lys Asp Leu
1220 1225 1230

Ile Ile Lys Leu Pro Lys Tyr Ser Leu Phe Glu Leu Glu Asn Gly
1235 1240 1245

Arg Lys Arg Met Leu Ala Ser Ala Gly Glu Leu Gln Lys Gly Asn
1250 1255 1260

Glu Leu Ala Leu Pro Ser Lys Tyr Val Asn Phe Leu Tyr Leu Ala
1265 1270 1275

Ser His Tyr Glu Lys Leu Lys Gly Ser Pro Glu Asp Asn Glu Gln
1280 1285 1290

Lys Gln Leu Phe Val Glu Gln His Lys His Tyr Leu Asp Glu Ile
1295 1300 1305

Ile Glu Gln Ile Ser Glu Phe Ser Lys Arg Val Ile Leu Ala Asp
1310 1315 1320

Ala Asn Leu Asp Lys Val Leu Ser Ala Tyr Asn Lys His Arg Asp
1325 1330 1335

Lys Pro Ile Arg Glu Gln Ala Glu Asn Ile Ile His Leu Phe Thr
1340 1345 1350

Leu Thr Asn Leu Gly Ala Pro Ala Ala Phe Lys Tyr Phe Asp Thr
1355 1360 1365

Thr Ile Asp Arg Lys Arg Tyr Thr Ser Thr Lys Glu Val Leu Asp
1370 1375 1380

Ala Thr Leu Ile His Gln Ser Ile Thr Gly Leu Tyr Glu Thr Arg
1385 1390 1395

Ile Asp Leu Ser Gln Leu Gly Gly Asp Lys Arg Pro Ala Ala Thr
1400 1405 1410

Lys Lys Ala Gly Gln Ala Lys Lys Lys Lys Glu Phe Gly Gly Ser
1415 1420 1425

Gly Gly Gly Ser Gly Gly Gly Ser Gly Gly Gly Ser Gly Gly Gly
1430 1435 1440

Ser Gly Gly Gly Ser Gly Gly Gly Ser Gly Gly Gly Ser Gly Gly
1445 1450 1455

Gly Ser Gly Gly Gly Ser Gly Met Asn Ile Asn Asp Leu Ile Arg
1460 1465 1470

Glu Ile Lys Asn Lys Asp Tyr Thr Val Lys Leu Ser Gly Thr Asp
1475 1480 1485

Ser Asn Ser Ile Thr Gln Leu Ile Ile Arg Val Asn Asn Asp Gly
1490 1495 1500

Asn Glu Tyr Val Ile Ser Glu Ser Glu Asn Glu Ser Ile Val Glu
1505 1510 1515

Lys Phe Ile Ser Ala Phe Lys Asn Gly Trp Asn Gln Glu Tyr Glu
1520 1525 1530

Asp Glu Glu Glu Phe Tyr Asn Asp Met Gln Thr Ile Thr Leu Lys
1535 1540 1545

Ser Glu Leu Asn
1550

<210> 16
<211> 1692
<212> PRT
<213> Artificial Sequence

<220>
<223> CasCAID#2 (NLS-Cas9-NLS fused to AcrIIA4 with N64/Q65/E66 deletion and an insertion of a LOV-domain)

<400> 16

Met Asp Tyr Lys Asp His Asp Gly Asp Tyr Lys Asp His Asp Ile Asp
1 5 10 15

Tyr Lys Asp Asp Asp Asp Lys Met Ala Pro Lys Lys Lys Arg Lys Val
20 25 30

```
Gly Ile His Gly Val Pro Ala Ala Asp Lys Lys Tyr Ser Ile Gly Leu
        35                  40                  45

Asp Ile Gly Thr Asn Ser Val Gly Trp Ala Val Ile Thr Asp Glu Tyr
    50                  55                  60

Lys Val Pro Ser Lys Lys Phe Lys Val Leu Gly Asn Thr Asp Arg His
65                  70                  75                  80

Ser Ile Lys Lys Asn Leu Ile Gly Ala Leu Leu Phe Asp Ser Gly Glu
                85                  90                  95

Thr Ala Glu Ala Thr Arg Leu Lys Arg Thr Ala Arg Arg Arg Tyr Thr
            100                 105                 110

Arg Arg Lys Asn Arg Ile Cys Tyr Leu Gln Glu Ile Phe Ser Asn Glu
        115                 120                 125

Met Ala Lys Val Asp Asp Ser Phe Phe His Arg Leu Glu Glu Ser Phe
    130                 135                 140

Leu Val Glu Glu Asp Lys Lys His Glu Arg His Pro Ile Phe Gly Asn
145                 150                 155                 160

Ile Val Asp Glu Val Ala Tyr His Glu Lys Tyr Pro Thr Ile Tyr His
                165                 170                 175

Leu Arg Lys Lys Leu Val Asp Ser Thr Asp Lys Ala Asp Leu Arg Leu
            180                 185                 190

Ile Tyr Leu Ala Leu Ala His Met Ile Lys Phe Arg Gly His Phe Leu
        195                 200                 205

Ile Glu Gly Asp Leu Asn Pro Asp Asn Ser Asp Val Asp Lys Leu Phe
    210                 215                 220

Ile Gln Leu Val Gln Thr Tyr Asn Gln Leu Phe Glu Glu Asn Pro Ile
225                 230                 235                 240

Asn Ala Ser Gly Val Asp Ala Lys Ala Ile Leu Ser Ala Arg Leu Ser
                245                 250                 255

Lys Ser Arg Arg Leu Glu Asn Leu Ile Ala Gln Leu Pro Gly Glu Lys
            260                 265                 270

Lys Asn Gly Leu Phe Gly Asn Leu Ile Ala Leu Ser Leu Gly Leu Thr
        275                 280                 285
```

```
Pro Asn Phe Lys Ser Asn Phe Asp Leu Ala Glu Asp Ala Lys Leu Gln
    290                 295                 300

Leu Ser Lys Asp Thr Tyr Asp Asp Asp Leu Asp Asn Leu Leu Ala Gln
305                 310                 315                 320

Ile Gly Asp Gln Tyr Ala Asp Leu Phe Leu Ala Ala Lys Asn Leu Ser
                325                 330                 335

Asp Ala Ile Leu Leu Ser Asp Ile Leu Arg Val Asn Thr Glu Ile Thr
            340                 345                 350

Lys Ala Pro Leu Ser Ala Ser Met Ile Lys Arg Tyr Asp Glu His His
        355                 360                 365

Gln Asp Leu Thr Leu Leu Lys Ala Leu Val Arg Gln Gln Leu Pro Glu
    370                 375                 380

Lys Tyr Lys Glu Ile Phe Phe Asp Gln Ser Lys Asn Gly Tyr Ala Gly
385                 390                 395                 400

Tyr Ile Asp Gly Gly Ala Ser Gln Glu Glu Phe Tyr Lys Phe Ile Lys
                405                 410                 415

Pro Ile Leu Glu Lys Met Asp Gly Thr Glu Glu Leu Leu Val Lys Leu
            420                 425                 430

Asn Arg Glu Asp Leu Leu Arg Lys Gln Arg Thr Phe Asp Asn Gly Ser
        435                 440                 445

Ile Pro His Gln Ile His Leu Gly Glu Leu His Ala Ile Leu Arg Arg
    450                 455                 460

Gln Glu Asp Phe Tyr Pro Phe Leu Lys Asp Asn Arg Glu Lys Ile Glu
465                 470                 475                 480

Lys Ile Leu Thr Phe Arg Ile Pro Tyr Tyr Val Gly Pro Leu Ala Arg
                485                 490                 495

Gly Asn Ser Arg Phe Ala Trp Met Thr Arg Lys Ser Glu Glu Thr Ile
            500                 505                 510

Thr Pro Trp Asn Phe Glu Glu Val Val Asp Lys Gly Ala Ser Ala Gln
        515                 520                 525

Ser Phe Ile Glu Arg Met Thr Asn Phe Asp Lys Asn Leu Pro Asn Glu
```

530 535 540

Lys Val Leu Pro Lys His Ser Leu Leu Tyr Glu Tyr Phe Thr Val Tyr
545 550 555 560

Asn Glu Leu Thr Lys Val Lys Tyr Val Thr Glu Gly Met Arg Lys Pro
565 570 575

Ala Phe Leu Ser Gly Glu Gln Lys Lys Ala Ile Val Asp Leu Leu Phe
580 585 590

Lys Thr Asn Arg Lys Val Thr Val Lys Gln Leu Lys Glu Asp Tyr Phe
595 600 605

Lys Lys Ile Glu Cys Phe Asp Ser Val Glu Ile Ser Gly Val Glu Asp
610 615 620

Arg Phe Asn Ala Ser Leu Gly Thr Tyr His Asp Leu Leu Lys Ile Ile
625 630 635 640

Lys Asp Lys Asp Phe Leu Asp Asn Glu Glu Asn Glu Asp Ile Leu Glu
645 650 655

Asp Ile Val Leu Thr Leu Thr Leu Phe Glu Asp Arg Glu Met Ile Glu
660 665 670

Glu Arg Leu Lys Thr Tyr Ala His Leu Phe Asp Asp Lys Val Met Lys
675 680 685

Gln Leu Lys Arg Arg Arg Tyr Thr Gly Trp Gly Arg Leu Ser Arg Lys
690 695 700

Leu Ile Asn Gly Ile Arg Asp Lys Gln Ser Gly Lys Thr Ile Leu Asp
705 710 715 720

Phe Leu Lys Ser Asp Gly Phe Ala Asn Arg Asn Phe Met Gln Leu Ile
725 730 735

His Asp Asp Ser Leu Thr Phe Lys Glu Asp Ile Gln Lys Ala Gln Val
740 745 750

Ser Gly Gln Gly Asp Ser Leu His Glu His Ile Ala Asn Leu Ala Gly
755 760 765

Ser Pro Ala Ile Lys Lys Gly Ile Leu Gln Thr Val Lys Val Val Asp
770 775 780

Glu Leu Val Lys Val Met Gly Arg His Lys Pro Glu Asn Ile Val Ile
785                 790                 795                 800

Glu Met Ala Arg Glu Asn Gln Thr Thr Gln Lys Gly Gln Lys Asn Ser
                805                 810                 815

Arg Glu Arg Met Lys Arg Ile Glu Glu Gly Ile Lys Glu Leu Gly Ser
            820                 825                 830

Gln Ile Leu Lys Glu His Pro Val Glu Asn Thr Gln Leu Gln Asn Glu
        835                 840                 845

Lys Leu Tyr Leu Tyr Tyr Leu Gln Asn Gly Arg Asp Met Tyr Val Asp
    850                 855                 860

Gln Glu Leu Asp Ile Asn Arg Leu Ser Asp Tyr Asp Val Asp His Ile
865                 870                 875                 880

Val Pro Gln Ser Phe Leu Lys Asp Asp Ser Ile Asp Asn Lys Val Leu
                885                 890                 895

Thr Arg Ser Asp Lys Asn Arg Gly Lys Ser Asp Asn Val Pro Ser Glu
            900                 905                 910

Glu Val Val Lys Lys Met Lys Asn Tyr Trp Arg Gln Leu Leu Asn Ala
        915                 920                 925

Lys Leu Ile Thr Gln Arg Lys Phe Asp Asn Leu Thr Lys Ala Glu Arg
    930                 935                 940

Gly Gly Leu Ser Glu Leu Asp Lys Ala Gly Phe Ile Lys Arg Gln Leu
945                 950                 955                 960

Val Glu Thr Arg Gln Ile Thr Lys His Val Ala Gln Ile Leu Asp Ser
                965                 970                 975

Arg Met Asn Thr Lys Tyr Asp Glu Asn Asp Lys Leu Ile Arg Glu Val
            980                 985                 990

Lys Val Ile Thr Leu Lys Ser Lys  Leu Val Ser Asp Phe  Arg Lys Asp
        995                 1000                1005

Phe Gln  Phe Tyr Lys Val Arg  Glu Ile Asn Asn Tyr  His His Ala
    1010                1015                1020

His Asp  Ala Tyr Leu Asn Ala  Val Val Gly Thr Ala  Leu Ile Lys
    1025                1030                1035

Lys Tyr Pro Lys Leu Glu Ser Glu Phe Val Tyr Gly Asp Tyr Lys
1040 1045 1050

Val Tyr Asp Val Arg Lys Met Ile Ala Lys Ser Glu Gln Glu Ile
1055 1060 1065

Gly Lys Ala Thr Ala Lys Tyr Phe Phe Tyr Ser Asn Ile Met Asn
1070 1075 1080

Phe Phe Lys Thr Glu Ile Thr Leu Ala Asn Gly Glu Ile Arg Lys
1085 1090 1095

Arg Pro Leu Ile Glu Thr Asn Gly Glu Thr Gly Glu Ile Val Trp
1100 1105 1110

Asp Lys Gly Arg Asp Phe Ala Thr Val Arg Lys Val Leu Ser Met
1115 1120 1125

Pro Gln Val Asn Ile Val Lys Lys Thr Glu Val Gln Thr Gly Gly
1130 1135 1140

Phe Ser Lys Glu Ser Ile Leu Pro Lys Arg Asn Ser Asp Lys Leu
1145 1150 1155

Ile Ala Arg Lys Lys Asp Trp Asp Pro Lys Lys Tyr Gly Gly Phe
1160 1165 1170

Asp Ser Pro Thr Val Ala Tyr Ser Val Leu Val Val Ala Lys Val
1175 1180 1185

Glu Lys Gly Lys Ser Lys Lys Leu Lys Ser Val Lys Glu Leu Leu
1190 1195 1200

Gly Ile Thr Ile Met Glu Arg Ser Ser Phe Glu Lys Asn Pro Ile
1205 1210 1215

Asp Phe Leu Glu Ala Lys Gly Tyr Lys Glu Val Lys Lys Asp Leu
1220 1225 1230

Ile Ile Lys Leu Pro Lys Tyr Ser Leu Phe Glu Leu Glu Asn Gly
1235 1240 1245

Arg Lys Arg Met Leu Ala Ser Ala Gly Glu Leu Gln Lys Gly Asn
1250 1255 1260

Glu Leu Ala Leu Pro Ser Lys Tyr Val Asn Phe Leu Tyr Leu Ala
1265 1270 1275

```
Ser His Tyr Glu Lys Leu Lys Gly Ser Pro Glu Asp Asn Glu Gln
    1280                1285                1290

Lys Gln Leu Phe Val Glu Gln His Lys His Tyr Leu Asp Glu Ile
    1295                1300                1305

Ile Glu Gln Ile Ser Glu Phe Ser Lys Arg Val Ile Leu Ala Asp
    1310                1315                1320

Ala Asn Leu Asp Lys Val Leu Ser Ala Tyr Asn Lys His Arg Asp
    1325                1330                1335

Lys Pro Ile Arg Glu Gln Ala Glu Asn Ile Ile His Leu Phe Thr
    1340                1345                1350

Leu Thr Asn Leu Gly Ala Pro Ala Ala Phe Lys Tyr Phe Asp Thr
    1355                1360                1365

Thr Ile Asp Arg Lys Arg Tyr Thr Ser Thr Lys Glu Val Leu Asp
    1370                1375                1380

Ala Thr Leu Ile His Gln Ser Ile Thr Gly Leu Tyr Glu Thr Arg
    1385                1390                1395

Ile Asp Leu Ser Gln Leu Gly Gly Asp Lys Arg Pro Ala Ala Thr
    1400                1405                1410

Lys Lys Ala Gly Gln Ala Lys Lys Lys Lys Glu Phe Gly Gly Ser
    1415                1420                1425

Gly Gly Gly Ser Gly Gly Gly Ser Gly Gly Gly Ser Gly Gly Gly
    1430                1435                1440

Ser Gly Gly Gly Ser Gly Gly Gly Ser Gly Gly Gly Ser Gly Gly
    1445                1450                1455

Gly Ser Gly Gly Gly Ser Gly Met Asn Ile Asn Asp Leu Ile Arg
    1460                1465                1470

Glu Ile Lys Asn Lys Asp Tyr Thr Val Lys Leu Ser Gly Thr Asp
    1475                1480                1485

Ser Asn Ser Ile Thr Gln Leu Ile Ile Arg Val Asn Asn Asp Gly
    1490                1495                1500

Asn Glu Tyr Val Ile Ser Glu Ser Glu Asn Glu Ser Ile Val Glu
```

1505 1510 1515

Lys Phe Ile Ser Ala Phe Lys Asn Gly Trp Leu Ala Thr Thr Leu
1520 1525 1530

Glu Arg Ile Glu Lys Asn Phe Val Ile Thr Asp Pro Arg Leu Pro
1535 1540 1545

Asp Asn Pro Ile Ile Phe Ala Ser Asp Ser Phe Leu Gln Leu Thr
1550 1555 1560

Glu Tyr Ser Arg Glu Glu Ile Leu Gly Arg Asn Cys Arg Phe Leu
1565 1570 1575

Gln Gly Pro Glu Thr Asp Arg Ala Thr Val Arg Lys Ile Arg Asp
1580 1585 1590

Ala Ile Asp Asn Gln Thr Glu Val Thr Val Gln Leu Ile Asn Tyr
1595 1600 1605

Thr Lys Ser Gly Lys Lys Phe Trp Asn Leu Phe His Leu Gln Pro
1610 1615 1620

Met Arg Asp Gln Lys Gly Asp Val Gln Tyr Phe Ile Gly Val Gln
1625 1630 1635

Leu Asp Gly Thr Glu His Val Arg Asp Ala Ala Glu Arg Glu Gly
1640 1645 1650

Val Met Leu Ile Lys Lys Thr Ala Glu Asn Ile Asp Glu Ala Ala
1655 1660 1665

Lys Glu Leu Tyr Glu Asp Glu Glu Glu Phe Tyr Asn Asp Met Gln
1670 1675 1680

Thr Ile Thr Leu Lys Ser Glu Leu Asn
1685 1690

<210> 17
<211> 1695
<212> PRT
<213> Artificial Sequence

<220>
<223> CasCAID#3 (NLS-Cas9-NLS fused to AcrIIA4 with Q65/E66 deletion and an insertion of a LOV-domain flanked by glycine linker residues)

<400> 17

```
Met Asp Tyr Lys Asp His Asp Gly Asp Tyr Lys Asp His Asp Ile Asp
1               5                   10                  15

Tyr Lys Asp Asp Asp Asp Lys Met Ala Pro Lys Lys Lys Arg Lys Val
            20                  25                  30

Gly Ile His Gly Val Pro Ala Ala Asp Lys Lys Tyr Ser Ile Gly Leu
        35                  40                  45

Asp Ile Gly Thr Asn Ser Val Gly Trp Ala Val Ile Thr Asp Glu Tyr
    50                  55                  60

Lys Val Pro Ser Lys Lys Phe Lys Val Leu Gly Asn Thr Asp Arg His
65                  70                  75                  80

Ser Ile Lys Lys Asn Leu Ile Gly Ala Leu Leu Phe Asp Ser Gly Glu
                85                  90                  95

Thr Ala Glu Ala Thr Arg Leu Lys Arg Thr Ala Arg Arg Arg Tyr Thr
            100                 105                 110

Arg Arg Lys Asn Arg Ile Cys Tyr Leu Gln Glu Ile Phe Ser Asn Glu
        115                 120                 125

Met Ala Lys Val Asp Asp Ser Phe Phe His Arg Leu Glu Glu Ser Phe
    130                 135                 140

Leu Val Glu Glu Asp Lys Lys His Glu Arg His Pro Ile Phe Gly Asn
145                 150                 155                 160

Ile Val Asp Glu Val Ala Tyr His Glu Lys Tyr Pro Thr Ile Tyr His
                165                 170                 175

Leu Arg Lys Lys Leu Val Asp Ser Thr Asp Lys Ala Asp Leu Arg Leu
            180                 185                 190

Ile Tyr Leu Ala Leu Ala His Met Ile Lys Phe Arg Gly His Phe Leu
        195                 200                 205

Ile Glu Gly Asp Leu Asn Pro Asp Asn Ser Asp Val Asp Lys Leu Phe
    210                 215                 220

Ile Gln Leu Val Gln Thr Tyr Asn Gln Leu Phe Glu Glu Asn Pro Ile
225                 230                 235                 240

Asn Ala Ser Gly Val Asp Ala Lys Ala Ile Leu Ser Ala Arg Leu Ser
                245                 250                 255
```

```
Lys Ser Arg Arg Leu Glu Asn Leu Ile Ala Gln Leu Pro Gly Glu Lys
            260                 265                 270

Lys Asn Gly Leu Phe Gly Asn Leu Ile Ala Leu Ser Leu Gly Leu Thr
        275                 280                 285

Pro Asn Phe Lys Ser Asn Phe Asp Leu Ala Glu Asp Ala Lys Leu Gln
    290                 295                 300

Leu Ser Lys Asp Thr Tyr Asp Asp Asp Leu Asp Asn Leu Leu Ala Gln
305                 310                 315                 320

Ile Gly Asp Gln Tyr Ala Asp Leu Phe Leu Ala Ala Lys Asn Leu Ser
                325                 330                 335

Asp Ala Ile Leu Leu Ser Asp Ile Leu Arg Val Asn Thr Glu Ile Thr
            340                 345                 350

Lys Ala Pro Leu Ser Ala Ser Met Ile Lys Arg Tyr Asp Glu His His
        355                 360                 365

Gln Asp Leu Thr Leu Leu Lys Ala Leu Val Arg Gln Gln Leu Pro Glu
    370                 375                 380

Lys Tyr Lys Glu Ile Phe Phe Asp Gln Ser Lys Asn Gly Tyr Ala Gly
385                 390                 395                 400

Tyr Ile Asp Gly Gly Ala Ser Gln Glu Glu Phe Tyr Lys Phe Ile Lys
                405                 410                 415

Pro Ile Leu Glu Lys Met Asp Gly Thr Glu Glu Leu Leu Val Lys Leu
            420                 425                 430

Asn Arg Glu Asp Leu Leu Arg Lys Gln Arg Thr Phe Asp Asn Gly Ser
        435                 440                 445

Ile Pro His Gln Ile His Leu Gly Glu Leu His Ala Ile Leu Arg Arg
    450                 455                 460

Gln Glu Asp Phe Tyr Pro Phe Leu Lys Asp Asn Arg Glu Lys Ile Glu
465                 470                 475                 480

Lys Ile Leu Thr Phe Arg Ile Pro Tyr Tyr Val Gly Pro Leu Ala Arg
                485                 490                 495

Gly Asn Ser Arg Phe Ala Trp Met Thr Arg Lys Ser Glu Glu Thr Ile
            500                 505                 510
```

Thr Pro Trp Asn Phe Glu Glu Val Val Asp Lys Gly Ala Ser Ala Gln
515 520 525

Ser Phe Ile Glu Arg Met Thr Asn Phe Asp Lys Asn Leu Pro Asn Glu
530 535 540

Lys Val Leu Pro Lys His Ser Leu Leu Tyr Glu Tyr Phe Thr Val Tyr
545 550 555 560

Asn Glu Leu Thr Lys Val Lys Tyr Val Thr Glu Gly Met Arg Lys Pro
565 570 575

Ala Phe Leu Ser Gly Glu Gln Lys Lys Ala Ile Val Asp Leu Leu Phe
580 585 590

Lys Thr Asn Arg Lys Val Thr Val Lys Gln Leu Lys Glu Asp Tyr Phe
595 600 605

Lys Lys Ile Glu Cys Phe Asp Ser Val Glu Ile Ser Gly Val Glu Asp
610 615 620

Arg Phe Asn Ala Ser Leu Gly Thr Tyr His Asp Leu Leu Lys Ile Ile
625 630 635 640

Lys Asp Lys Asp Phe Leu Asp Asn Glu Glu Asn Glu Asp Ile Leu Glu
645 650 655

Asp Ile Val Leu Thr Leu Thr Leu Phe Glu Asp Arg Glu Met Ile Glu
660 665 670

Glu Arg Leu Lys Thr Tyr Ala His Leu Phe Asp Asp Lys Val Met Lys
675 680 685

Gln Leu Lys Arg Arg Arg Tyr Thr Gly Trp Gly Arg Leu Ser Arg Lys
690 695 700

Leu Ile Asn Gly Ile Arg Asp Lys Gln Ser Gly Lys Thr Ile Leu Asp
705 710 715 720

Phe Leu Lys Ser Asp Gly Phe Ala Asn Arg Asn Phe Met Gln Leu Ile
725 730 735

His Asp Asp Ser Leu Thr Phe Lys Glu Asp Ile Gln Lys Ala Gln Val
740 745 750

Ser Gly Gln Gly Asp Ser Leu His Glu His Ile Ala Asn Leu Ala Gly

755 760 765

Ser Pro Ala Ile Lys Lys Gly Ile Leu Gln Thr Val Lys Val Val Asp
770 775 780

Glu Leu Val Lys Val Met Gly Arg His Lys Pro Glu Asn Ile Val Ile
785 790 795 800

Glu Met Ala Arg Glu Asn Gln Thr Thr Gln Lys Gly Gln Lys Asn Ser
805 810 815

Arg Glu Arg Met Lys Arg Ile Glu Glu Gly Ile Lys Glu Leu Gly Ser
820 825 830

Gln Ile Leu Lys Glu His Pro Val Glu Asn Thr Gln Leu Gln Asn Glu
835 840 845

Lys Leu Tyr Leu Tyr Tyr Leu Gln Asn Gly Arg Asp Met Tyr Val Asp
850 855 860

Gln Glu Leu Asp Ile Asn Arg Leu Ser Asp Tyr Asp Val Asp His Ile
865 870 875 880

Val Pro Gln Ser Phe Leu Lys Asp Asp Ser Ile Asp Asn Lys Val Leu
885 890 895

Thr Arg Ser Asp Lys Asn Arg Gly Lys Ser Asp Asn Val Pro Ser Glu
900 905 910

Glu Val Val Lys Lys Met Lys Asn Tyr Trp Arg Gln Leu Leu Asn Ala
915 920 925

Lys Leu Ile Thr Gln Arg Lys Phe Asp Asn Leu Thr Lys Ala Glu Arg
930 935 940

Gly Gly Leu Ser Glu Leu Asp Lys Ala Gly Phe Ile Lys Arg Gln Leu
945 950 955 960

Val Glu Thr Arg Gln Ile Thr Lys His Val Ala Gln Ile Leu Asp Ser
965 970 975

Arg Met Asn Thr Lys Tyr Asp Glu Asn Asp Lys Leu Ile Arg Glu Val
980 985 990

Lys Val Ile Thr Leu Lys Ser Lys Leu Val Ser Asp Phe Arg Lys Asp
995 1000 1005

```
Phe Gln  Phe Tyr Lys Val Arg  Glu Ile Asn Asn Tyr  His His Ala
    1010                 1015                 1020

His Asp  Ala Tyr Leu Asn Ala  Val Val Gly Thr Ala  Leu Ile Lys
    1025                 1030                 1035

Lys Tyr  Pro Lys Leu Glu Ser  Glu Phe Val Tyr Gly  Asp Tyr Lys
    1040                 1045                 1050

Val Tyr  Asp Val Arg Lys Met  Ile Ala Lys Ser Glu  Gln Glu Ile
    1055                 1060                 1065

Gly Lys  Ala Thr Ala Lys Tyr  Phe Phe Tyr Ser Asn  Ile Met Asn
    1070                 1075                 1080

Phe Phe  Lys Thr Glu Ile Thr  Leu Ala Asn Gly Glu  Ile Arg Lys
    1085                 1090                 1095

Arg Pro  Leu Ile Glu Thr Asn  Gly Glu Thr Gly Glu  Ile Val Trp
    1100                 1105                 1110

Asp Lys  Gly Arg Asp Phe Ala  Thr Val Arg Lys Val  Leu Ser Met
    1115                 1120                 1125

Pro Gln  Val Asn Ile Val Lys  Lys Thr Glu Val Gln  Thr Gly Gly
    1130                 1135                 1140

Phe Ser  Lys Glu Ser Ile Leu  Pro Lys Arg Asn Ser  Asp Lys Leu
    1145                 1150                 1155

Ile Ala  Arg Lys Lys Asp Trp  Asp Pro Lys Lys Tyr  Gly Gly Phe
    1160                 1165                 1170

Asp Ser  Pro Thr Val Ala Tyr  Ser Val Leu Val Val  Ala Lys Val
    1175                 1180                 1185

Glu Lys  Gly Lys Ser Lys Lys  Leu Lys Ser Val Lys  Glu Leu Leu
    1190                 1195                 1200

Gly Ile  Thr Ile Met Glu Arg  Ser Ser Phe Glu Lys  Asn Pro Ile
    1205                 1210                 1215

Asp Phe  Leu Glu Ala Lys Gly  Tyr Lys Glu Val Lys  Lys Asp Leu
    1220                 1225                 1230

Ile Ile  Lys Leu Pro Lys Tyr  Ser Leu Phe Glu Leu  Glu Asn Gly
    1235                 1240                 1245
```

Arg Lys Arg Met Leu Ala Ser Ala Gly Glu Leu Gln Lys Gly Asn
1250 1255 1260

Glu Leu Ala Leu Pro Ser Lys Tyr Val Asn Phe Leu Tyr Leu Ala
1265 1270 1275

Ser His Tyr Glu Lys Leu Lys Gly Ser Pro Glu Asp Asn Glu Gln
1280 1285 1290

Lys Gln Leu Phe Val Glu Gln His Lys His Tyr Leu Asp Glu Ile
1295 1300 1305

Ile Glu Gln Ile Ser Glu Phe Ser Lys Arg Val Ile Leu Ala Asp
1310 1315 1320

Ala Asn Leu Asp Lys Val Leu Ser Ala Tyr Asn Lys His Arg Asp
1325 1330 1335

Lys Pro Ile Arg Glu Gln Ala Glu Asn Ile Ile His Leu Phe Thr
1340 1345 1350

Leu Thr Asn Leu Gly Ala Pro Ala Ala Phe Lys Tyr Phe Asp Thr
1355 1360 1365

Thr Ile Asp Arg Lys Arg Tyr Thr Ser Thr Lys Glu Val Leu Asp
1370 1375 1380

Ala Thr Leu Ile His Gln Ser Ile Thr Gly Leu Tyr Glu Thr Arg
1385 1390 1395

Ile Asp Leu Ser Gln Leu Gly Gly Asp Lys Arg Pro Ala Ala Thr
1400 1405 1410

Lys Lys Ala Gly Gln Ala Lys Lys Lys Lys Glu Phe Gly Gly Ser
1415 1420 1425

Gly Gly Gly Ser Gly Gly Gly Ser Gly Gly Gly Ser Gly Gly Gly
1430 1435 1440

Ser Gly Gly Gly Ser Gly Gly Gly Ser Gly Gly Gly Ser Gly Gly
1445 1450 1455

Gly Ser Gly Gly Gly Ser Gly Met Asn Ile Asn Asp Leu Ile Arg
1460 1465 1470

Glu Ile Lys Asn Lys Asp Tyr Thr Val Lys Leu Ser Gly Thr Asp
1475 1480 1485

Ser Asn Ser Ile Thr Gln Leu Ile Ile Arg Val Asn Asn Asp Gly
1490 1495 1500

Asn Glu Tyr Val Ile Ser Glu Ser Glu Asn Glu Ser Ile Val Glu
1505 1510 1515

Lys Phe Ile Ser Ala Phe Lys Asn Gly Trp Asn Gly Leu Ala Thr
1520 1525 1530

Thr Leu Glu Arg Ile Glu Lys Asn Phe Val Ile Thr Asp Pro Arg
1535 1540 1545

Leu Pro Asp Asn Pro Ile Ile Phe Ala Ser Asp Ser Phe Leu Gln
1550 1555 1560

Leu Thr Glu Tyr Ser Arg Glu Glu Ile Leu Gly Arg Asn Cys Arg
1565 1570 1575

Phe Leu Gln Gly Pro Glu Thr Asp Arg Ala Thr Val Arg Lys Ile
1580 1585 1590

Arg Asp Ala Ile Asp Asn Gln Thr Glu Val Thr Val Gln Leu Ile
1595 1600 1605

Asn Tyr Thr Lys Ser Gly Lys Lys Phe Trp Asn Leu Phe His Leu
1610 1615 1620

Gln Pro Met Arg Asp Gln Lys Gly Asp Val Gln Tyr Phe Ile Gly
1625 1630 1635

Val Gln Leu Asp Gly Thr Glu His Val Arg Asp Ala Ala Glu Arg
1640 1645 1650

Glu Gly Val Met Leu Ile Lys Lys Thr Ala Glu Asn Ile Asp Glu
1655 1660 1665

Ala Ala Lys Glu Leu Gly Tyr Glu Asp Glu Glu Glu Phe Tyr Asn
1670 1675 1680

Asp Met Gln Thr Ile Thr Leu Lys Ser Glu Leu Asn
1685 1690 1695

<210> 18
<211> 1699
<212> PRT
<213> Artificial Sequence

<220>
<223> CasCAID#4 (NLS-Cas9-NLS fused to AcrIIA4 with Q65/E66 deletion and an insertion of a LOV-domain flanked by Gly-Ser-Gly linkers)

<400> 18

Met Asp Tyr Lys Asp His Asp Gly Asp Tyr Lys Asp His Asp Ile Asp
1 5 10 15

Tyr Lys Asp Asp Asp Asp Lys Met Ala Pro Lys Lys Lys Arg Lys Val
20 25 30

Gly Ile His Gly Val Pro Ala Ala Asp Lys Lys Tyr Ser Ile Gly Leu
35 40 45

Asp Ile Gly Thr Asn Ser Val Gly Trp Ala Val Ile Thr Asp Glu Tyr
50 55 60

Lys Val Pro Ser Lys Lys Phe Lys Val Leu Gly Asn Thr Asp Arg His
65 70 75 80

Ser Ile Lys Lys Asn Leu Ile Gly Ala Leu Leu Phe Asp Ser Gly Glu
85 90 95

Thr Ala Glu Ala Thr Arg Leu Lys Arg Thr Ala Arg Arg Arg Tyr Thr
100 105 110

Arg Arg Lys Asn Arg Ile Cys Tyr Leu Gln Glu Ile Phe Ser Asn Glu
115 120 125

Met Ala Lys Val Asp Asp Ser Phe Phe His Arg Leu Glu Glu Ser Phe
130 135 140

Leu Val Glu Glu Asp Lys Lys His Glu Arg His Pro Ile Phe Gly Asn
145 150 155 160

Ile Val Asp Glu Val Ala Tyr His Glu Lys Tyr Pro Thr Ile Tyr His
165 170 175

Leu Arg Lys Lys Leu Val Asp Ser Thr Asp Lys Ala Asp Leu Arg Leu
180 185 190

Ile Tyr Leu Ala Leu Ala His Met Ile Lys Phe Arg Gly His Phe Leu
195 200 205

Ile Glu Gly Asp Leu Asn Pro Asp Asn Ser Asp Val Asp Lys Leu Phe
210 215 220

Ile Gln Leu Val Gln Thr Tyr Asn Gln Leu Phe Glu Glu Asn Pro Ile

```
225                 230                 235                 240

Asn Ala Ser Gly Val Asp Ala Lys Ala Ile Leu Ser Ala Arg Leu Ser
                245                 250                 255

Lys Ser Arg Arg Leu Glu Asn Leu Ile Ala Gln Leu Pro Gly Glu Lys
            260                 265                 270

Lys Asn Gly Leu Phe Gly Asn Leu Ile Ala Leu Ser Leu Gly Leu Thr
        275                 280                 285

Pro Asn Phe Lys Ser Asn Phe Asp Leu Ala Glu Asp Ala Lys Leu Gln
    290                 295                 300

Leu Ser Lys Asp Thr Tyr Asp Asp Asp Leu Asp Asn Leu Leu Ala Gln
305                 310                 315                 320

Ile Gly Asp Gln Tyr Ala Asp Leu Phe Leu Ala Ala Lys Asn Leu Ser
                325                 330                 335

Asp Ala Ile Leu Leu Ser Asp Ile Leu Arg Val Asn Thr Glu Ile Thr
            340                 345                 350

Lys Ala Pro Leu Ser Ala Ser Met Ile Lys Arg Tyr Asp Glu His His
        355                 360                 365

Gln Asp Leu Thr Leu Leu Lys Ala Leu Val Arg Gln Gln Leu Pro Glu
    370                 375                 380

Lys Tyr Lys Glu Ile Phe Phe Asp Gln Ser Lys Asn Gly Tyr Ala Gly
385                 390                 395                 400

Tyr Ile Asp Gly Gly Ala Ser Gln Glu Glu Phe Tyr Lys Phe Ile Lys
                405                 410                 415

Pro Ile Leu Glu Lys Met Asp Gly Thr Glu Glu Leu Leu Val Lys Leu
            420                 425                 430

Asn Arg Glu Asp Leu Leu Arg Lys Gln Arg Thr Phe Asp Asn Gly Ser
        435                 440                 445

Ile Pro His Gln Ile His Leu Gly Glu Leu His Ala Ile Leu Arg Arg
    450                 455                 460

Gln Glu Asp Phe Tyr Pro Phe Leu Lys Asp Asn Arg Glu Lys Ile Glu
465                 470                 475                 480
```

Lys Ile Leu Thr Phe Arg Ile Pro Tyr Tyr Val Gly Pro Leu Ala Arg
485 490 495

Gly Asn Ser Arg Phe Ala Trp Met Thr Arg Lys Ser Glu Glu Thr Ile
500 505 510

Thr Pro Trp Asn Phe Glu Glu Val Val Asp Lys Gly Ala Ser Ala Gln
515 520 525

Ser Phe Ile Glu Arg Met Thr Asn Phe Asp Lys Asn Leu Pro Asn Glu
530 535 540

Lys Val Leu Pro Lys His Ser Leu Leu Tyr Glu Tyr Phe Thr Val Tyr
545 550 555 560

Asn Glu Leu Thr Lys Val Lys Tyr Val Thr Glu Gly Met Arg Lys Pro
565 570 575

Ala Phe Leu Ser Gly Glu Gln Lys Lys Ala Ile Val Asp Leu Leu Phe
580 585 590

Lys Thr Asn Arg Lys Val Thr Val Lys Gln Leu Lys Glu Asp Tyr Phe
595 600 605

Lys Lys Ile Glu Cys Phe Asp Ser Val Glu Ile Ser Gly Val Glu Asp
610 615 620

Arg Phe Asn Ala Ser Leu Gly Thr Tyr His Asp Leu Leu Lys Ile Ile
625 630 635 640

Lys Asp Lys Asp Phe Leu Asp Asn Glu Glu Asn Glu Asp Ile Leu Glu
645 650 655

Asp Ile Val Leu Thr Leu Thr Leu Phe Glu Asp Arg Glu Met Ile Glu
660 665 670

Glu Arg Leu Lys Thr Tyr Ala His Leu Phe Asp Asp Lys Val Met Lys
675 680 685

Gln Leu Lys Arg Arg Arg Tyr Thr Gly Trp Gly Arg Leu Ser Arg Lys
690 695 700

Leu Ile Asn Gly Ile Arg Asp Lys Gln Ser Gly Lys Thr Ile Leu Asp
705 710 715 720

Phe Leu Lys Ser Asp Gly Phe Ala Asn Arg Asn Phe Met Gln Leu Ile
725 730 735

His Asp Asp Ser Leu Thr Phe Lys Glu Asp Ile Gln Lys Ala Gln Val
740 745 750

Ser Gly Gln Gly Asp Ser Leu His Glu His Ile Ala Asn Leu Ala Gly
755 760 765

Ser Pro Ala Ile Lys Lys Gly Ile Leu Gln Thr Val Lys Val Val Asp
770 775 780

Glu Leu Val Lys Val Met Gly Arg His Lys Pro Glu Asn Ile Val Ile
785 790 795 800

Glu Met Ala Arg Glu Asn Gln Thr Thr Gln Lys Gly Gln Lys Asn Ser
805 810 815

Arg Glu Arg Met Lys Arg Ile Glu Glu Gly Ile Lys Glu Leu Gly Ser
820 825 830

Gln Ile Leu Lys Glu His Pro Val Glu Asn Thr Gln Leu Gln Asn Glu
835 840 845

Lys Leu Tyr Leu Tyr Tyr Leu Gln Asn Gly Arg Asp Met Tyr Val Asp
850 855 860

Gln Glu Leu Asp Ile Asn Arg Leu Ser Asp Tyr Asp Val Asp His Ile
865 870 875 880

Val Pro Gln Ser Phe Leu Lys Asp Asp Ser Ile Asp Asn Lys Val Leu
885 890 895

Thr Arg Ser Asp Lys Asn Arg Gly Lys Ser Asp Asn Val Pro Ser Glu
900 905 910

Glu Val Val Lys Lys Met Lys Asn Tyr Trp Arg Gln Leu Leu Asn Ala
915 920 925

Lys Leu Ile Thr Gln Arg Lys Phe Asp Asn Leu Thr Lys Ala Glu Arg
930 935 940

Gly Gly Leu Ser Glu Leu Asp Lys Ala Gly Phe Ile Lys Arg Gln Leu
945 950 955 960

Val Glu Thr Arg Gln Ile Thr Lys His Val Ala Gln Ile Leu Asp Ser
965 970 975

Arg Met Asn Thr Lys Tyr Asp Glu Asn Asp Lys Leu Ile Arg Glu Val
980 985 990

Lys Val Ile Thr Leu Lys Ser Lys Leu Val Ser Asp Phe Arg Lys Asp
995 1000 1005

Phe Gln Phe Tyr Lys Val Arg Glu Ile Asn Asn Tyr His His Ala
1010 1015 1020

His Asp Ala Tyr Leu Asn Ala Val Val Gly Thr Ala Leu Ile Lys
1025 1030 1035

Lys Tyr Pro Lys Leu Glu Ser Glu Phe Val Tyr Gly Asp Tyr Lys
1040 1045 1050

Val Tyr Asp Val Arg Lys Met Ile Ala Lys Ser Glu Gln Glu Ile
1055 1060 1065

Gly Lys Ala Thr Ala Lys Tyr Phe Phe Tyr Ser Asn Ile Met Asn
1070 1075 1080

Phe Phe Lys Thr Glu Ile Thr Leu Ala Asn Gly Glu Ile Arg Lys
1085 1090 1095

Arg Pro Leu Ile Glu Thr Asn Gly Glu Thr Gly Glu Ile Val Trp
1100 1105 1110

Asp Lys Gly Arg Asp Phe Ala Thr Val Arg Lys Val Leu Ser Met
1115 1120 1125

Pro Gln Val Asn Ile Val Lys Lys Thr Glu Val Gln Thr Gly Gly
1130 1135 1140

Phe Ser Lys Glu Ser Ile Leu Pro Lys Arg Asn Ser Asp Lys Leu
1145 1150 1155

Ile Ala Arg Lys Lys Asp Trp Asp Pro Lys Lys Tyr Gly Gly Phe
1160 1165 1170

Asp Ser Pro Thr Val Ala Tyr Ser Val Leu Val Val Ala Lys Val
1175 1180 1185

Glu Lys Gly Lys Ser Lys Lys Leu Lys Ser Val Lys Glu Leu Leu
1190 1195 1200

Gly Ile Thr Ile Met Glu Arg Ser Ser Phe Glu Lys Asn Pro Ile
1205 1210 1215

Asp Phe Leu Glu Ala Lys Gly Tyr Lys Glu Val Lys Lys Asp Leu

1220 1225 1230

Ile Ile Lys Leu Pro Lys Tyr Ser Leu Phe Glu Leu Glu Asn Gly
1235 1240 1245

Arg Lys Arg Met Leu Ala Ser Ala Gly Glu Leu Gln Lys Gly Asn
1250 1255 1260

Glu Leu Ala Leu Pro Ser Lys Tyr Val Asn Phe Leu Tyr Leu Ala
1265 1270 1275

Ser His Tyr Glu Lys Leu Lys Gly Ser Pro Glu Asp Asn Glu Gln
1280 1285 1290

Lys Gln Leu Phe Val Glu Gln His Lys His Tyr Leu Asp Glu Ile
1295 1300 1305

Ile Glu Gln Ile Ser Glu Phe Ser Lys Arg Val Ile Leu Ala Asp
1310 1315 1320

Ala Asn Leu Asp Lys Val Leu Ser Ala Tyr Asn Lys His Arg Asp
1325 1330 1335

Lys Pro Ile Arg Glu Gln Ala Glu Asn Ile Ile His Leu Phe Thr
1340 1345 1350

Leu Thr Asn Leu Gly Ala Pro Ala Ala Phe Lys Tyr Phe Asp Thr
1355 1360 1365

Thr Ile Asp Arg Lys Arg Tyr Thr Ser Thr Lys Glu Val Leu Asp
1370 1375 1380

Ala Thr Leu Ile His Gln Ser Ile Thr Gly Leu Tyr Glu Thr Arg
1385 1390 1395

Ile Asp Leu Ser Gln Leu Gly Gly Asp Lys Arg Pro Ala Ala Thr
1400 1405 1410

Lys Lys Ala Gly Gln Ala Lys Lys Lys Lys Glu Phe Gly Gly Ser
1415 1420 1425

Gly Gly Gly Ser Gly Gly Gly Ser Gly Gly Gly Ser Gly Gly Gly
1430 1435 1440

Ser Gly Gly Gly Ser Gly Gly Gly Ser Gly Gly Gly Ser Gly Gly
1445 1450 1455

Gly Ser Gly Gly Gly Ser Gly Met Asn Ile Asn Asp Leu Ile Arg
1460 1465 1470

Glu Ile Lys Asn Lys Asp Tyr Thr Val Lys Leu Ser Gly Thr Asp
1475 1480 1485

Ser Asn Ser Ile Thr Gln Leu Ile Ile Arg Val Asn Asn Asp Gly
1490 1495 1500

Asn Glu Tyr Val Ile Ser Glu Ser Glu Asn Glu Ser Ile Val Glu
1505 1510 1515

Lys Phe Ile Ser Ala Phe Lys Asn Gly Trp Asn Gly Ser Gly Leu
1520 1525 1530

Ala Thr Thr Leu Glu Arg Ile Glu Lys Asn Phe Val Ile Thr Asp
1535 1540 1545

Pro Arg Leu Pro Asp Asn Pro Ile Ile Phe Ala Ser Asp Ser Phe
1550 1555 1560

Leu Gln Leu Thr Glu Tyr Ser Arg Glu Glu Ile Leu Gly Arg Asn
1565 1570 1575

Cys Arg Phe Leu Gln Gly Pro Glu Thr Asp Arg Ala Thr Val Arg
1580 1585 1590

Lys Ile Arg Asp Ala Ile Asp Asn Gln Thr Glu Val Thr Val Gln
1595 1600 1605

Leu Ile Asn Tyr Thr Lys Ser Gly Lys Lys Phe Trp Asn Leu Phe
1610 1615 1620

His Leu Gln Pro Met Arg Asp Gln Lys Gly Asp Val Gln Tyr Phe
1625 1630 1635

Ile Gly Val Gln Leu Asp Gly Thr Glu His Val Arg Asp Ala Ala
1640 1645 1650

Glu Arg Glu Gly Val Met Leu Ile Lys Lys Thr Ala Glu Asn Ile
1655 1660 1665

Asp Glu Ala Ala Lys Glu Leu Gly Ser Gly Tyr Glu Asp Glu Glu
1670 1675 1680

Glu Phe Tyr Asn Asp Met Gln Thr Ile Thr Leu Lys Ser Glu Leu
1685 1690 1695

Asn

<210> 19
<211> 1692
<212> PRT
<213> Artificial Sequence

<220>
<223> CasCAID#5 (NLS-Cas9-NLS fused to AcrIIA4 with N64/Q65/E66 deletion and an insertion of a LOV-domain bearing T406A/T407A/G528A/N538E mutations)

<400> 19

Met Asp Tyr Lys Asp His Asp Gly Asp Tyr Lys Asp His Asp Ile Asp
1 5 10 15

Tyr Lys Asp Asp Asp Asp Lys Met Ala Pro Lys Lys Lys Arg Lys Val
20 25 30

Gly Ile His Gly Val Pro Ala Ala Asp Lys Lys Tyr Ser Ile Gly Leu
35 40 45

Asp Ile Gly Thr Asn Ser Val Gly Trp Ala Val Ile Thr Asp Glu Tyr
50 55 60

Lys Val Pro Ser Lys Lys Phe Lys Val Leu Gly Asn Thr Asp Arg His
65 70 75 80

Ser Ile Lys Lys Asn Leu Ile Gly Ala Leu Leu Phe Asp Ser Gly Glu
85 90 95

Thr Ala Glu Ala Thr Arg Leu Lys Arg Thr Ala Arg Arg Arg Tyr Thr
100 105 110

Arg Arg Lys Asn Arg Ile Cys Tyr Leu Gln Glu Ile Phe Ser Asn Glu
115 120 125

Met Ala Lys Val Asp Asp Ser Phe Phe His Arg Leu Glu Glu Ser Phe
130 135 140

Leu Val Glu Glu Asp Lys Lys His Glu Arg His Pro Ile Phe Gly Asn
145 150 155 160

Ile Val Asp Glu Val Ala Tyr His Glu Lys Tyr Pro Thr Ile Tyr His
165 170 175

Leu Arg Lys Lys Leu Val Asp Ser Thr Asp Lys Ala Asp Leu Arg Leu
180 185 190

Ile Tyr Leu Ala Leu Ala His Met Ile Lys Phe Arg Gly His Phe Leu
195 200 205

Ile Glu Gly Asp Leu Asn Pro Asp Asn Ser Asp Val Asp Lys Leu Phe
210 215 220

Ile Gln Leu Val Gln Thr Tyr Asn Gln Leu Phe Glu Glu Asn Pro Ile
225 230 235 240

Asn Ala Ser Gly Val Asp Ala Lys Ala Ile Leu Ser Ala Arg Leu Ser
245 250 255

Lys Ser Arg Arg Leu Glu Asn Leu Ile Ala Gln Leu Pro Gly Glu Lys
260 265 270

Lys Asn Gly Leu Phe Gly Asn Leu Ile Ala Leu Ser Leu Gly Leu Thr
275 280 285

Pro Asn Phe Lys Ser Asn Phe Asp Leu Ala Glu Asp Ala Lys Leu Gln
290 295 300

Leu Ser Lys Asp Thr Tyr Asp Asp Asp Leu Asp Asn Leu Leu Ala Gln
305 310 315 320

Ile Gly Asp Gln Tyr Ala Asp Leu Phe Leu Ala Ala Lys Asn Leu Ser
325 330 335

Asp Ala Ile Leu Leu Ser Asp Ile Leu Arg Val Asn Thr Glu Ile Thr
340 345 350

Lys Ala Pro Leu Ser Ala Ser Met Ile Lys Arg Tyr Asp Glu His His
355 360 365

Gln Asp Leu Thr Leu Leu Lys Ala Leu Val Arg Gln Gln Leu Pro Glu
370 375 380

Lys Tyr Lys Glu Ile Phe Phe Asp Gln Ser Lys Asn Gly Tyr Ala Gly
385 390 395 400

Tyr Ile Asp Gly Gly Ala Ser Gln Glu Glu Phe Tyr Lys Phe Ile Lys
405 410 415

Pro Ile Leu Glu Lys Met Asp Gly Thr Glu Glu Leu Leu Val Lys Leu
420 425 430

Asn Arg Glu Asp Leu Leu Arg Lys Gln Arg Thr Phe Asp Asn Gly Ser

```
        435                 440                 445

Ile Pro His Gln Ile His Leu Gly Glu Leu His Ala Ile Leu Arg Arg
    450                 455                 460

Gln Glu Asp Phe Tyr Pro Phe Leu Lys Asp Asn Arg Glu Lys Ile Glu
465                 470                 475                 480

Lys Ile Leu Thr Phe Arg Ile Pro Tyr Tyr Val Gly Pro Leu Ala Arg
                485                 490                 495

Gly Asn Ser Arg Phe Ala Trp Met Thr Arg Lys Ser Glu Glu Thr Ile
            500                 505                 510

Thr Pro Trp Asn Phe Glu Glu Val Val Asp Lys Gly Ala Ser Ala Gln
        515                 520                 525

Ser Phe Ile Glu Arg Met Thr Asn Phe Asp Lys Asn Leu Pro Asn Glu
    530                 535                 540

Lys Val Leu Pro Lys His Ser Leu Leu Tyr Glu Tyr Phe Thr Val Tyr
545                 550                 555                 560

Asn Glu Leu Thr Lys Val Lys Tyr Val Thr Glu Gly Met Arg Lys Pro
                565                 570                 575

Ala Phe Leu Ser Gly Glu Gln Lys Lys Ala Ile Val Asp Leu Leu Phe
            580                 585                 590

Lys Thr Asn Arg Lys Val Thr Val Lys Gln Leu Lys Glu Asp Tyr Phe
        595                 600                 605

Lys Lys Ile Glu Cys Phe Asp Ser Val Glu Ile Ser Gly Val Glu Asp
    610                 615                 620

Arg Phe Asn Ala Ser Leu Gly Thr Tyr His Asp Leu Leu Lys Ile Ile
625                 630                 635                 640

Lys Asp Lys Asp Phe Leu Asp Asn Glu Glu Asn Glu Asp Ile Leu Glu
                645                 650                 655

Asp Ile Val Leu Thr Leu Thr Leu Phe Glu Asp Arg Glu Met Ile Glu
            660                 665                 670

Glu Arg Leu Lys Thr Tyr Ala His Leu Phe Asp Asp Lys Val Met Lys
        675                 680                 685
```

Gln Leu Lys Arg Arg Arg Tyr Thr Gly Trp Gly Arg Leu Ser Arg Lys
690 695 700

Leu Ile Asn Gly Ile Arg Asp Lys Gln Ser Gly Lys Thr Ile Leu Asp
705 710 715 720

Phe Leu Lys Ser Asp Gly Phe Ala Asn Arg Asn Phe Met Gln Leu Ile
725 730 735

His Asp Asp Ser Leu Thr Phe Lys Glu Asp Ile Gln Lys Ala Gln Val
740 745 750

Ser Gly Gln Gly Asp Ser Leu His Glu His Ile Ala Asn Leu Ala Gly
755 760 765

Ser Pro Ala Ile Lys Lys Gly Ile Leu Gln Thr Val Lys Val Val Asp
770 775 780

Glu Leu Val Lys Val Met Gly Arg His Lys Pro Glu Asn Ile Val Ile
785 790 795 800

Glu Met Ala Arg Glu Asn Gln Thr Thr Gln Lys Gly Gln Lys Asn Ser
805 810 815

Arg Glu Arg Met Lys Arg Ile Glu Glu Gly Ile Lys Glu Leu Gly Ser
820 825 830

Gln Ile Leu Lys Glu His Pro Val Glu Asn Thr Gln Leu Gln Asn Glu
835 840 845

Lys Leu Tyr Leu Tyr Tyr Leu Gln Asn Gly Arg Asp Met Tyr Val Asp
850 855 860

Gln Glu Leu Asp Ile Asn Arg Leu Ser Asp Tyr Asp Val Asp His Ile
865 870 875 880

Val Pro Gln Ser Phe Leu Lys Asp Asp Ser Ile Asp Asn Lys Val Leu
885 890 895

Thr Arg Ser Asp Lys Asn Arg Gly Lys Ser Asp Asn Val Pro Ser Glu
900 905 910

Glu Val Val Lys Lys Met Lys Asn Tyr Trp Arg Gln Leu Leu Asn Ala
915 920 925

Lys Leu Ile Thr Gln Arg Lys Phe Asp Asn Leu Thr Lys Ala Glu Arg
930 935 940

```
Gly Gly Leu Ser Glu Leu Asp Lys Ala Gly Phe Ile Lys Arg Gln Leu
945                 950                 955                 960

Val Glu Thr Arg Gln Ile Thr Lys His Val Ala Gln Ile Leu Asp Ser
                965                 970                 975

Arg Met Asn Thr Lys Tyr Asp Glu Asn Asp Lys Leu Ile Arg Glu Val
            980                 985                 990

Lys Val Ile Thr Leu Lys Ser Lys  Leu Val Ser Asp Phe  Arg Lys Asp
        995                 1000                1005

Phe Gln  Phe Tyr Lys Val Arg  Glu Ile Asn Asn Tyr  His His Ala
    1010                 1015                 1020

His Asp  Ala Tyr Leu Asn Ala  Val Val Gly Thr Ala  Leu Ile Lys
    1025                 1030                 1035

Lys Tyr  Pro Lys Leu Glu Ser  Glu Phe Val Tyr Gly  Asp Tyr Lys
    1040                 1045                 1050

Val Tyr  Asp Val Arg Lys Met  Ile Ala Lys Ser Glu  Gln Glu Ile
    1055                 1060                 1065

Gly Lys  Ala Thr Ala Lys Tyr  Phe Phe Tyr Ser Asn  Ile Met Asn
    1070                 1075                 1080

Phe Phe  Lys Thr Glu Ile Thr  Leu Ala Asn Gly Glu  Ile Arg Lys
    1085                 1090                 1095

Arg Pro  Leu Ile Glu Thr Asn  Gly Glu Thr Gly Glu  Ile Val Trp
    1100                 1105                 1110

Asp Lys  Gly Arg Asp Phe Ala  Thr Val Arg Lys Val  Leu Ser Met
    1115                 1120                 1125

Pro Gln  Val Asn Ile Val Lys  Lys Thr Glu Val Gln  Thr Gly Gly
    1130                 1135                 1140

Phe Ser  Lys Glu Ser Ile Leu  Pro Lys Arg Asn Ser  Asp Lys Leu
    1145                 1150                 1155

Ile Ala  Arg Lys Lys Asp Trp  Asp Pro Lys Lys Tyr  Gly Gly Phe
    1160                 1165                 1170

Asp Ser  Pro Thr Val Ala Tyr  Ser Val Leu Val Val  Ala Lys Val
    1175                 1180                 1185
```

Glu Lys Gly Lys Ser Lys Lys Leu Lys Ser Val Lys Glu Leu Leu
1190 1195 1200

Gly Ile Thr Ile Met Glu Arg Ser Ser Phe Glu Lys Asn Pro Ile
1205 1210 1215

Asp Phe Leu Glu Ala Lys Gly Tyr Lys Glu Val Lys Lys Asp Leu
1220 1225 1230

Ile Ile Lys Leu Pro Lys Tyr Ser Leu Phe Glu Leu Glu Asn Gly
1235 1240 1245

Arg Lys Arg Met Leu Ala Ser Ala Gly Glu Leu Gln Lys Gly Asn
1250 1255 1260

Glu Leu Ala Leu Pro Ser Lys Tyr Val Asn Phe Leu Tyr Leu Ala
1265 1270 1275

Ser His Tyr Glu Lys Leu Lys Gly Ser Pro Glu Asp Asn Glu Gln
1280 1285 1290

Lys Gln Leu Phe Val Glu Gln His Lys His Tyr Leu Asp Glu Ile
1295 1300 1305

Ile Glu Gln Ile Ser Glu Phe Ser Lys Arg Val Ile Leu Ala Asp
1310 1315 1320

Ala Asn Leu Asp Lys Val Leu Ser Ala Tyr Asn Lys His Arg Asp
1325 1330 1335

Lys Pro Ile Arg Glu Gln Ala Glu Asn Ile Ile His Leu Phe Thr
1340 1345 1350

Leu Thr Asn Leu Gly Ala Pro Ala Ala Phe Lys Tyr Phe Asp Thr
1355 1360 1365

Thr Ile Asp Arg Lys Arg Tyr Thr Ser Thr Lys Glu Val Leu Asp
1370 1375 1380

Ala Thr Leu Ile His Gln Ser Ile Thr Gly Leu Tyr Glu Thr Arg
1385 1390 1395

Ile Asp Leu Ser Gln Leu Gly Gly Asp Lys Arg Pro Ala Ala Thr
1400 1405 1410

Lys Lys Ala Gly Gln Ala Lys Lys Lys Lys Glu Phe Gly Gly Ser

1415 1420 1425

Gly Gly Gly Ser Gly Gly Gly Ser Gly Gly Gly Ser Gly Gly Gly
1430 1435 1440

Ser Gly Gly Gly Ser Gly Gly Gly Ser Gly Gly Gly Ser Gly Gly
1445 1450 1455

Gly Ser Gly Gly Gly Ser Gly Met Asn Ile Asn Asp Leu Ile Arg
1460 1465 1470

Glu Ile Lys Asn Lys Asp Tyr Thr Val Lys Leu Ser Gly Thr Asp
1475 1480 1485

Ser Asn Ser Ile Thr Gln Leu Ile Ile Arg Val Asn Asn Asp Gly
1490 1495 1500

Asn Glu Tyr Val Ile Ser Glu Ser Glu Asn Glu Ser Ile Val Glu
1505 1510 1515

Lys Phe Ile Ser Ala Phe Lys Asn Gly Trp Leu Ala Ala Ala Leu
1520 1525 1530

Glu Arg Ile Glu Lys Asn Phe Val Ile Thr Asp Pro Arg Leu Pro
1535 1540 1545

Asp Asn Pro Ile Ile Phe Ala Ser Asp Ser Phe Leu Gln Leu Thr
1550 1555 1560

Glu Tyr Ser Arg Glu Glu Ile Leu Gly Arg Asn Cys Arg Phe Leu
1565 1570 1575

Gln Gly Pro Glu Thr Asp Arg Ala Thr Val Arg Lys Ile Arg Asp
1580 1585 1590

Ala Ile Asp Asn Gln Thr Glu Val Thr Val Gln Leu Ile Asn Tyr
1595 1600 1605

Thr Lys Ser Gly Lys Lys Phe Trp Asn Leu Phe His Leu Gln Pro
1610 1615 1620

Met Arg Asp Gln Lys Gly Asp Val Gln Tyr Phe Ile Gly Val Gln
1625 1630 1635

Leu Asp Gly Thr Glu His Val Arg Asp Ala Ala Glu Arg Glu Ala
1640 1645 1650

Val Met Leu Ile Lys Lys Thr Ala Glu Glu Ile Asp Glu Ala Ala
1655 1660 1665

Lys Glu Leu Tyr Glu Asp Glu Glu Glu Phe Tyr Asn Asp Met Gln
1670 1675 1680

Thr Ile Thr Leu Lys Ser Glu Leu Asn
1685 1690

<210> 20
<211> 1692
<212> PRT
<213> Artificial Sequence

<220>
<223> CasCAID#6 (NLS-Cas9-NLS fused to AcrIIA4 with N64/Q65/E66 deletion and an insertion of a LOV-domain bearing T406A/T407A mutations)

<400> 20

Met Asp Tyr Lys Asp His Asp Gly Asp Tyr Lys Asp His Asp Ile Asp
1 5 10 15

Tyr Lys Asp Asp Asp Asp Lys Met Ala Pro Lys Lys Lys Arg Lys Val
20 25 30

Gly Ile His Gly Val Pro Ala Ala Asp Lys Lys Tyr Ser Ile Gly Leu
35 40 45

Asp Ile Gly Thr Asn Ser Val Gly Trp Ala Val Ile Thr Asp Glu Tyr
50 55 60

Lys Val Pro Ser Lys Lys Phe Lys Val Leu Gly Asn Thr Asp Arg His
65 70 75 80

Ser Ile Lys Lys Asn Leu Ile Gly Ala Leu Leu Phe Asp Ser Gly Glu
85 90 95

Thr Ala Glu Ala Thr Arg Leu Lys Arg Thr Ala Arg Arg Arg Tyr Thr
100 105 110

Arg Arg Lys Asn Arg Ile Cys Tyr Leu Gln Glu Ile Phe Ser Asn Glu
115 120 125

Met Ala Lys Val Asp Asp Ser Phe Phe His Arg Leu Glu Glu Ser Phe
130 135 140

Leu Val Glu Glu Asp Lys Lys His Glu Arg His Pro Ile Phe Gly Asn
145 150 155 160

```
Ile Val Asp Glu Val Ala Tyr His Glu Lys Tyr Pro Thr Ile Tyr His
                165                 170                 175

Leu Arg Lys Lys Leu Val Asp Ser Thr Asp Lys Ala Asp Leu Arg Leu
            180                 185                 190

Ile Tyr Leu Ala Leu Ala His Met Ile Lys Phe Arg Gly His Phe Leu
        195                 200                 205

Ile Glu Gly Asp Leu Asn Pro Asp Asn Ser Asp Val Asp Lys Leu Phe
    210                 215                 220

Ile Gln Leu Val Gln Thr Tyr Asn Gln Leu Phe Glu Glu Asn Pro Ile
225                 230                 235                 240

Asn Ala Ser Gly Val Asp Ala Lys Ala Ile Leu Ser Ala Arg Leu Ser
                245                 250                 255

Lys Ser Arg Arg Leu Glu Asn Leu Ile Ala Gln Leu Pro Gly Glu Lys
            260                 265                 270

Lys Asn Gly Leu Phe Gly Asn Leu Ile Ala Leu Ser Leu Gly Leu Thr
        275                 280                 285

Pro Asn Phe Lys Ser Asn Phe Asp Leu Ala Glu Asp Ala Lys Leu Gln
    290                 295                 300

Leu Ser Lys Asp Thr Tyr Asp Asp Asp Leu Asp Asn Leu Leu Ala Gln
305                 310                 315                 320

Ile Gly Asp Gln Tyr Ala Asp Leu Phe Leu Ala Ala Lys Asn Leu Ser
                325                 330                 335

Asp Ala Ile Leu Leu Ser Asp Ile Leu Arg Val Asn Thr Glu Ile Thr
            340                 345                 350

Lys Ala Pro Leu Ser Ala Ser Met Ile Lys Arg Tyr Asp Glu His His
        355                 360                 365

Gln Asp Leu Thr Leu Leu Lys Ala Leu Val Arg Gln Gln Leu Pro Glu
    370                 375                 380

Lys Tyr Lys Glu Ile Phe Phe Asp Gln Ser Lys Asn Gly Tyr Ala Gly
385                 390                 395                 400

Tyr Ile Asp Gly Gly Ala Ser Gln Glu Glu Phe Tyr Lys Phe Ile Lys
                405                 410                 415
```

```
Pro Ile Leu Glu Lys Met Asp Gly Thr Glu Glu Leu Leu Val Lys Leu
            420                 425                 430

Asn Arg Glu Asp Leu Leu Arg Lys Gln Arg Thr Phe Asp Asn Gly Ser
        435                 440                 445

Ile Pro His Gln Ile His Leu Gly Glu Leu His Ala Ile Leu Arg Arg
    450                 455                 460

Gln Glu Asp Phe Tyr Pro Phe Leu Lys Asp Asn Arg Glu Lys Ile Glu
465                 470                 475                 480

Lys Ile Leu Thr Phe Arg Ile Pro Tyr Tyr Val Gly Pro Leu Ala Arg
                485                 490                 495

Gly Asn Ser Arg Phe Ala Trp Met Thr Arg Lys Ser Glu Glu Thr Ile
            500                 505                 510

Thr Pro Trp Asn Phe Glu Glu Val Val Asp Lys Gly Ala Ser Ala Gln
        515                 520                 525

Ser Phe Ile Glu Arg Met Thr Asn Phe Asp Lys Asn Leu Pro Asn Glu
    530                 535                 540

Lys Val Leu Pro Lys His Ser Leu Leu Tyr Glu Tyr Phe Thr Val Tyr
545                 550                 555                 560

Asn Glu Leu Thr Lys Val Lys Tyr Val Thr Glu Gly Met Arg Lys Pro
                565                 570                 575

Ala Phe Leu Ser Gly Glu Gln Lys Lys Ala Ile Val Asp Leu Leu Phe
            580                 585                 590

Lys Thr Asn Arg Lys Val Thr Val Lys Gln Leu Lys Glu Asp Tyr Phe
        595                 600                 605

Lys Lys Ile Glu Cys Phe Asp Ser Val Glu Ile Ser Gly Val Glu Asp
    610                 615                 620

Arg Phe Asn Ala Ser Leu Gly Thr Tyr His Asp Leu Leu Lys Ile Ile
625                 630                 635                 640

Lys Asp Lys Asp Phe Leu Asp Asn Glu Glu Asn Glu Asp Ile Leu Glu
                645                 650                 655

Asp Ile Val Leu Thr Leu Thr Leu Phe Glu Asp Arg Glu Met Ile Glu
```

660 665 670

Glu Arg Leu Lys Thr Tyr Ala His Leu Phe Asp Asp Lys Val Met Lys
675 680 685

Gln Leu Lys Arg Arg Arg Tyr Thr Gly Trp Gly Arg Leu Ser Arg Lys
690 695 700

Leu Ile Asn Gly Ile Arg Asp Lys Gln Ser Gly Lys Thr Ile Leu Asp
705 710 715 720

Phe Leu Lys Ser Asp Gly Phe Ala Asn Arg Asn Phe Met Gln Leu Ile
725 730 735

His Asp Asp Ser Leu Thr Phe Lys Glu Asp Ile Gln Lys Ala Gln Val
740 745 750

Ser Gly Gln Gly Asp Ser Leu His Glu His Ile Ala Asn Leu Ala Gly
755 760 765

Ser Pro Ala Ile Lys Lys Gly Ile Leu Gln Thr Val Lys Val Val Asp
770 775 780

Glu Leu Val Lys Val Met Gly Arg His Lys Pro Glu Asn Ile Val Ile
785 790 795 800

Glu Met Ala Arg Glu Asn Gln Thr Thr Gln Lys Gly Gln Lys Asn Ser
805 810 815

Arg Glu Arg Met Lys Arg Ile Glu Glu Gly Ile Lys Glu Leu Gly Ser
820 825 830

Gln Ile Leu Lys Glu His Pro Val Glu Asn Thr Gln Leu Gln Asn Glu
835 840 845

Lys Leu Tyr Leu Tyr Tyr Leu Gln Asn Gly Arg Asp Met Tyr Val Asp
850 855 860

Gln Glu Leu Asp Ile Asn Arg Leu Ser Asp Tyr Asp Val Asp His Ile
865 870 875 880

Val Pro Gln Ser Phe Leu Lys Asp Asp Ser Ile Asp Asn Lys Val Leu
885 890 895

Thr Arg Ser Asp Lys Asn Arg Gly Lys Ser Asp Asn Val Pro Ser Glu
900 905 910

```
Glu Val Val Lys Lys Met Lys Asn Tyr Trp Arg Gln Leu Leu Asn Ala
        915                 920                 925

Lys Leu Ile Thr Gln Arg Lys Phe Asp Asn Leu Thr Lys Ala Glu Arg
    930                 935                 940

Gly Gly Leu Ser Glu Leu Asp Lys Ala Gly Phe Ile Lys Arg Gln Leu
945                 950                 955                 960

Val Glu Thr Arg Gln Ile Thr Lys His Val Ala Gln Ile Leu Asp Ser
                965                 970                 975

Arg Met Asn Thr Lys Tyr Asp Glu Asn Asp Lys Leu Ile Arg Glu Val
            980                 985                 990

Lys Val Ile Thr Leu Lys Ser Lys  Leu Val Ser Asp Phe  Arg Lys Asp
        995                 1000                 1005

Phe Gln  Phe Tyr Lys Val Arg  Glu Ile Asn Asn Tyr  His His Ala
    1010                 1015                 1020

His Asp  Ala Tyr Leu Asn Ala  Val Val Gly Thr Ala  Leu Ile Lys
    1025                 1030                 1035

Lys Tyr  Pro Lys Leu Glu Ser  Glu Phe Val Tyr Gly  Asp Tyr Lys
    1040                 1045                 1050

Val Tyr  Asp Val Arg Lys Met  Ile Ala Lys Ser Glu  Gln Glu Ile
    1055                 1060                 1065

Gly Lys  Ala Thr Ala Lys Tyr  Phe Phe Tyr Ser Asn  Ile Met Asn
    1070                 1075                 1080

Phe Phe  Lys Thr Glu Ile Thr  Leu Ala Asn Gly Glu  Ile Arg Lys
    1085                 1090                 1095

Arg Pro  Leu Ile Glu Thr Asn  Gly Glu Thr Gly Glu  Ile Val Trp
    1100                 1105                 1110

Asp Lys  Gly Arg Asp Phe Ala  Thr Val Arg Lys Val  Leu Ser Met
    1115                 1120                 1125

Pro Gln  Val Asn Ile Val Lys  Lys Thr Glu Val Gln  Thr Gly Gly
    1130                 1135                 1140

Phe Ser  Lys Glu Ser Ile Leu  Pro Lys Arg Asn Ser  Asp Lys Leu
    1145                 1150                 1155
```

Ile Ala Arg Lys Lys Asp Trp Asp Pro Lys Lys Tyr Gly Gly Phe
1160 1165 1170

Asp Ser Pro Thr Val Ala Tyr Ser Val Leu Val Val Ala Lys Val
1175 1180 1185

Glu Lys Gly Lys Ser Lys Lys Leu Lys Ser Val Lys Glu Leu Leu
1190 1195 1200

Gly Ile Thr Ile Met Glu Arg Ser Ser Phe Glu Lys Asn Pro Ile
1205 1210 1215

Asp Phe Leu Glu Ala Lys Gly Tyr Lys Glu Val Lys Lys Asp Leu
1220 1225 1230

Ile Ile Lys Leu Pro Lys Tyr Ser Leu Phe Glu Leu Glu Asn Gly
1235 1240 1245

Arg Lys Arg Met Leu Ala Ser Ala Gly Glu Leu Gln Lys Gly Asn
1250 1255 1260

Glu Leu Ala Leu Pro Ser Lys Tyr Val Asn Phe Leu Tyr Leu Ala
1265 1270 1275

Ser His Tyr Glu Lys Leu Lys Gly Ser Pro Glu Asp Asn Glu Gln
1280 1285 1290

Lys Gln Leu Phe Val Glu Gln His Lys His Tyr Leu Asp Glu Ile
1295 1300 1305

Ile Glu Gln Ile Ser Glu Phe Ser Lys Arg Val Ile Leu Ala Asp
1310 1315 1320

Ala Asn Leu Asp Lys Val Leu Ser Ala Tyr Asn Lys His Arg Asp
1325 1330 1335

Lys Pro Ile Arg Glu Gln Ala Glu Asn Ile Ile His Leu Phe Thr
1340 1345 1350

Leu Thr Asn Leu Gly Ala Pro Ala Ala Phe Lys Tyr Phe Asp Thr
1355 1360 1365

Thr Ile Asp Arg Lys Arg Tyr Thr Ser Thr Lys Glu Val Leu Asp
1370 1375 1380

Ala Thr Leu Ile His Gln Ser Ile Thr Gly Leu Tyr Glu Thr Arg
1385 1390 1395

Ile Asp Leu Ser Gln Leu Gly Gly Asp Lys Arg Pro Ala Ala Thr
1400 1405 1410

Lys Lys Ala Gly Gln Ala Lys Lys Lys Lys Glu Phe Gly Gly Ser
1415 1420 1425

Gly Gly Gly Ser Gly Gly Gly Ser Gly Gly Gly Ser Gly Gly Gly
1430 1435 1440

Ser Gly Gly Gly Ser Gly Gly Gly Ser Gly Gly Gly Ser Gly Gly
1445 1450 1455

Gly Ser Gly Gly Gly Ser Gly Met Asn Ile Asn Asp Leu Ile Arg
1460 1465 1470

Glu Ile Lys Asn Lys Asp Tyr Thr Val Lys Leu Ser Gly Thr Asp
1475 1480 1485

Ser Asn Ser Ile Thr Gln Leu Ile Ile Arg Val Asn Asn Asp Gly
1490 1495 1500

Asn Glu Tyr Val Ile Ser Glu Ser Glu Asn Glu Ser Ile Val Glu
1505 1510 1515

Lys Phe Ile Ser Ala Phe Lys Asn Gly Trp Leu Ala Ala Ala Leu
1520 1525 1530

Glu Arg Ile Glu Lys Asn Phe Val Ile Thr Asp Pro Arg Leu Pro
1535 1540 1545

Asp Asn Pro Ile Ile Phe Ala Ser Asp Ser Phe Leu Gln Leu Thr
1550 1555 1560

Glu Tyr Ser Arg Glu Glu Ile Leu Gly Arg Asn Cys Arg Phe Leu
1565 1570 1575

Gln Gly Pro Glu Thr Asp Arg Ala Thr Val Arg Lys Ile Arg Asp
1580 1585 1590

Ala Ile Asp Asn Gln Thr Glu Val Thr Val Gln Leu Ile Asn Tyr
1595 1600 1605

Thr Lys Ser Gly Lys Lys Phe Trp Asn Leu Phe His Leu Gln Pro
1610 1615 1620

Met Arg Asp Gln Lys Gly Asp Val Gln Tyr Phe Ile Gly Val Gln

1625 1630 1635

Leu Asp Gly Thr Glu His Val Arg Asp Ala Ala Glu Arg Glu Gly
1640 1645 1650

Val Met Leu Ile Lys Lys Thr Ala Glu Asn Ile Asp Glu Ala Ala
1655 1660 1665

Lys Glu Leu Tyr Glu Asp Glu Glu Glu Phe Tyr Asn Asp Met Gln
1670 1675 1680

Thr Ile Thr Leu Lys Ser Glu Leu Asn
1685 1690

<210> 21
<211> 1552
<212> PRT
<213> Artificial Sequence

<220>
<223> CasCAID#7 (NLS-Cas9-NLS fused to AcrIIA4 bearing a M77A mutation)

<400> 21

Met Asp Tyr Lys Asp His Asp Gly Asp Tyr Lys Asp His Asp Ile Asp
1 5 10 15

Tyr Lys Asp Asp Asp Asp Lys Met Ala Pro Lys Lys Lys Arg Lys Val
20 25 30

Gly Ile His Gly Val Pro Ala Ala Asp Lys Lys Tyr Ser Ile Gly Leu
35 40 45

Asp Ile Gly Thr Asn Ser Val Gly Trp Ala Val Ile Thr Asp Glu Tyr
50 55 60

Lys Val Pro Ser Lys Lys Phe Lys Val Leu Gly Asn Thr Asp Arg His
65 70 75 80

Ser Ile Lys Lys Asn Leu Ile Gly Ala Leu Leu Phe Asp Ser Gly Glu
85 90 95

Thr Ala Glu Ala Thr Arg Leu Lys Arg Thr Ala Arg Arg Arg Tyr Thr
100 105 110

Arg Arg Lys Asn Arg Ile Cys Tyr Leu Gln Glu Ile Phe Ser Asn Glu
115 120 125

Met Ala Lys Val Asp Asp Ser Phe Phe His Arg Leu Glu Glu Ser Phe
130 135 140

```
Leu Val Glu Glu Asp Lys Lys His Glu Arg His Pro Ile Phe Gly Asn
145                 150                 155                 160

Ile Val Asp Glu Val Ala Tyr His Glu Lys Tyr Pro Thr Ile Tyr His
                165                 170                 175

Leu Arg Lys Lys Leu Val Asp Ser Thr Asp Lys Ala Asp Leu Arg Leu
            180                 185                 190

Ile Tyr Leu Ala Leu Ala His Met Ile Lys Phe Arg Gly His Phe Leu
        195                 200                 205

Ile Glu Gly Asp Leu Asn Pro Asp Asn Ser Asp Val Asp Lys Leu Phe
    210                 215                 220

Ile Gln Leu Val Gln Thr Tyr Asn Gln Leu Phe Glu Glu Asn Pro Ile
225                 230                 235                 240

Asn Ala Ser Gly Val Asp Ala Lys Ala Ile Leu Ser Ala Arg Leu Ser
                245                 250                 255

Lys Ser Arg Arg Leu Glu Asn Leu Ile Ala Gln Leu Pro Gly Glu Lys
            260                 265                 270

Lys Asn Gly Leu Phe Gly Asn Leu Ile Ala Leu Ser Leu Gly Leu Thr
        275                 280                 285

Pro Asn Phe Lys Ser Asn Phe Asp Leu Ala Glu Asp Ala Lys Leu Gln
    290                 295                 300

Leu Ser Lys Asp Thr Tyr Asp Asp Asp Leu Asp Asn Leu Leu Ala Gln
305                 310                 315                 320

Ile Gly Asp Gln Tyr Ala Asp Leu Phe Leu Ala Ala Lys Asn Leu Ser
                325                 330                 335

Asp Ala Ile Leu Leu Ser Asp Ile Leu Arg Val Asn Thr Glu Ile Thr
            340                 345                 350

Lys Ala Pro Leu Ser Ala Ser Met Ile Lys Arg Tyr Asp Glu His His
        355                 360                 365

Gln Asp Leu Thr Leu Leu Lys Ala Leu Val Arg Gln Gln Leu Pro Glu
    370                 375                 380

Lys Tyr Lys Glu Ile Phe Phe Asp Gln Ser Lys Asn Gly Tyr Ala Gly
```

385 390 395 400

Tyr Ile Asp Gly Gly Ala Ser Gln Glu Glu Phe Tyr Lys Phe Ile Lys
405 410 415

Pro Ile Leu Glu Lys Met Asp Gly Thr Glu Glu Leu Leu Val Lys Leu
420 425 430

Asn Arg Glu Asp Leu Leu Arg Lys Gln Arg Thr Phe Asp Asn Gly Ser
435 440 445

Ile Pro His Gln Ile His Leu Gly Glu Leu His Ala Ile Leu Arg Arg
450 455 460

Gln Glu Asp Phe Tyr Pro Phe Leu Lys Asp Asn Arg Glu Lys Ile Glu
465 470 475 480

Lys Ile Leu Thr Phe Arg Ile Pro Tyr Tyr Val Gly Pro Leu Ala Arg
485 490 495

Gly Asn Ser Arg Phe Ala Trp Met Thr Arg Lys Ser Glu Glu Thr Ile
500 505 510

Thr Pro Trp Asn Phe Glu Glu Val Val Asp Lys Gly Ala Ser Ala Gln
515 520 525

Ser Phe Ile Glu Arg Met Thr Asn Phe Asp Lys Asn Leu Pro Asn Glu
530 535 540

Lys Val Leu Pro Lys His Ser Leu Leu Tyr Glu Tyr Phe Thr Val Tyr
545 550 555 560

Asn Glu Leu Thr Lys Val Lys Tyr Val Thr Glu Gly Met Arg Lys Pro
565 570 575

Ala Phe Leu Ser Gly Glu Gln Lys Lys Ala Ile Val Asp Leu Leu Phe
580 585 590

Lys Thr Asn Arg Lys Val Thr Val Lys Gln Leu Lys Glu Asp Tyr Phe
595 600 605

Lys Lys Ile Glu Cys Phe Asp Ser Val Glu Ile Ser Gly Val Glu Asp
610 615 620

Arg Phe Asn Ala Ser Leu Gly Thr Tyr His Asp Leu Leu Lys Ile Ile
625 630 635 640

Lys Asp Lys Asp Phe Leu Asp Asn Glu Glu Asn Glu Asp Ile Leu Glu
645 650 655

Asp Ile Val Leu Thr Leu Thr Leu Phe Glu Asp Arg Glu Met Ile Glu
660 665 670

Glu Arg Leu Lys Thr Tyr Ala His Leu Phe Asp Asp Lys Val Met Lys
675 680 685

Gln Leu Lys Arg Arg Arg Tyr Thr Gly Trp Gly Arg Leu Ser Arg Lys
690 695 700

Leu Ile Asn Gly Ile Arg Asp Lys Gln Ser Gly Lys Thr Ile Leu Asp
705 710 715 720

Phe Leu Lys Ser Asp Gly Phe Ala Asn Arg Asn Phe Met Gln Leu Ile
725 730 735

His Asp Asp Ser Leu Thr Phe Lys Glu Asp Ile Gln Lys Ala Gln Val
740 745 750

Ser Gly Gln Gly Asp Ser Leu His Glu His Ile Ala Asn Leu Ala Gly
755 760 765

Ser Pro Ala Ile Lys Lys Gly Ile Leu Gln Thr Val Lys Val Val Asp
770 775 780

Glu Leu Val Lys Val Met Gly Arg His Lys Pro Glu Asn Ile Val Ile
785 790 795 800

Glu Met Ala Arg Glu Asn Gln Thr Thr Gln Lys Gly Gln Lys Asn Ser
805 810 815

Arg Glu Arg Met Lys Arg Ile Glu Glu Gly Ile Lys Glu Leu Gly Ser
820 825 830

Gln Ile Leu Lys Glu His Pro Val Glu Asn Thr Gln Leu Gln Asn Glu
835 840 845

Lys Leu Tyr Leu Tyr Tyr Leu Gln Asn Gly Arg Asp Met Tyr Val Asp
850 855 860

Gln Glu Leu Asp Ile Asn Arg Leu Ser Asp Tyr Asp Val Asp His Ile
865 870 875 880

Val Pro Gln Ser Phe Leu Lys Asp Asp Ser Ile Asp Asn Lys Val Leu
885 890 895

Thr Arg Ser Asp Lys Asn Arg Gly Lys Ser Asp Asn Val Pro Ser Glu
900 905 910

Glu Val Val Lys Lys Met Lys Asn Tyr Trp Arg Gln Leu Leu Asn Ala
915 920 925

Lys Leu Ile Thr Gln Arg Lys Phe Asp Asn Leu Thr Lys Ala Glu Arg
930 935 940

Gly Gly Leu Ser Glu Leu Asp Lys Ala Gly Phe Ile Lys Arg Gln Leu
945 950 955 960

Val Glu Thr Arg Gln Ile Thr Lys His Val Ala Gln Ile Leu Asp Ser
965 970 975

Arg Met Asn Thr Lys Tyr Asp Glu Asn Asp Lys Leu Ile Arg Glu Val
980 985 990

Lys Val Ile Thr Leu Lys Ser Lys Leu Val Ser Asp Phe Arg Lys Asp
995 1000 1005

Phe Gln Phe Tyr Lys Val Arg Glu Ile Asn Asn Tyr His His Ala
1010 1015 1020

His Asp Ala Tyr Leu Asn Ala Val Val Gly Thr Ala Leu Ile Lys
1025 1030 1035

Lys Tyr Pro Lys Leu Glu Ser Glu Phe Val Tyr Gly Asp Tyr Lys
1040 1045 1050

Val Tyr Asp Val Arg Lys Met Ile Ala Lys Ser Glu Gln Glu Ile
1055 1060 1065

Gly Lys Ala Thr Ala Lys Tyr Phe Phe Tyr Ser Asn Ile Met Asn
1070 1075 1080

Phe Phe Lys Thr Glu Ile Thr Leu Ala Asn Gly Glu Ile Arg Lys
1085 1090 1095

Arg Pro Leu Ile Glu Thr Asn Gly Glu Thr Gly Glu Ile Val Trp
1100 1105 1110

Asp Lys Gly Arg Asp Phe Ala Thr Val Arg Lys Val Leu Ser Met
1115 1120 1125

Pro Gln Val Asn Ile Val Lys Lys Thr Glu Val Gln Thr Gly Gly
1130 1135 1140

Phe Ser Lys Glu Ser Ile Leu Pro Lys Arg Asn Ser Asp Lys Leu
1145 1150 1155

Ile Ala Arg Lys Lys Asp Trp Asp Pro Lys Lys Tyr Gly Gly Phe
1160 1165 1170

Asp Ser Pro Thr Val Ala Tyr Ser Val Leu Val Val Ala Lys Val
1175 1180 1185

Glu Lys Gly Lys Ser Lys Lys Leu Lys Ser Val Lys Glu Leu Leu
1190 1195 1200

Gly Ile Thr Ile Met Glu Arg Ser Ser Phe Glu Lys Asn Pro Ile
1205 1210 1215

Asp Phe Leu Glu Ala Lys Gly Tyr Lys Glu Val Lys Lys Asp Leu
1220 1225 1230

Ile Ile Lys Leu Pro Lys Tyr Ser Leu Phe Glu Leu Glu Asn Gly
1235 1240 1245

Arg Lys Arg Met Leu Ala Ser Ala Gly Glu Leu Gln Lys Gly Asn
1250 1255 1260

Glu Leu Ala Leu Pro Ser Lys Tyr Val Asn Phe Leu Tyr Leu Ala
1265 1270 1275

Ser His Tyr Glu Lys Leu Lys Gly Ser Pro Glu Asp Asn Glu Gln
1280 1285 1290

Lys Gln Leu Phe Val Glu Gln His Lys His Tyr Leu Asp Glu Ile
1295 1300 1305

Ile Glu Gln Ile Ser Glu Phe Ser Lys Arg Val Ile Leu Ala Asp
1310 1315 1320

Ala Asn Leu Asp Lys Val Leu Ser Ala Tyr Asn Lys His Arg Asp
1325 1330 1335

Lys Pro Ile Arg Glu Gln Ala Glu Asn Ile Ile His Leu Phe Thr
1340 1345 1350

Leu Thr Asn Leu Gly Ala Pro Ala Ala Phe Lys Tyr Phe Asp Thr
1355 1360 1365

Thr Ile Asp Arg Lys Arg Tyr Thr Ser Thr Lys Glu Val Leu Asp

1370 1375 1380

Ala Thr Leu Ile His Gln Ser Ile Thr Gly Leu Tyr Glu Thr Arg
1385 1390 1395

Ile Asp Leu Ser Gln Leu Gly Gly Asp Lys Arg Pro Ala Ala Thr
1400 1405 1410

Lys Lys Ala Gly Gln Ala Lys Lys Lys Lys Glu Phe Gly Gly Ser
1415 1420 1425

Gly Gly Gly Ser Gly Gly Gly Ser Gly Gly Gly Ser Gly Gly Gly
1430 1435 1440

Ser Gly Gly Gly Ser Gly Gly Gly Ser Gly Gly Gly Ser Gly Gly
1445 1450 1455

Gly Ser Gly Gly Gly Ser Gly Met Asn Ile Asn Asp Leu Ile Arg
1460 1465 1470

Glu Ile Lys Asn Lys Asp Tyr Thr Val Lys Leu Ser Gly Thr Asp
1475 1480 1485

Ser Asn Ser Ile Thr Gln Leu Ile Ile Arg Val Asn Asn Asp Gly
1490 1495 1500

Asn Glu Tyr Val Ile Ser Glu Ser Glu Asn Glu Ser Ile Val Glu
1505 1510 1515

Lys Phe Ile Ser Ala Phe Lys Asn Gly Trp Asn Gln Glu Tyr Glu
1520 1525 1530

Asp Glu Glu Glu Phe Tyr Asn Asp Ala Gln Thr Ile Thr Leu Lys
1535 1540 1545

Ser Glu Leu Asn
1550

<210> 22
<211> 1552
<212> PRT
<213> Artificial Sequence

<220>
<223> CasCAID#8 (NLS-Cas9-NLS fused to AcrIIA4 bearing D76A/M77A mutations)

<400> 22

Met Asp Tyr Lys Asp His Asp Gly Asp Tyr Lys Asp His Asp Ile Asp

```
1               5                   10                  15

Tyr Lys Asp Asp Asp Asp Lys Met Ala Pro Lys Lys Lys Arg Lys Val
            20                  25                  30

Gly Ile His Gly Val Pro Ala Ala Asp Lys Lys Tyr Ser Ile Gly Leu
        35                  40                  45

Asp Ile Gly Thr Asn Ser Val Gly Trp Ala Val Ile Thr Asp Glu Tyr
    50                  55                  60

Lys Val Pro Ser Lys Lys Phe Lys Val Leu Gly Asn Thr Asp Arg His
65                  70                  75                  80

Ser Ile Lys Lys Asn Leu Ile Gly Ala Leu Leu Phe Asp Ser Gly Glu
                85                  90                  95

Thr Ala Glu Ala Thr Arg Leu Lys Arg Thr Ala Arg Arg Arg Tyr Thr
            100                 105                 110

Arg Arg Lys Asn Arg Ile Cys Tyr Leu Gln Glu Ile Phe Ser Asn Glu
        115                 120                 125

Met Ala Lys Val Asp Asp Ser Phe Phe His Arg Leu Glu Glu Ser Phe
    130                 135                 140

Leu Val Glu Glu Asp Lys Lys His Glu Arg His Pro Ile Phe Gly Asn
145                 150                 155                 160

Ile Val Asp Glu Val Ala Tyr His Glu Lys Tyr Pro Thr Ile Tyr His
                165                 170                 175

Leu Arg Lys Lys Leu Val Asp Ser Thr Asp Lys Ala Asp Leu Arg Leu
            180                 185                 190

Ile Tyr Leu Ala Leu Ala His Met Ile Lys Phe Arg Gly His Phe Leu
        195                 200                 205

Ile Glu Gly Asp Leu Asn Pro Asp Asn Ser Asp Val Asp Lys Leu Phe
    210                 215                 220

Ile Gln Leu Val Gln Thr Tyr Asn Gln Leu Phe Glu Glu Asn Pro Ile
225                 230                 235                 240

Asn Ala Ser Gly Val Asp Ala Lys Ala Ile Leu Ser Ala Arg Leu Ser
                245                 250                 255
```

Lys Ser Arg Arg Leu Glu Asn Leu Ile Ala Gln Leu Pro Gly Glu Lys
260 265 270

Lys Asn Gly Leu Phe Gly Asn Leu Ile Ala Leu Ser Leu Gly Leu Thr
275 280 285

Pro Asn Phe Lys Ser Asn Phe Asp Leu Ala Glu Asp Ala Lys Leu Gln
290 295 300

Leu Ser Lys Asp Thr Tyr Asp Asp Asp Leu Asp Asn Leu Leu Ala Gln
305 310 315 320

Ile Gly Asp Gln Tyr Ala Asp Leu Phe Leu Ala Ala Lys Asn Leu Ser
325 330 335

Asp Ala Ile Leu Leu Ser Asp Ile Leu Arg Val Asn Thr Glu Ile Thr
340 345 350

Lys Ala Pro Leu Ser Ala Ser Met Ile Lys Arg Tyr Asp Glu His His
355 360 365

Gln Asp Leu Thr Leu Leu Lys Ala Leu Val Arg Gln Gln Leu Pro Glu
370 375 380

Lys Tyr Lys Glu Ile Phe Phe Asp Gln Ser Lys Asn Gly Tyr Ala Gly
385 390 395 400

Tyr Ile Asp Gly Gly Ala Ser Gln Glu Glu Phe Tyr Lys Phe Ile Lys
405 410 415

Pro Ile Leu Glu Lys Met Asp Gly Thr Glu Glu Leu Leu Val Lys Leu
420 425 430

Asn Arg Glu Asp Leu Leu Arg Lys Gln Arg Thr Phe Asp Asn Gly Ser
435 440 445

Ile Pro His Gln Ile His Leu Gly Glu Leu His Ala Ile Leu Arg Arg
450 455 460

Gln Glu Asp Phe Tyr Pro Phe Leu Lys Asp Asn Arg Glu Lys Ile Glu
465 470 475 480

Lys Ile Leu Thr Phe Arg Ile Pro Tyr Tyr Val Gly Pro Leu Ala Arg
485 490 495

Gly Asn Ser Arg Phe Ala Trp Met Thr Arg Lys Ser Glu Glu Thr Ile
500 505 510

```
Thr Pro Trp Asn Phe Glu Glu Val Val Asp Lys Gly Ala Ser Ala Gln
        515                 520                 525

Ser Phe Ile Glu Arg Met Thr Asn Phe Asp Lys Asn Leu Pro Asn Glu
    530                 535                 540

Lys Val Leu Pro Lys His Ser Leu Leu Tyr Glu Tyr Phe Thr Val Tyr
545                 550                 555                 560

Asn Glu Leu Thr Lys Val Lys Tyr Val Thr Glu Gly Met Arg Lys Pro
                565                 570                 575

Ala Phe Leu Ser Gly Glu Gln Lys Lys Ala Ile Val Asp Leu Leu Phe
            580                 585                 590

Lys Thr Asn Arg Lys Val Thr Val Lys Gln Leu Lys Glu Asp Tyr Phe
        595                 600                 605

Lys Lys Ile Glu Cys Phe Asp Ser Val Glu Ile Ser Gly Val Glu Asp
    610                 615                 620

Arg Phe Asn Ala Ser Leu Gly Thr Tyr His Asp Leu Leu Lys Ile Ile
625                 630                 635                 640

Lys Asp Lys Asp Phe Leu Asp Asn Glu Glu Asn Glu Asp Ile Leu Glu
                645                 650                 655

Asp Ile Val Leu Thr Leu Thr Leu Phe Glu Asp Arg Glu Met Ile Glu
            660                 665                 670

Glu Arg Leu Lys Thr Tyr Ala His Leu Phe Asp Asp Lys Val Met Lys
        675                 680                 685

Gln Leu Lys Arg Arg Arg Tyr Thr Gly Trp Gly Arg Leu Ser Arg Lys
    690                 695                 700

Leu Ile Asn Gly Ile Arg Asp Lys Gln Ser Gly Lys Thr Ile Leu Asp
705                 710                 715                 720

Phe Leu Lys Ser Asp Gly Phe Ala Asn Arg Asn Phe Met Gln Leu Ile
                725                 730                 735

His Asp Asp Ser Leu Thr Phe Lys Glu Asp Ile Gln Lys Ala Gln Val
            740                 745                 750

Ser Gly Gln Gly Asp Ser Leu His Glu His Ile Ala Asn Leu Ala Gly
        755                 760                 765
```

Ser Pro Ala Ile Lys Lys Gly Ile Leu Gln Thr Val Lys Val Val Asp
    770                 775                 780

Glu Leu Val Lys Val Met Gly Arg His Lys Pro Glu Asn Ile Val Ile
785                 790                 795                 800

Glu Met Ala Arg Glu Asn Gln Thr Thr Gln Lys Gly Gln Lys Asn Ser
                805                 810                 815

Arg Glu Arg Met Lys Arg Ile Glu Glu Gly Ile Lys Glu Leu Gly Ser
            820                 825                 830

Gln Ile Leu Lys Glu His Pro Val Glu Asn Thr Gln Leu Gln Asn Glu
        835                 840                 845

Lys Leu Tyr Leu Tyr Tyr Leu Gln Asn Gly Arg Asp Met Tyr Val Asp
    850                 855                 860

Gln Glu Leu Asp Ile Asn Arg Leu Ser Asp Tyr Asp Val Asp His Ile
865                 870                 875                 880

Val Pro Gln Ser Phe Leu Lys Asp Asp Ser Ile Asp Asn Lys Val Leu
                885                 890                 895

Thr Arg Ser Asp Lys Asn Arg Gly Lys Ser Asp Asn Val Pro Ser Glu
            900                 905                 910

Glu Val Val Lys Lys Met Lys Asn Tyr Trp Arg Gln Leu Leu Asn Ala
        915                 920                 925

Lys Leu Ile Thr Gln Arg Lys Phe Asp Asn Leu Thr Lys Ala Glu Arg
    930                 935                 940

Gly Gly Leu Ser Glu Leu Asp Lys Ala Gly Phe Ile Lys Arg Gln Leu
945                 950                 955                 960

Val Glu Thr Arg Gln Ile Thr Lys His Val Ala Gln Ile Leu Asp Ser
                965                 970                 975

Arg Met Asn Thr Lys Tyr Asp Glu Asn Asp Lys Leu Ile Arg Glu Val
            980                 985                 990

Lys Val Ile Thr Leu Lys Ser Lys Leu Val Ser Asp Phe Arg Lys Asp
        995                 1000                1005

Phe Gln Phe Tyr Lys Val Arg Glu Ile Asn Asn Tyr His His Ala

1010 1015 1020

His Asp Ala Tyr Leu Asn Ala Val Val Gly Thr Ala Leu Ile Lys
1025 1030 1035

Lys Tyr Pro Lys Leu Glu Ser Glu Phe Val Tyr Gly Asp Tyr Lys
1040 1045 1050

Val Tyr Asp Val Arg Lys Met Ile Ala Lys Ser Glu Gln Glu Ile
1055 1060 1065

Gly Lys Ala Thr Ala Lys Tyr Phe Phe Tyr Ser Asn Ile Met Asn
1070 1075 1080

Phe Phe Lys Thr Glu Ile Thr Leu Ala Asn Gly Glu Ile Arg Lys
1085 1090 1095

Arg Pro Leu Ile Glu Thr Asn Gly Glu Thr Gly Glu Ile Val Trp
1100 1105 1110

Asp Lys Gly Arg Asp Phe Ala Thr Val Arg Lys Val Leu Ser Met
1115 1120 1125

Pro Gln Val Asn Ile Val Lys Lys Thr Glu Val Gln Thr Gly Gly
1130 1135 1140

Phe Ser Lys Glu Ser Ile Leu Pro Lys Arg Asn Ser Asp Lys Leu
1145 1150 1155

Ile Ala Arg Lys Lys Asp Trp Asp Pro Lys Lys Tyr Gly Gly Phe
1160 1165 1170

Asp Ser Pro Thr Val Ala Tyr Ser Val Leu Val Val Ala Lys Val
1175 1180 1185

Glu Lys Gly Lys Ser Lys Lys Leu Lys Ser Val Lys Glu Leu Leu
1190 1195 1200

Gly Ile Thr Ile Met Glu Arg Ser Ser Phe Glu Lys Asn Pro Ile
1205 1210 1215

Asp Phe Leu Glu Ala Lys Gly Tyr Lys Glu Val Lys Lys Asp Leu
1220 1225 1230

Ile Ile Lys Leu Pro Lys Tyr Ser Leu Phe Glu Leu Glu Asn Gly
1235 1240 1245

Arg Lys Arg Met Leu Ala Ser Ala Gly Glu Leu Gln Lys Gly Asn
1250 1255 1260

Glu Leu Ala Leu Pro Ser Lys Tyr Val Asn Phe Leu Tyr Leu Ala
1265 1270 1275

Ser His Tyr Glu Lys Leu Lys Gly Ser Pro Glu Asp Asn Glu Gln
1280 1285 1290

Lys Gln Leu Phe Val Glu Gln His Lys His Tyr Leu Asp Glu Ile
1295 1300 1305

Ile Glu Gln Ile Ser Glu Phe Ser Lys Arg Val Ile Leu Ala Asp
1310 1315 1320

Ala Asn Leu Asp Lys Val Leu Ser Ala Tyr Asn Lys His Arg Asp
1325 1330 1335

Lys Pro Ile Arg Glu Gln Ala Glu Asn Ile Ile His Leu Phe Thr
1340 1345 1350

Leu Thr Asn Leu Gly Ala Pro Ala Ala Phe Lys Tyr Phe Asp Thr
1355 1360 1365

Thr Ile Asp Arg Lys Arg Tyr Thr Ser Thr Lys Glu Val Leu Asp
1370 1375 1380

Ala Thr Leu Ile His Gln Ser Ile Thr Gly Leu Tyr Glu Thr Arg
1385 1390 1395

Ile Asp Leu Ser Gln Leu Gly Gly Asp Lys Arg Pro Ala Ala Thr
1400 1405 1410

Lys Lys Ala Gly Gln Ala Lys Lys Lys Lys Glu Phe Gly Gly Ser
1415 1420 1425

Gly Gly Gly Ser Gly Gly Gly Ser Gly Gly Gly Ser Gly Gly Gly
1430 1435 1440

Ser Gly Gly Gly Ser Gly Gly Gly Ser Gly Gly Gly Ser Gly Gly
1445 1450 1455

Gly Ser Gly Gly Gly Ser Gly Met Asn Ile Asn Asp Leu Ile Arg
1460 1465 1470

Glu Ile Lys Asn Lys Asp Tyr Thr Val Lys Leu Ser Gly Thr Asp
1475 1480 1485

Ser Asn Ser Ile Thr Gln Leu Ile Ile Arg Val Asn Asn Asp Gly
1490 1495 1500

Asn Glu Tyr Val Ile Ser Glu Ser Glu Asn Glu Ser Ile Val Glu
1505 1510 1515

Lys Phe Ile Ser Ala Phe Lys Asn Gly Trp Asn Gln Glu Tyr Glu
1520 1525 1530

Asp Glu Glu Glu Phe Tyr Asn Ala Ala Gln Thr Ile Thr Leu Lys
1535 1540 1545

Ser Glu Leu Asn
1550

<210> 23
<211> 1552
<212> PRT
<213> Artificial Sequence

<220>
<223> CasCAID#9 (NLS-Cas9-NLS fused to AcrIIA4 bearing a D14A mutation)

<400> 23

Met Asp Tyr Lys Asp His Asp Gly Asp Tyr Lys Asp His Asp Ile Asp
1 5 10 15

Tyr Lys Asp Asp Asp Asp Lys Met Ala Pro Lys Lys Lys Arg Lys Val
20 25 30

Gly Ile His Gly Val Pro Ala Ala Asp Lys Lys Tyr Ser Ile Gly Leu
35 40 45

Asp Ile Gly Thr Asn Ser Val Gly Trp Ala Val Ile Thr Asp Glu Tyr
50 55 60

Lys Val Pro Ser Lys Lys Phe Lys Val Leu Gly Asn Thr Asp Arg His
65 70 75 80

Ser Ile Lys Lys Asn Leu Ile Gly Ala Leu Leu Phe Asp Ser Gly Glu
85 90 95

Thr Ala Glu Ala Thr Arg Leu Lys Arg Thr Ala Arg Arg Arg Tyr Thr
100 105 110

Arg Arg Lys Asn Arg Ile Cys Tyr Leu Gln Glu Ile Phe Ser Asn Glu
115 120 125

Met Ala Lys Val Asp Asp Ser Phe Phe His Arg Leu Glu Glu Ser Phe
    130                 135                 140

Leu Val Glu Glu Asp Lys Lys His Glu Arg His Pro Ile Phe Gly Asn
145                 150                 155                 160

Ile Val Asp Glu Val Ala Tyr His Glu Lys Tyr Pro Thr Ile Tyr His
                165                 170                 175

Leu Arg Lys Lys Leu Val Asp Ser Thr Asp Lys Ala Asp Leu Arg Leu
            180                 185                 190

Ile Tyr Leu Ala Leu Ala His Met Ile Lys Phe Arg Gly His Phe Leu
        195                 200                 205

Ile Glu Gly Asp Leu Asn Pro Asp Asn Ser Asp Val Asp Lys Leu Phe
    210                 215                 220

Ile Gln Leu Val Gln Thr Tyr Asn Gln Leu Phe Glu Glu Asn Pro Ile
225                 230                 235                 240

Asn Ala Ser Gly Val Asp Ala Lys Ala Ile Leu Ser Ala Arg Leu Ser
                245                 250                 255

Lys Ser Arg Arg Leu Glu Asn Leu Ile Ala Gln Leu Pro Gly Glu Lys
            260                 265                 270

Lys Asn Gly Leu Phe Gly Asn Leu Ile Ala Leu Ser Leu Gly Leu Thr
        275                 280                 285

Pro Asn Phe Lys Ser Asn Phe Asp Leu Ala Glu Asp Ala Lys Leu Gln
    290                 295                 300

Leu Ser Lys Asp Thr Tyr Asp Asp Asp Leu Asp Asn Leu Leu Ala Gln
305                 310                 315                 320

Ile Gly Asp Gln Tyr Ala Asp Leu Phe Leu Ala Ala Lys Asn Leu Ser
                325                 330                 335

Asp Ala Ile Leu Leu Ser Asp Ile Leu Arg Val Asn Thr Glu Ile Thr
            340                 345                 350

Lys Ala Pro Leu Ser Ala Ser Met Ile Lys Arg Tyr Asp Glu His His
        355                 360                 365

Gln Asp Leu Thr Leu Leu Lys Ala Leu Val Arg Gln Gln Leu Pro Glu
    370                 375                 380

Lys Tyr Lys Glu Ile Phe Phe Asp Gln Ser Lys Asn Gly Tyr Ala Gly
385 390 395 400

Tyr Ile Asp Gly Gly Ala Ser Gln Glu Glu Phe Tyr Lys Phe Ile Lys
405 410 415

Pro Ile Leu Glu Lys Met Asp Gly Thr Glu Glu Leu Leu Val Lys Leu
420 425 430

Asn Arg Glu Asp Leu Leu Arg Lys Gln Arg Thr Phe Asp Asn Gly Ser
435 440 445

Ile Pro His Gln Ile His Leu Gly Glu Leu His Ala Ile Leu Arg Arg
450 455 460

Gln Glu Asp Phe Tyr Pro Phe Leu Lys Asp Asn Arg Glu Lys Ile Glu
465 470 475 480

Lys Ile Leu Thr Phe Arg Ile Pro Tyr Tyr Val Gly Pro Leu Ala Arg
485 490 495

Gly Asn Ser Arg Phe Ala Trp Met Thr Arg Lys Ser Glu Glu Thr Ile
500 505 510

Thr Pro Trp Asn Phe Glu Glu Val Val Asp Lys Gly Ala Ser Ala Gln
515 520 525

Ser Phe Ile Glu Arg Met Thr Asn Phe Asp Lys Asn Leu Pro Asn Glu
530 535 540

Lys Val Leu Pro Lys His Ser Leu Leu Tyr Glu Tyr Phe Thr Val Tyr
545 550 555 560

Asn Glu Leu Thr Lys Val Lys Tyr Val Thr Glu Gly Met Arg Lys Pro
565 570 575

Ala Phe Leu Ser Gly Glu Gln Lys Lys Ala Ile Val Asp Leu Leu Phe
580 585 590

Lys Thr Asn Arg Lys Val Thr Val Lys Gln Leu Lys Glu Asp Tyr Phe
595 600 605

Lys Lys Ile Glu Cys Phe Asp Ser Val Glu Ile Ser Gly Val Glu Asp
610 615 620

Arg Phe Asn Ala Ser Leu Gly Thr Tyr His Asp Leu Leu Lys Ile Ile
625 630 635 640

Lys Asp Lys Asp Phe Leu Asp Asn Glu Glu Asn Glu Asp Ile Leu Glu
645 650 655

Asp Ile Val Leu Thr Leu Thr Leu Phe Glu Asp Arg Glu Met Ile Glu
660 665 670

Glu Arg Leu Lys Thr Tyr Ala His Leu Phe Asp Asp Lys Val Met Lys
675 680 685

Gln Leu Lys Arg Arg Arg Tyr Thr Gly Trp Gly Arg Leu Ser Arg Lys
690 695 700

Leu Ile Asn Gly Ile Arg Asp Lys Gln Ser Gly Lys Thr Ile Leu Asp
705 710 715 720

Phe Leu Lys Ser Asp Gly Phe Ala Asn Arg Asn Phe Met Gln Leu Ile
725 730 735

His Asp Asp Ser Leu Thr Phe Lys Glu Asp Ile Gln Lys Ala Gln Val
740 745 750

Ser Gly Gln Gly Asp Ser Leu His Glu His Ile Ala Asn Leu Ala Gly
755 760 765

Ser Pro Ala Ile Lys Lys Gly Ile Leu Gln Thr Val Lys Val Val Asp
770 775 780

Glu Leu Val Lys Val Met Gly Arg His Lys Pro Glu Asn Ile Val Ile
785 790 795 800

Glu Met Ala Arg Glu Asn Gln Thr Thr Gln Lys Gly Gln Lys Asn Ser
805 810 815

Arg Glu Arg Met Lys Arg Ile Glu Glu Gly Ile Lys Glu Leu Gly Ser
820 825 830

Gln Ile Leu Lys Glu His Pro Val Glu Asn Thr Gln Leu Gln Asn Glu
835 840 845

Lys Leu Tyr Leu Tyr Tyr Leu Gln Asn Gly Arg Asp Met Tyr Val Asp
850 855 860

Gln Glu Leu Asp Ile Asn Arg Leu Ser Asp Tyr Asp Val Asp His Ile
865 870 875 880

Val Pro Gln Ser Phe Leu Lys Asp Asp Ser Ile Asp Asn Lys Val Leu

```
                885                 890                 895

Thr Arg Ser Asp Lys Asn Arg Gly Lys Ser Asp Asn Val Pro Ser Glu
            900                 905                 910

Glu Val Val Lys Lys Met Lys Asn Tyr Trp Arg Gln Leu Leu Asn Ala
        915                 920                 925

Lys Leu Ile Thr Gln Arg Lys Phe Asp Asn Leu Thr Lys Ala Glu Arg
    930                 935                 940

Gly Gly Leu Ser Glu Leu Asp Lys Ala Gly Phe Ile Lys Arg Gln Leu
945                 950                 955                 960

Val Glu Thr Arg Gln Ile Thr Lys His Val Ala Gln Ile Leu Asp Ser
                965                 970                 975

Arg Met Asn Thr Lys Tyr Asp Glu Asn Asp Lys Leu Ile Arg Glu Val
            980                 985                 990

Lys Val Ile Thr Leu Lys Ser Lys  Leu Val Ser Asp Phe  Arg Lys Asp
        995                 1000                1005

Phe Gln  Phe Tyr Lys Val Arg  Glu Ile Asn Asn Tyr  His His Ala
    1010                1015                1020

His Asp  Ala Tyr Leu Asn Ala  Val Val Gly Thr Ala  Leu Ile Lys
    1025                1030                1035

Lys Tyr  Pro Lys Leu Glu Ser  Glu Phe Val Tyr Gly  Asp Tyr Lys
    1040                1045                1050

Val Tyr  Asp Val Arg Lys Met  Ile Ala Lys Ser Glu  Gln Glu Ile
    1055                1060                1065

Gly Lys  Ala Thr Ala Lys Tyr  Phe Phe Tyr Ser Asn  Ile Met Asn
    1070                1075                1080

Phe Phe  Lys Thr Glu Ile Thr  Leu Ala Asn Gly Glu  Ile Arg Lys
    1085                1090                1095

Arg Pro  Leu Ile Glu Thr Asn  Gly Glu Thr Gly Glu  Ile Val Trp
    1100                1105                1110

Asp Lys  Gly Arg Asp Phe Ala  Thr Val Arg Lys Val  Leu Ser Met
    1115                1120                1125
```

Pro Gln Val Asn Ile Val Lys Lys Thr Glu Val Gln Thr Gly Gly
1130 1135 1140

Phe Ser Lys Glu Ser Ile Leu Pro Lys Arg Asn Ser Asp Lys Leu
1145 1150 1155

Ile Ala Arg Lys Lys Asp Trp Asp Pro Lys Lys Tyr Gly Gly Phe
1160 1165 1170

Asp Ser Pro Thr Val Ala Tyr Ser Val Leu Val Val Ala Lys Val
1175 1180 1185

Glu Lys Gly Lys Ser Lys Lys Leu Lys Ser Val Lys Glu Leu Leu
1190 1195 1200

Gly Ile Thr Ile Met Glu Arg Ser Ser Phe Glu Lys Asn Pro Ile
1205 1210 1215

Asp Phe Leu Glu Ala Lys Gly Tyr Lys Glu Val Lys Lys Asp Leu
1220 1225 1230

Ile Ile Lys Leu Pro Lys Tyr Ser Leu Phe Glu Leu Glu Asn Gly
1235 1240 1245

Arg Lys Arg Met Leu Ala Ser Ala Gly Glu Leu Gln Lys Gly Asn
1250 1255 1260

Glu Leu Ala Leu Pro Ser Lys Tyr Val Asn Phe Leu Tyr Leu Ala
1265 1270 1275

Ser His Tyr Glu Lys Leu Lys Gly Ser Pro Glu Asp Asn Glu Gln
1280 1285 1290

Lys Gln Leu Phe Val Glu Gln His Lys His Tyr Leu Asp Glu Ile
1295 1300 1305

Ile Glu Gln Ile Ser Glu Phe Ser Lys Arg Val Ile Leu Ala Asp
1310 1315 1320

Ala Asn Leu Asp Lys Val Leu Ser Ala Tyr Asn Lys His Arg Asp
1325 1330 1335

Lys Pro Ile Arg Glu Gln Ala Glu Asn Ile Ile His Leu Phe Thr
1340 1345 1350

Leu Thr Asn Leu Gly Ala Pro Ala Ala Phe Lys Tyr Phe Asp Thr
1355 1360 1365

Thr Ile Asp Arg Lys Arg Tyr Thr Ser Thr Lys Glu Val Leu Asp
1370 1375 1380

Ala Thr Leu Ile His Gln Ser Ile Thr Gly Leu Tyr Glu Thr Arg
1385 1390 1395

Ile Asp Leu Ser Gln Leu Gly Gly Asp Lys Arg Pro Ala Ala Thr
1400 1405 1410

Lys Lys Ala Gly Gln Ala Lys Lys Lys Lys Glu Phe Gly Gly Ser
1415 1420 1425

Gly Gly Gly Ser Gly Gly Gly Ser Gly Gly Gly Ser Gly Gly Gly
1430 1435 1440

Ser Gly Gly Gly Ser Gly Gly Gly Ser Gly Gly Gly Ser Gly Gly
1445 1450 1455

Gly Ser Gly Gly Gly Ser Gly Met Asn Ile Asn Asp Leu Ile Arg
1460 1465 1470

Glu Ile Lys Asn Lys Ala Tyr Thr Val Lys Leu Ser Gly Thr Asp
1475 1480 1485

Ser Asn Ser Ile Thr Gln Leu Ile Ile Arg Val Asn Asn Asp Gly
1490 1495 1500

Asn Glu Tyr Val Ile Ser Glu Ser Glu Asn Glu Ser Ile Val Glu
1505 1510 1515

Lys Phe Ile Ser Ala Phe Lys Asn Gly Trp Asn Gln Glu Tyr Glu
1520 1525 1530

Asp Glu Glu Glu Phe Tyr Asn Asp Met Gln Thr Ile Thr Leu Lys
1535 1540 1545

Ser Glu Leu Asn
1550

<210> 24
<211> 1552
<212> PRT
<213> Artificial Sequence

<220>
<223> CasCAID#10 (NLS-Cas9-NLS fused to AcrIIA4 bearing D14A/Y15A mutation)

<400> 24

```
Met Asp Tyr Lys Asp His Asp Gly Asp Tyr Lys Asp His Asp Ile Asp
1               5                   10                  15

Tyr Lys Asp Asp Asp Asp Lys Met Ala Pro Lys Lys Lys Arg Lys Val
            20                  25                  30

Gly Ile His Gly Val Pro Ala Ala Asp Lys Lys Tyr Ser Ile Gly Leu
        35                  40                  45

Asp Ile Gly Thr Asn Ser Val Gly Trp Ala Val Ile Thr Asp Glu Tyr
    50                  55                  60

Lys Val Pro Ser Lys Lys Phe Lys Val Leu Gly Asn Thr Asp Arg His
65                  70                  75                  80

Ser Ile Lys Lys Asn Leu Ile Gly Ala Leu Leu Phe Asp Ser Gly Glu
                85                  90                  95

Thr Ala Glu Ala Thr Arg Leu Lys Arg Thr Ala Arg Arg Arg Tyr Thr
            100                 105                 110

Arg Arg Lys Asn Arg Ile Cys Tyr Leu Gln Glu Ile Phe Ser Asn Glu
        115                 120                 125

Met Ala Lys Val Asp Asp Ser Phe Phe His Arg Leu Glu Glu Ser Phe
    130                 135                 140

Leu Val Glu Glu Asp Lys Lys His Glu Arg His Pro Ile Phe Gly Asn
145                 150                 155                 160

Ile Val Asp Glu Val Ala Tyr His Glu Lys Tyr Pro Thr Ile Tyr His
                165                 170                 175

Leu Arg Lys Lys Leu Val Asp Ser Thr Asp Lys Ala Asp Leu Arg Leu
            180                 185                 190

Ile Tyr Leu Ala Leu Ala His Met Ile Lys Phe Arg Gly His Phe Leu
        195                 200                 205

Ile Glu Gly Asp Leu Asn Pro Asp Asn Ser Asp Val Asp Lys Leu Phe
    210                 215                 220

Ile Gln Leu Val Gln Thr Tyr Asn Gln Leu Phe Glu Glu Asn Pro Ile
225                 230                 235                 240

Asn Ala Ser Gly Val Asp Ala Lys Ala Ile Leu Ser Ala Arg Leu Ser
                245                 250                 255
```

Lys Ser Arg Arg Leu Glu Asn Leu Ile Ala Gln Leu Pro Gly Glu Lys
260 265 270

Lys Asn Gly Leu Phe Gly Asn Leu Ile Ala Leu Ser Leu Gly Leu Thr
275 280 285

Pro Asn Phe Lys Ser Asn Phe Asp Leu Ala Glu Asp Ala Lys Leu Gln
290 295 300

Leu Ser Lys Asp Thr Tyr Asp Asp Asp Leu Asp Asn Leu Leu Ala Gln
305 310 315 320

Ile Gly Asp Gln Tyr Ala Asp Leu Phe Leu Ala Ala Lys Asn Leu Ser
325 330 335

Asp Ala Ile Leu Leu Ser Asp Ile Leu Arg Val Asn Thr Glu Ile Thr
340 345 350

Lys Ala Pro Leu Ser Ala Ser Met Ile Lys Arg Tyr Asp Glu His His
355 360 365

Gln Asp Leu Thr Leu Leu Lys Ala Leu Val Arg Gln Gln Leu Pro Glu
370 375 380

Lys Tyr Lys Glu Ile Phe Phe Asp Gln Ser Lys Asn Gly Tyr Ala Gly
385 390 395 400

Tyr Ile Asp Gly Gly Ala Ser Gln Glu Glu Phe Tyr Lys Phe Ile Lys
405 410 415

Pro Ile Leu Glu Lys Met Asp Gly Thr Glu Glu Leu Leu Val Lys Leu
420 425 430

Asn Arg Glu Asp Leu Leu Arg Lys Gln Arg Thr Phe Asp Asn Gly Ser
435 440 445

Ile Pro His Gln Ile His Leu Gly Glu Leu His Ala Ile Leu Arg Arg
450 455 460

Gln Glu Asp Phe Tyr Pro Phe Leu Lys Asp Asn Arg Glu Lys Ile Glu
465 470 475 480

Lys Ile Leu Thr Phe Arg Ile Pro Tyr Tyr Val Gly Pro Leu Ala Arg
485 490 495

Gly Asn Ser Arg Phe Ala Trp Met Thr Arg Lys Ser Glu Glu Thr Ile

500 505 510

Thr Pro Trp Asn Phe Glu Glu Val Val Asp Lys Gly Ala Ser Ala Gln
515 520 525

Ser Phe Ile Glu Arg Met Thr Asn Phe Asp Lys Asn Leu Pro Asn Glu
530 535 540

Lys Val Leu Pro Lys His Ser Leu Leu Tyr Glu Tyr Phe Thr Val Tyr
545 550 555 560

Asn Glu Leu Thr Lys Val Lys Tyr Val Thr Glu Gly Met Arg Lys Pro
565 570 575

Ala Phe Leu Ser Gly Glu Gln Lys Lys Ala Ile Val Asp Leu Leu Phe
580 585 590

Lys Thr Asn Arg Lys Val Thr Val Lys Gln Leu Lys Glu Asp Tyr Phe
595 600 605

Lys Lys Ile Glu Cys Phe Asp Ser Val Glu Ile Ser Gly Val Glu Asp
610 615 620

Arg Phe Asn Ala Ser Leu Gly Thr Tyr His Asp Leu Leu Lys Ile Ile
625 630 635 640

Lys Asp Lys Asp Phe Leu Asp Asn Glu Glu Asn Glu Asp Ile Leu Glu
645 650 655

Asp Ile Val Leu Thr Leu Thr Leu Phe Glu Asp Arg Glu Met Ile Glu
660 665 670

Glu Arg Leu Lys Thr Tyr Ala His Leu Phe Asp Asp Lys Val Met Lys
675 680 685

Gln Leu Lys Arg Arg Arg Tyr Thr Gly Trp Gly Arg Leu Ser Arg Lys
690 695 700

Leu Ile Asn Gly Ile Arg Asp Lys Gln Ser Gly Lys Thr Ile Leu Asp
705 710 715 720

Phe Leu Lys Ser Asp Gly Phe Ala Asn Arg Asn Phe Met Gln Leu Ile
725 730 735

His Asp Asp Ser Leu Thr Phe Lys Glu Asp Ile Gln Lys Ala Gln Val
740 745 750

Ser Gly Gln Gly Asp Ser Leu His Glu His Ile Ala Asn Leu Ala Gly
755 760 765

Ser Pro Ala Ile Lys Lys Gly Ile Leu Gln Thr Val Lys Val Val Asp
770 775 780

Glu Leu Val Lys Val Met Gly Arg His Lys Pro Glu Asn Ile Val Ile
785 790 795 800

Glu Met Ala Arg Glu Asn Gln Thr Thr Gln Lys Gly Gln Lys Asn Ser
805 810 815

Arg Glu Arg Met Lys Arg Ile Glu Glu Gly Ile Lys Glu Leu Gly Ser
820 825 830

Gln Ile Leu Lys Glu His Pro Val Glu Asn Thr Gln Leu Gln Asn Glu
835 840 845

Lys Leu Tyr Leu Tyr Tyr Leu Gln Asn Gly Arg Asp Met Tyr Val Asp
850 855 860

Gln Glu Leu Asp Ile Asn Arg Leu Ser Asp Tyr Asp Val Asp His Ile
865 870 875 880

Val Pro Gln Ser Phe Leu Lys Asp Asp Ser Ile Asp Asn Lys Val Leu
885 890 895

Thr Arg Ser Asp Lys Asn Arg Gly Lys Ser Asp Asn Val Pro Ser Glu
900 905 910

Glu Val Val Lys Lys Met Lys Asn Tyr Trp Arg Gln Leu Leu Asn Ala
915 920 925

Lys Leu Ile Thr Gln Arg Lys Phe Asp Asn Leu Thr Lys Ala Glu Arg
930 935 940

Gly Gly Leu Ser Glu Leu Asp Lys Ala Gly Phe Ile Lys Arg Gln Leu
945 950 955 960

Val Glu Thr Arg Gln Ile Thr Lys His Val Ala Gln Ile Leu Asp Ser
965 970 975

Arg Met Asn Thr Lys Tyr Asp Glu Asn Asp Lys Leu Ile Arg Glu Val
980 985 990

Lys Val Ile Thr Leu Lys Ser Lys Leu Val Ser Asp Phe Arg Lys Asp
995 1000 1005

```
Phe Gln  Phe Tyr Lys Val Arg  Glu Ile Asn Asn Tyr  His His Ala
    1010                 1015                 1020

His Asp  Ala Tyr Leu Asn Ala  Val Val Gly Thr Ala  Leu Ile Lys
    1025                 1030                 1035

Lys Tyr  Pro Lys Leu Glu Ser  Glu Phe Val Tyr Gly  Asp Tyr Lys
    1040                 1045                 1050

Val Tyr  Asp Val Arg Lys Met  Ile Ala Lys Ser Glu  Gln Glu Ile
    1055                 1060                 1065

Gly Lys  Ala Thr Ala Lys Tyr  Phe Phe Tyr Ser Asn  Ile Met Asn
    1070                 1075                 1080

Phe Phe  Lys Thr Glu Ile Thr  Leu Ala Asn Gly Glu  Ile Arg Lys
    1085                 1090                 1095

Arg Pro  Leu Ile Glu Thr Asn  Gly Glu Thr Gly Glu  Ile Val Trp
    1100                 1105                 1110

Asp Lys  Gly Arg Asp Phe Ala  Thr Val Arg Lys Val  Leu Ser Met
    1115                 1120                 1125

Pro Gln  Val Asn Ile Val Lys  Lys Thr Glu Val Gln  Thr Gly Gly
    1130                 1135                 1140

Phe Ser  Lys Glu Ser Ile Leu  Pro Lys Arg Asn Ser  Asp Lys Leu
    1145                 1150                 1155

Ile Ala  Arg Lys Lys Asp Trp  Asp Pro Lys Lys Tyr  Gly Gly Phe
    1160                 1165                 1170

Asp Ser  Pro Thr Val Ala Tyr  Ser Val Leu Val Val  Ala Lys Val
    1175                 1180                 1185

Glu Lys  Gly Lys Ser Lys Lys  Leu Lys Ser Val Lys  Glu Leu Leu
    1190                 1195                 1200

Gly Ile  Thr Ile Met Glu Arg  Ser Ser Phe Glu Lys  Asn Pro Ile
    1205                 1210                 1215

Asp Phe  Leu Glu Ala Lys Gly  Tyr Lys Glu Val Lys  Lys Asp Leu
    1220                 1225                 1230

Ile Ile  Lys Leu Pro Lys Tyr  Ser Leu Phe Glu Leu  Glu Asn Gly
    1235                 1240                 1245
```

Arg Lys Arg Met Leu Ala Ser Ala Gly Glu Leu Gln Lys Gly Asn
    1250                1255                1260

Glu Leu Ala Leu Pro Ser Lys Tyr Val Asn Phe Leu Tyr Leu Ala
    1265                1270                1275

Ser His Tyr Glu Lys Leu Lys Gly Ser Pro Glu Asp Asn Glu Gln
    1280                1285                1290

Lys Gln Leu Phe Val Glu Gln His Lys His Tyr Leu Asp Glu Ile
    1295                1300                1305

Ile Glu Gln Ile Ser Glu Phe Ser Lys Arg Val Ile Leu Ala Asp
    1310                1315                1320

Ala Asn Leu Asp Lys Val Leu Ser Ala Tyr Asn Lys His Arg Asp
    1325                1330                1335

Lys Pro Ile Arg Glu Gln Ala Glu Asn Ile Ile His Leu Phe Thr
    1340                1345                1350

Leu Thr Asn Leu Gly Ala Pro Ala Ala Phe Lys Tyr Phe Asp Thr
    1355                1360                1365

Thr Ile Asp Arg Lys Arg Tyr Thr Ser Thr Lys Glu Val Leu Asp
    1370                1375                1380

Ala Thr Leu Ile His Gln Ser Ile Thr Gly Leu Tyr Glu Thr Arg
    1385                1390                1395

Ile Asp Leu Ser Gln Leu Gly Gly Asp Lys Arg Pro Ala Ala Thr
    1400                1405                1410

Lys Lys Ala Gly Gln Ala Lys Lys Lys Lys Glu Phe Gly Gly Ser
    1415                1420                1425

Gly Gly Gly Ser Gly Gly Gly Ser Gly Gly Gly Ser Gly Gly Gly
    1430                1435                1440

Ser Gly Gly Gly Ser Gly Gly Gly Ser Gly Gly Gly Ser Gly Gly
    1445                1450                1455

Gly Ser Gly Gly Gly Ser Gly Met Asn Ile Asn Asp Leu Ile Arg
    1460                1465                1470

Glu Ile Lys Asn Lys Ala Ala Thr Val Lys Leu Ser Gly Thr Asp

    1475                1480                1485

Ser Asn Ser Ile Thr Gln Leu Ile Ile Arg Val Asn Asn Asp Gly
    1490                1495                1500

Asn Glu Tyr Val Ile Ser Glu Ser Glu Asn Glu Ser Ile Val Glu
    1505                1510                1515

Lys Phe Ile Ser Ala Phe Lys Asn Gly Trp Asn Gln Glu Tyr Glu
    1520                1525                1530

Asp Glu Glu Glu Phe Tyr Asn Asp Met Gln Thr Ile Thr Leu Lys
    1535                1540                1545

Ser Glu Leu Asn
    1550

<210> 25
<211> 1552
<212> PRT
<213> Artificial Sequence

<220>
<223> CasCAID#11 (NLS-Cas9-NLS fused to AcrIIA4 bearing a N39A mutation)

<400> 25

Met Asp Tyr Lys Asp His Asp Gly Asp Tyr Lys Asp His Asp Ile Asp
1               5                   10                  15

Tyr Lys Asp Asp Asp Asp Lys Met Ala Pro Lys Lys Lys Arg Lys Val
            20                  25                  30

Gly Ile His Gly Val Pro Ala Ala Asp Lys Lys Tyr Ser Ile Gly Leu
        35                  40                  45

Asp Ile Gly Thr Asn Ser Val Gly Trp Ala Val Ile Thr Asp Glu Tyr
    50                  55                  60

Lys Val Pro Ser Lys Lys Phe Lys Val Leu Gly Asn Thr Asp Arg His
65                  70                  75                  80

Ser Ile Lys Lys Asn Leu Ile Gly Ala Leu Leu Phe Asp Ser Gly Glu
                85                  90                  95

Thr Ala Glu Ala Thr Arg Leu Lys Arg Thr Ala Arg Arg Arg Tyr Thr
            100                 105                 110

Arg Arg Lys Asn Arg Ile Cys Tyr Leu Gln Glu Ile Phe Ser Asn Glu

115 120 125

Met Ala Lys Val Asp Asp Ser Phe Phe His Arg Leu Glu Glu Ser Phe
130 135 140

Leu Val Glu Glu Asp Lys Lys His Glu Arg His Pro Ile Phe Gly Asn
145 150 155 160

Ile Val Asp Glu Val Ala Tyr His Glu Lys Tyr Pro Thr Ile Tyr His
165 170 175

Leu Arg Lys Lys Leu Val Asp Ser Thr Asp Lys Ala Asp Leu Arg Leu
180 185 190

Ile Tyr Leu Ala Leu Ala His Met Ile Lys Phe Arg Gly His Phe Leu
195 200 205

Ile Glu Gly Asp Leu Asn Pro Asp Asn Ser Asp Val Asp Lys Leu Phe
210 215 220

Ile Gln Leu Val Gln Thr Tyr Asn Gln Leu Phe Glu Glu Asn Pro Ile
225 230 235 240

Asn Ala Ser Gly Val Asp Ala Lys Ala Ile Leu Ser Ala Arg Leu Ser
245 250 255

Lys Ser Arg Arg Leu Glu Asn Leu Ile Ala Gln Leu Pro Gly Glu Lys
260 265 270

Lys Asn Gly Leu Phe Gly Asn Leu Ile Ala Leu Ser Leu Gly Leu Thr
275 280 285

Pro Asn Phe Lys Ser Asn Phe Asp Leu Ala Glu Asp Ala Lys Leu Gln
290 295 300

Leu Ser Lys Asp Thr Tyr Asp Asp Asp Leu Asp Asn Leu Leu Ala Gln
305 310 315 320

Ile Gly Asp Gln Tyr Ala Asp Leu Phe Leu Ala Ala Lys Asn Leu Ser
325 330 335

Asp Ala Ile Leu Leu Ser Asp Ile Leu Arg Val Asn Thr Glu Ile Thr
340 345 350

Lys Ala Pro Leu Ser Ala Ser Met Ile Lys Arg Tyr Asp Glu His His
355 360 365

```
Gln Asp Leu Thr Leu Leu Lys Ala Leu Val Arg Gln Gln Leu Pro Glu
    370                 375                 380

Lys Tyr Lys Glu Ile Phe Phe Asp Gln Ser Lys Asn Gly Tyr Ala Gly
385                 390                 395                 400

Tyr Ile Asp Gly Gly Ala Ser Gln Glu Glu Phe Tyr Lys Phe Ile Lys
                405                 410                 415

Pro Ile Leu Glu Lys Met Asp Gly Thr Glu Glu Leu Leu Val Lys Leu
            420                 425                 430

Asn Arg Glu Asp Leu Leu Arg Lys Gln Arg Thr Phe Asp Asn Gly Ser
        435                 440                 445

Ile Pro His Gln Ile His Leu Gly Glu Leu His Ala Ile Leu Arg Arg
    450                 455                 460

Gln Glu Asp Phe Tyr Pro Phe Leu Lys Asp Asn Arg Glu Lys Ile Glu
465                 470                 475                 480

Lys Ile Leu Thr Phe Arg Ile Pro Tyr Tyr Val Gly Pro Leu Ala Arg
                485                 490                 495

Gly Asn Ser Arg Phe Ala Trp Met Thr Arg Lys Ser Glu Glu Thr Ile
            500                 505                 510

Thr Pro Trp Asn Phe Glu Glu Val Val Asp Lys Gly Ala Ser Ala Gln
        515                 520                 525

Ser Phe Ile Glu Arg Met Thr Asn Phe Asp Lys Asn Leu Pro Asn Glu
    530                 535                 540

Lys Val Leu Pro Lys His Ser Leu Leu Tyr Glu Tyr Phe Thr Val Tyr
545                 550                 555                 560

Asn Glu Leu Thr Lys Val Lys Tyr Val Thr Glu Gly Met Arg Lys Pro
                565                 570                 575

Ala Phe Leu Ser Gly Glu Gln Lys Lys Ala Ile Val Asp Leu Leu Phe
            580                 585                 590

Lys Thr Asn Arg Lys Val Thr Val Lys Gln Leu Lys Glu Asp Tyr Phe
        595                 600                 605

Lys Lys Ile Glu Cys Phe Asp Ser Val Glu Ile Ser Gly Val Glu Asp
    610                 615                 620
```

```
Arg Phe Asn Ala Ser Leu Gly Thr Tyr His Asp Leu Leu Lys Ile Ile
625                 630                 635                 640

Lys Asp Lys Asp Phe Leu Asp Asn Glu Glu Asn Glu Asp Ile Leu Glu
                645                 650                 655

Asp Ile Val Leu Thr Leu Thr Leu Phe Glu Asp Arg Glu Met Ile Glu
            660                 665                 670

Glu Arg Leu Lys Thr Tyr Ala His Leu Phe Asp Asp Lys Val Met Lys
        675                 680                 685

Gln Leu Lys Arg Arg Arg Tyr Thr Gly Trp Gly Arg Leu Ser Arg Lys
    690                 695                 700

Leu Ile Asn Gly Ile Arg Asp Lys Gln Ser Gly Lys Thr Ile Leu Asp
705                 710                 715                 720

Phe Leu Lys Ser Asp Gly Phe Ala Asn Arg Asn Phe Met Gln Leu Ile
                725                 730                 735

His Asp Asp Ser Leu Thr Phe Lys Glu Asp Ile Gln Lys Ala Gln Val
            740                 745                 750

Ser Gly Gln Gly Asp Ser Leu His Glu His Ile Ala Asn Leu Ala Gly
        755                 760                 765

Ser Pro Ala Ile Lys Lys Gly Ile Leu Gln Thr Val Lys Val Val Asp
    770                 775                 780

Glu Leu Val Lys Val Met Gly Arg His Lys Pro Glu Asn Ile Val Ile
785                 790                 795                 800

Glu Met Ala Arg Glu Asn Gln Thr Thr Gln Lys Gly Gln Lys Asn Ser
                805                 810                 815

Arg Glu Arg Met Lys Arg Ile Glu Glu Gly Ile Lys Glu Leu Gly Ser
            820                 825                 830

Gln Ile Leu Lys Glu His Pro Val Glu Asn Thr Gln Leu Gln Asn Glu
        835                 840                 845

Lys Leu Tyr Leu Tyr Tyr Leu Gln Asn Gly Arg Asp Met Tyr Val Asp
    850                 855                 860

Gln Glu Leu Asp Ile Asn Arg Leu Ser Asp Tyr Asp Val Asp His Ile
865                 870                 875                 880
```

```
Val Pro Gln Ser Phe Leu Lys Asp Asp Ser Ile Asp Asn Lys Val Leu
                885                 890                 895

Thr Arg Ser Asp Lys Asn Arg Gly Lys Ser Asp Asn Val Pro Ser Glu
            900                 905                 910

Glu Val Val Lys Lys Met Lys Asn Tyr Trp Arg Gln Leu Leu Asn Ala
        915                 920                 925

Lys Leu Ile Thr Gln Arg Lys Phe Asp Asn Leu Thr Lys Ala Glu Arg
    930                 935                 940

Gly Gly Leu Ser Glu Leu Asp Lys Ala Gly Phe Ile Lys Arg Gln Leu
945                 950                 955                 960

Val Glu Thr Arg Gln Ile Thr Lys His Val Ala Gln Ile Leu Asp Ser
                965                 970                 975

Arg Met Asn Thr Lys Tyr Asp Glu Asn Asp Lys Leu Ile Arg Glu Val
            980                 985                 990

Lys Val Ile Thr Leu Lys Ser Lys  Leu Val Ser Asp Phe  Arg Lys Asp
        995                 1000                 1005

Phe Gln  Phe Tyr Lys Val Arg  Glu Ile Asn Asn Tyr  His His Ala
    1010                 1015                 1020

His Asp  Ala Tyr Leu Asn Ala  Val Val Gly Thr Ala  Leu Ile Lys
    1025                 1030                 1035

Lys Tyr  Pro Lys Leu Glu Ser  Glu Phe Val Tyr Gly  Asp Tyr Lys
    1040                 1045                 1050

Val Tyr  Asp Val Arg Lys Met  Ile Ala Lys Ser Glu  Gln Glu Ile
    1055                 1060                 1065

Gly Lys  Ala Thr Ala Lys Tyr  Phe Phe Tyr Ser Asn  Ile Met Asn
    1070                 1075                 1080

Phe Phe  Lys Thr Glu Ile Thr  Leu Ala Asn Gly Glu  Ile Arg Lys
    1085                 1090                 1095

Arg Pro  Leu Ile Glu Thr Asn  Gly Glu Thr Gly Glu  Ile Val Trp
    1100                 1105                 1110

Asp Lys  Gly Arg Asp Phe Ala  Thr Val Arg Lys Val  Leu Ser Met
```

1115 1120 1125

Pro Gln Val Asn Ile Val Lys Lys Thr Glu Val Gln Thr Gly Gly
1130 1135 1140

Phe Ser Lys Glu Ser Ile Leu Pro Lys Arg Asn Ser Asp Lys Leu
1145 1150 1155

Ile Ala Arg Lys Lys Asp Trp Asp Pro Lys Lys Tyr Gly Gly Phe
1160 1165 1170

Asp Ser Pro Thr Val Ala Tyr Ser Val Leu Val Val Ala Lys Val
1175 1180 1185

Glu Lys Gly Lys Ser Lys Lys Leu Lys Ser Val Lys Glu Leu Leu
1190 1195 1200

Gly Ile Thr Ile Met Glu Arg Ser Ser Phe Glu Lys Asn Pro Ile
1205 1210 1215

Asp Phe Leu Glu Ala Lys Gly Tyr Lys Glu Val Lys Lys Asp Leu
1220 1225 1230

Ile Ile Lys Leu Pro Lys Tyr Ser Leu Phe Glu Leu Glu Asn Gly
1235 1240 1245

Arg Lys Arg Met Leu Ala Ser Ala Gly Glu Leu Gln Lys Gly Asn
1250 1255 1260

Glu Leu Ala Leu Pro Ser Lys Tyr Val Asn Phe Leu Tyr Leu Ala
1265 1270 1275

Ser His Tyr Glu Lys Leu Lys Gly Ser Pro Glu Asp Asn Glu Gln
1280 1285 1290

Lys Gln Leu Phe Val Glu Gln His Lys His Tyr Leu Asp Glu Ile
1295 1300 1305

Ile Glu Gln Ile Ser Glu Phe Ser Lys Arg Val Ile Leu Ala Asp
1310 1315 1320

Ala Asn Leu Asp Lys Val Leu Ser Ala Tyr Asn Lys His Arg Asp
1325 1330 1335

Lys Pro Ile Arg Glu Gln Ala Glu Asn Ile Ile His Leu Phe Thr
1340 1345 1350

```
Leu Thr  Asn Leu Gly Ala Pro  Ala Ala Phe Lys Tyr  Phe Asp Thr
    1355                 1360                 1365

Thr Ile  Asp Arg Lys Arg Tyr  Thr Ser Thr Lys Glu  Val Leu Asp
    1370                 1375                 1380

Ala Thr  Leu Ile His Gln Ser  Ile Thr Gly Leu Tyr  Glu Thr Arg
    1385                 1390                 1395

Ile Asp  Leu Ser Gln Leu Gly  Gly Asp Lys Arg Pro  Ala Ala Thr
    1400                 1405                 1410

Lys Lys  Ala Gly Gln Ala Lys  Lys Lys Lys Glu Phe  Gly Gly Ser
    1415                 1420                 1425

Gly Gly  Gly Ser Gly Gly Gly  Ser Gly Gly Gly Ser  Gly Gly Gly
    1430                 1435                 1440

Ser Gly  Gly Gly Ser Gly Gly  Gly Ser Gly Gly Gly  Ser Gly Gly
    1445                 1450                 1455

Gly Ser  Gly Gly Gly Ser Gly  Met Asn Ile Asn Asp  Leu Ile Arg
    1460                 1465                 1470

Glu Ile  Lys Asn Lys Asp Tyr  Thr Val Lys Leu Ser  Gly Thr Asp
    1475                 1480                 1485

Ser Asn  Ser Ile Thr Gln Leu  Ile Ile Arg Val Asn  Asn Asp Gly
    1490                 1495                 1500

Ala Glu  Tyr Val Ile Ser Glu  Ser Glu Asn Glu Ser  Ile Val Glu
    1505                 1510                 1515

Lys Phe  Ile Ser Ala Phe Lys  Asn Gly Trp Asn Gln  Glu Tyr Glu
    1520                 1525                 1530

Asp Glu  Glu Glu Phe Tyr Asn  Asp Met Gln Thr Ile  Thr Leu Lys
    1535                 1540                 1545

Ser Glu  Leu Asn
    1550
```

<210>  26
<211>  1552
<212>  PRT
<213>  Artificial Sequence

<220>
<223>  CasCAID#12 (NLS-Cas9-NLS fused to AcrIIA4 bearing a G38A

mutation)

<400> 26

Met Asp Tyr Lys Asp His Asp Gly Asp Tyr Lys Asp His Asp Ile Asp
1               5                   10                  15

Tyr Lys Asp Asp Asp Asp Lys Met Ala Pro Lys Lys Lys Arg Lys Val
            20                  25                  30

Gly Ile His Gly Val Pro Ala Ala Asp Lys Lys Tyr Ser Ile Gly Leu
        35                  40                  45

Asp Ile Gly Thr Asn Ser Val Gly Trp Ala Val Ile Thr Asp Glu Tyr
    50                  55                  60

Lys Val Pro Ser Lys Lys Phe Lys Val Leu Gly Asn Thr Asp Arg His
65                  70                  75                  80

Ser Ile Lys Lys Asn Leu Ile Gly Ala Leu Leu Phe Asp Ser Gly Glu
                85                  90                  95

Thr Ala Glu Ala Thr Arg Leu Lys Arg Thr Ala Arg Arg Arg Tyr Thr
            100                 105                 110

Arg Arg Lys Asn Arg Ile Cys Tyr Leu Gln Glu Ile Phe Ser Asn Glu
        115                 120                 125

Met Ala Lys Val Asp Asp Ser Phe Phe His Arg Leu Glu Glu Ser Phe
    130                 135                 140

Leu Val Glu Glu Asp Lys Lys His Glu Arg His Pro Ile Phe Gly Asn
145                 150                 155                 160

Ile Val Asp Glu Val Ala Tyr His Glu Lys Tyr Pro Thr Ile Tyr His
                165                 170                 175

Leu Arg Lys Lys Leu Val Asp Ser Thr Asp Lys Ala Asp Leu Arg Leu
            180                 185                 190

Ile Tyr Leu Ala Leu Ala His Met Ile Lys Phe Arg Gly His Phe Leu
        195                 200                 205

Ile Glu Gly Asp Leu Asn Pro Asp Asn Ser Asp Val Asp Lys Leu Phe
    210                 215                 220

Ile Gln Leu Val Gln Thr Tyr Asn Gln Leu Phe Glu Glu Asn Pro Ile
225                 230                 235                 240

Asn Ala Ser Gly Val Asp Ala Lys Ala Ile Leu Ser Ala Arg Leu Ser
245 250 255

Lys Ser Arg Arg Leu Glu Asn Leu Ile Ala Gln Leu Pro Gly Glu Lys
260 265 270

Lys Asn Gly Leu Phe Gly Asn Leu Ile Ala Leu Ser Leu Gly Leu Thr
275 280 285

Pro Asn Phe Lys Ser Asn Phe Asp Leu Ala Glu Asp Ala Lys Leu Gln
290 295 300

Leu Ser Lys Asp Thr Tyr Asp Asp Asp Leu Asp Asn Leu Leu Ala Gln
305 310 315 320

Ile Gly Asp Gln Tyr Ala Asp Leu Phe Leu Ala Ala Lys Asn Leu Ser
325 330 335

Asp Ala Ile Leu Leu Ser Asp Ile Leu Arg Val Asn Thr Glu Ile Thr
340 345 350

Lys Ala Pro Leu Ser Ala Ser Met Ile Lys Arg Tyr Asp Glu His His
355 360 365

Gln Asp Leu Thr Leu Leu Lys Ala Leu Val Arg Gln Gln Leu Pro Glu
370 375 380

Lys Tyr Lys Glu Ile Phe Phe Asp Gln Ser Lys Asn Gly Tyr Ala Gly
385 390 395 400

Tyr Ile Asp Gly Gly Ala Ser Gln Glu Glu Phe Tyr Lys Phe Ile Lys
405 410 415

Pro Ile Leu Glu Lys Met Asp Gly Thr Glu Glu Leu Leu Val Lys Leu
420 425 430

Asn Arg Glu Asp Leu Leu Arg Lys Gln Arg Thr Phe Asp Asn Gly Ser
435 440 445

Ile Pro His Gln Ile His Leu Gly Glu Leu His Ala Ile Leu Arg Arg
450 455 460

Gln Glu Asp Phe Tyr Pro Phe Leu Lys Asp Asn Arg Glu Lys Ile Glu
465 470 475 480

Lys Ile Leu Thr Phe Arg Ile Pro Tyr Tyr Val Gly Pro Leu Ala Arg
485 490 495

```
Gly Asn Ser Arg Phe Ala Trp Met Thr Arg Lys Ser Glu Glu Thr Ile
            500                 505                 510

Thr Pro Trp Asn Phe Glu Glu Val Val Asp Lys Gly Ala Ser Ala Gln
        515                 520                 525

Ser Phe Ile Glu Arg Met Thr Asn Phe Asp Lys Asn Leu Pro Asn Glu
    530                 535                 540

Lys Val Leu Pro Lys His Ser Leu Leu Tyr Glu Tyr Phe Thr Val Tyr
545                 550                 555                 560

Asn Glu Leu Thr Lys Val Lys Tyr Val Thr Glu Gly Met Arg Lys Pro
                565                 570                 575

Ala Phe Leu Ser Gly Glu Gln Lys Lys Ala Ile Val Asp Leu Leu Phe
            580                 585                 590

Lys Thr Asn Arg Lys Val Thr Val Lys Gln Leu Lys Glu Asp Tyr Phe
        595                 600                 605

Lys Lys Ile Glu Cys Phe Asp Ser Val Glu Ile Ser Gly Val Glu Asp
    610                 615                 620

Arg Phe Asn Ala Ser Leu Gly Thr Tyr His Asp Leu Leu Lys Ile Ile
625                 630                 635                 640

Lys Asp Lys Asp Phe Leu Asp Asn Glu Glu Asn Glu Asp Ile Leu Glu
                645                 650                 655

Asp Ile Val Leu Thr Leu Thr Leu Phe Glu Asp Arg Glu Met Ile Glu
            660                 665                 670

Glu Arg Leu Lys Thr Tyr Ala His Leu Phe Asp Asp Lys Val Met Lys
        675                 680                 685

Gln Leu Lys Arg Arg Arg Tyr Thr Gly Trp Gly Arg Leu Ser Arg Lys
    690                 695                 700

Leu Ile Asn Gly Ile Arg Asp Lys Gln Ser Gly Lys Thr Ile Leu Asp
705                 710                 715                 720

Phe Leu Lys Ser Asp Gly Phe Ala Asn Arg Asn Phe Met Gln Leu Ile
                725                 730                 735

His Asp Asp Ser Leu Thr Phe Lys Glu Asp Ile Gln Lys Ala Gln Val
```

740 745 750

Ser Gly Gln Gly Asp Ser Leu His Glu His Ile Ala Asn Leu Ala Gly
755 760 765

Ser Pro Ala Ile Lys Lys Gly Ile Leu Gln Thr Val Lys Val Val Asp
770 775 780

Glu Leu Val Lys Val Met Gly Arg His Lys Pro Glu Asn Ile Val Ile
785 790 795 800

Glu Met Ala Arg Glu Asn Gln Thr Thr Gln Lys Gly Gln Lys Asn Ser
805 810 815

Arg Glu Arg Met Lys Arg Ile Glu Glu Gly Ile Lys Glu Leu Gly Ser
820 825 830

Gln Ile Leu Lys Glu His Pro Val Glu Asn Thr Gln Leu Gln Asn Glu
835 840 845

Lys Leu Tyr Leu Tyr Tyr Leu Gln Asn Gly Arg Asp Met Tyr Val Asp
850 855 860

Gln Glu Leu Asp Ile Asn Arg Leu Ser Asp Tyr Asp Val Asp His Ile
865 870 875 880

Val Pro Gln Ser Phe Leu Lys Asp Asp Ser Ile Asp Asn Lys Val Leu
885 890 895

Thr Arg Ser Asp Lys Asn Arg Gly Lys Ser Asp Asn Val Pro Ser Glu
900 905 910

Glu Val Val Lys Lys Met Lys Asn Tyr Trp Arg Gln Leu Leu Asn Ala
915 920 925

Lys Leu Ile Thr Gln Arg Lys Phe Asp Asn Leu Thr Lys Ala Glu Arg
930 935 940

Gly Gly Leu Ser Glu Leu Asp Lys Ala Gly Phe Ile Lys Arg Gln Leu
945 950 955 960

Val Glu Thr Arg Gln Ile Thr Lys His Val Ala Gln Ile Leu Asp Ser
965 970 975

Arg Met Asn Thr Lys Tyr Asp Glu Asn Asp Lys Leu Ile Arg Glu Val
980 985 990

Lys Val Ile Thr Leu Lys Ser Lys Leu Val Ser Asp Phe Arg Lys Asp
995 1000 1005

Phe Gln Phe Tyr Lys Val Arg Glu Ile Asn Asn Tyr His His Ala
1010 1015 1020

His Asp Ala Tyr Leu Asn Ala Val Val Gly Thr Ala Leu Ile Lys
1025 1030 1035

Lys Tyr Pro Lys Leu Glu Ser Glu Phe Val Tyr Gly Asp Tyr Lys
1040 1045 1050

Val Tyr Asp Val Arg Lys Met Ile Ala Lys Ser Glu Gln Glu Ile
1055 1060 1065

Gly Lys Ala Thr Ala Lys Tyr Phe Phe Tyr Ser Asn Ile Met Asn
1070 1075 1080

Phe Phe Lys Thr Glu Ile Thr Leu Ala Asn Gly Glu Ile Arg Lys
1085 1090 1095

Arg Pro Leu Ile Glu Thr Asn Gly Glu Thr Gly Glu Ile Val Trp
1100 1105 1110

Asp Lys Gly Arg Asp Phe Ala Thr Val Arg Lys Val Leu Ser Met
1115 1120 1125

Pro Gln Val Asn Ile Val Lys Lys Thr Glu Val Gln Thr Gly Gly
1130 1135 1140

Phe Ser Lys Glu Ser Ile Leu Pro Lys Arg Asn Ser Asp Lys Leu
1145 1150 1155

Ile Ala Arg Lys Lys Asp Trp Asp Pro Lys Lys Tyr Gly Gly Phe
1160 1165 1170

Asp Ser Pro Thr Val Ala Tyr Ser Val Leu Val Val Ala Lys Val
1175 1180 1185

Glu Lys Gly Lys Ser Lys Lys Leu Lys Ser Val Lys Glu Leu Leu
1190 1195 1200

Gly Ile Thr Ile Met Glu Arg Ser Ser Phe Glu Lys Asn Pro Ile
1205 1210 1215

Asp Phe Leu Glu Ala Lys Gly Tyr Lys Glu Val Lys Lys Asp Leu
1220 1225 1230

Ile Ile Lys Leu Pro Lys Tyr Ser Leu Phe Glu Leu Glu Asn Gly
1235 1240 1245

Arg Lys Arg Met Leu Ala Ser Ala Gly Glu Leu Gln Lys Gly Asn
1250 1255 1260

Glu Leu Ala Leu Pro Ser Lys Tyr Val Asn Phe Leu Tyr Leu Ala
1265 1270 1275

Ser His Tyr Glu Lys Leu Lys Gly Ser Pro Glu Asp Asn Glu Gln
1280 1285 1290

Lys Gln Leu Phe Val Glu Gln His Lys His Tyr Leu Asp Glu Ile
1295 1300 1305

Ile Glu Gln Ile Ser Glu Phe Ser Lys Arg Val Ile Leu Ala Asp
1310 1315 1320

Ala Asn Leu Asp Lys Val Leu Ser Ala Tyr Asn Lys His Arg Asp
1325 1330 1335

Lys Pro Ile Arg Glu Gln Ala Glu Asn Ile Ile His Leu Phe Thr
1340 1345 1350

Leu Thr Asn Leu Gly Ala Pro Ala Ala Phe Lys Tyr Phe Asp Thr
1355 1360 1365

Thr Ile Asp Arg Lys Arg Tyr Thr Ser Thr Lys Glu Val Leu Asp
1370 1375 1380

Ala Thr Leu Ile His Gln Ser Ile Thr Gly Leu Tyr Glu Thr Arg
1385 1390 1395

Ile Asp Leu Ser Gln Leu Gly Gly Asp Lys Arg Pro Ala Ala Thr
1400 1405 1410

Lys Lys Ala Gly Gln Ala Lys Lys Lys Lys Glu Phe Gly Gly Ser
1415 1420 1425

Gly Gly Gly Ser Gly Gly Gly Ser Gly Gly Gly Ser Gly Gly Gly
1430 1435 1440

Ser Gly Gly Gly Ser Gly Gly Gly Ser Gly Gly Gly Ser Gly Gly
1445 1450 1455

Gly Ser Gly Gly Gly Ser Gly Met Asn Ile Asn Asp Leu Ile Arg
1460 1465 1470

Glu Ile Lys Asn Lys Asp Tyr Thr Val Lys Leu Ser Gly Thr Asp
1475 1480 1485

Ser Asn Ser Ile Thr Gln Leu Ile Ile Arg Val Asn Asn Asp Ala
1490 1495 1500

Asn Glu Tyr Val Ile Ser Glu Ser Glu Asn Glu Ser Ile Val Glu
1505 1510 1515

Lys Phe Ile Ser Ala Phe Lys Asn Gly Trp Asn Gln Glu Tyr Glu
1520 1525 1530

Asp Glu Glu Glu Phe Tyr Asn Asp Met Gln Thr Ile Thr Leu Lys
1535 1540 1545

Ser Glu Leu Asn
1550

<210> 27
<211> 1552
<212> PRT
<213> Artificial Sequence

<220>
<223> CasCAID#13 (NLS-Cas9-NLS fused to AcrIIA4 bearing D37A/G38A mutation)

<400> 27

Met Asp Tyr Lys Asp His Asp Gly Asp Tyr Lys Asp His Asp Ile Asp
1 5 10 15

Tyr Lys Asp Asp Asp Asp Lys Met Ala Pro Lys Lys Lys Arg Lys Val
20 25 30

Gly Ile His Gly Val Pro Ala Ala Asp Lys Lys Tyr Ser Ile Gly Leu
35 40 45

Asp Ile Gly Thr Asn Ser Val Gly Trp Ala Val Ile Thr Asp Glu Tyr
50 55 60

Lys Val Pro Ser Lys Lys Phe Lys Val Leu Gly Asn Thr Asp Arg His
65 70 75 80

Ser Ile Lys Lys Asn Leu Ile Gly Ala Leu Leu Phe Asp Ser Gly Glu
85 90 95

Thr Ala Glu Ala Thr Arg Leu Lys Arg Thr Ala Arg Arg Arg Tyr Thr
100 105 110

```
Arg Arg Lys Asn Arg Ile Cys Tyr Leu Gln Glu Ile Phe Ser Asn Glu
        115                 120                 125

Met Ala Lys Val Asp Asp Ser Phe Phe His Arg Leu Glu Glu Ser Phe
    130                 135                 140

Leu Val Glu Glu Asp Lys Lys His Glu Arg His Pro Ile Phe Gly Asn
145                 150                 155                 160

Ile Val Asp Glu Val Ala Tyr His Glu Lys Tyr Pro Thr Ile Tyr His
                165                 170                 175

Leu Arg Lys Lys Leu Val Asp Ser Thr Asp Lys Ala Asp Leu Arg Leu
            180                 185                 190

Ile Tyr Leu Ala Leu Ala His Met Ile Lys Phe Arg Gly His Phe Leu
        195                 200                 205

Ile Glu Gly Asp Leu Asn Pro Asp Asn Ser Asp Val Asp Lys Leu Phe
    210                 215                 220

Ile Gln Leu Val Gln Thr Tyr Asn Gln Leu Phe Glu Glu Asn Pro Ile
225                 230                 235                 240

Asn Ala Ser Gly Val Asp Ala Lys Ala Ile Leu Ser Ala Arg Leu Ser
                245                 250                 255

Lys Ser Arg Arg Leu Glu Asn Leu Ile Ala Gln Leu Pro Gly Glu Lys
            260                 265                 270

Lys Asn Gly Leu Phe Gly Asn Leu Ile Ala Leu Ser Leu Gly Leu Thr
        275                 280                 285

Pro Asn Phe Lys Ser Asn Phe Asp Leu Ala Glu Asp Ala Lys Leu Gln
    290                 295                 300

Leu Ser Lys Asp Thr Tyr Asp Asp Asp Leu Asp Asn Leu Leu Ala Gln
305                 310                 315                 320

Ile Gly Asp Gln Tyr Ala Asp Leu Phe Leu Ala Ala Lys Asn Leu Ser
                325                 330                 335

Asp Ala Ile Leu Leu Ser Asp Ile Leu Arg Val Asn Thr Glu Ile Thr
            340                 345                 350

Lys Ala Pro Leu Ser Ala Ser Met Ile Lys Arg Tyr Asp Glu His His
```

```
            355                 360                 365

Gln Asp Leu Thr Leu Leu Lys Ala Leu Val Arg Gln Gln Leu Pro Glu
        370                 375                 380

Lys Tyr Lys Glu Ile Phe Phe Asp Gln Ser Lys Asn Gly Tyr Ala Gly
385                 390                 395                 400

Tyr Ile Asp Gly Gly Ala Ser Gln Glu Glu Phe Tyr Lys Phe Ile Lys
                405                 410                 415

Pro Ile Leu Glu Lys Met Asp Gly Thr Glu Glu Leu Leu Val Lys Leu
            420                 425                 430

Asn Arg Glu Asp Leu Leu Arg Lys Gln Arg Thr Phe Asp Asn Gly Ser
        435                 440                 445

Ile Pro His Gln Ile His Leu Gly Glu Leu His Ala Ile Leu Arg Arg
    450                 455                 460

Gln Glu Asp Phe Tyr Pro Phe Leu Lys Asp Asn Arg Glu Lys Ile Glu
465                 470                 475                 480

Lys Ile Leu Thr Phe Arg Ile Pro Tyr Tyr Val Gly Pro Leu Ala Arg
                485                 490                 495

Gly Asn Ser Arg Phe Ala Trp Met Thr Arg Lys Ser Glu Glu Thr Ile
            500                 505                 510

Thr Pro Trp Asn Phe Glu Glu Val Val Asp Lys Gly Ala Ser Ala Gln
        515                 520                 525

Ser Phe Ile Glu Arg Met Thr Asn Phe Asp Lys Asn Leu Pro Asn Glu
    530                 535                 540

Lys Val Leu Pro Lys His Ser Leu Leu Tyr Glu Tyr Phe Thr Val Tyr
545                 550                 555                 560

Asn Glu Leu Thr Lys Val Lys Tyr Val Thr Glu Gly Met Arg Lys Pro
                565                 570                 575

Ala Phe Leu Ser Gly Glu Gln Lys Lys Ala Ile Val Asp Leu Leu Phe
            580                 585                 590

Lys Thr Asn Arg Lys Val Thr Val Lys Gln Leu Lys Glu Asp Tyr Phe
        595                 600                 605
```

Lys Lys Ile Glu Cys Phe Asp Ser Val Glu Ile Ser Gly Val Glu Asp
    610                 615                 620

Arg Phe Asn Ala Ser Leu Gly Thr Tyr His Asp Leu Leu Lys Ile Ile
625                 630                 635                 640

Lys Asp Lys Asp Phe Leu Asp Asn Glu Glu Asn Glu Asp Ile Leu Glu
                645                 650                 655

Asp Ile Val Leu Thr Leu Thr Leu Phe Glu Asp Arg Glu Met Ile Glu
            660                 665                 670

Glu Arg Leu Lys Thr Tyr Ala His Leu Phe Asp Asp Lys Val Met Lys
        675                 680                 685

Gln Leu Lys Arg Arg Arg Tyr Thr Gly Trp Gly Arg Leu Ser Arg Lys
    690                 695                 700

Leu Ile Asn Gly Ile Arg Asp Lys Gln Ser Gly Lys Thr Ile Leu Asp
705                 710                 715                 720

Phe Leu Lys Ser Asp Gly Phe Ala Asn Arg Asn Phe Met Gln Leu Ile
                725                 730                 735

His Asp Asp Ser Leu Thr Phe Lys Glu Asp Ile Gln Lys Ala Gln Val
            740                 745                 750

Ser Gly Gln Gly Asp Ser Leu His Glu His Ile Ala Asn Leu Ala Gly
        755                 760                 765

Ser Pro Ala Ile Lys Lys Gly Ile Leu Gln Thr Val Lys Val Val Asp
    770                 775                 780

Glu Leu Val Lys Val Met Gly Arg His Lys Pro Glu Asn Ile Val Ile
785                 790                 795                 800

Glu Met Ala Arg Glu Asn Gln Thr Thr Gln Lys Gly Gln Lys Asn Ser
                805                 810                 815

Arg Glu Arg Met Lys Arg Ile Glu Glu Gly Ile Lys Glu Leu Gly Ser
            820                 825                 830

Gln Ile Leu Lys Glu His Pro Val Glu Asn Thr Gln Leu Gln Asn Glu
        835                 840                 845

Lys Leu Tyr Leu Tyr Tyr Leu Gln Asn Gly Arg Asp Met Tyr Val Asp
    850                 855                 860

```
Gln Glu Leu Asp Ile Asn Arg Leu Ser Asp Tyr Asp Val Asp His Ile
865                 870                 875                 880

Val Pro Gln Ser Phe Leu Lys Asp Asp Ser Ile Asp Asn Lys Val Leu
                885                 890                 895

Thr Arg Ser Asp Lys Asn Arg Gly Lys Ser Asp Asn Val Pro Ser Glu
            900                 905                 910

Glu Val Val Lys Lys Met Lys Asn Tyr Trp Arg Gln Leu Leu Asn Ala
        915                 920                 925

Lys Leu Ile Thr Gln Arg Lys Phe Asp Asn Leu Thr Lys Ala Glu Arg
    930                 935                 940

Gly Gly Leu Ser Glu Leu Asp Lys Ala Gly Phe Ile Lys Arg Gln Leu
945                 950                 955                 960

Val Glu Thr Arg Gln Ile Thr Lys His Val Ala Gln Ile Leu Asp Ser
                965                 970                 975

Arg Met Asn Thr Lys Tyr Asp Glu Asn Asp Lys Leu Ile Arg Glu Val
            980                 985                 990

Lys Val Ile Thr Leu Lys Ser Lys  Leu Val Ser Asp Phe  Arg Lys Asp
        995                 1000                1005

Phe Gln  Phe Tyr Lys Val Arg  Glu Ile Asn Asn Tyr  His His Ala
    1010                 1015                 1020

His Asp  Ala Tyr Leu Asn Ala  Val Val Gly Thr Ala  Leu Ile Lys
    1025                 1030                 1035

Lys Tyr  Pro Lys Leu Glu Ser  Glu Phe Val Tyr Gly  Asp Tyr Lys
    1040                 1045                 1050

Val Tyr  Asp Val Arg Lys Met  Ile Ala Lys Ser Glu  Gln Glu Ile
    1055                 1060                 1065

Gly Lys  Ala Thr Ala Lys Tyr  Phe Phe Tyr Ser Asn  Ile Met Asn
    1070                 1075                 1080

Phe Phe  Lys Thr Glu Ile Thr  Leu Ala Asn Gly Glu  Ile Arg Lys
    1085                 1090                 1095

Arg Pro  Leu Ile Glu Thr Asn  Gly Glu Thr Gly Glu  Ile Val Trp
    1100                 1105                 1110
```

Asp Lys Gly Arg Asp Phe Ala Thr Val Arg Lys Val Leu Ser Met
1115 1120 1125

Pro Gln Val Asn Ile Val Lys Lys Thr Glu Val Gln Thr Gly Gly
1130 1135 1140

Phe Ser Lys Glu Ser Ile Leu Pro Lys Arg Asn Ser Asp Lys Leu
1145 1150 1155

Ile Ala Arg Lys Lys Asp Trp Asp Pro Lys Lys Tyr Gly Gly Phe
1160 1165 1170

Asp Ser Pro Thr Val Ala Tyr Ser Val Leu Val Val Ala Lys Val
1175 1180 1185

Glu Lys Gly Lys Ser Lys Lys Leu Lys Ser Val Lys Glu Leu Leu
1190 1195 1200

Gly Ile Thr Ile Met Glu Arg Ser Ser Phe Glu Lys Asn Pro Ile
1205 1210 1215

Asp Phe Leu Glu Ala Lys Gly Tyr Lys Glu Val Lys Lys Asp Leu
1220 1225 1230

Ile Ile Lys Leu Pro Lys Tyr Ser Leu Phe Glu Leu Glu Asn Gly
1235 1240 1245

Arg Lys Arg Met Leu Ala Ser Ala Gly Glu Leu Gln Lys Gly Asn
1250 1255 1260

Glu Leu Ala Leu Pro Ser Lys Tyr Val Asn Phe Leu Tyr Leu Ala
1265 1270 1275

Ser His Tyr Glu Lys Leu Lys Gly Ser Pro Glu Asp Asn Glu Gln
1280 1285 1290

Lys Gln Leu Phe Val Glu Gln His Lys His Tyr Leu Asp Glu Ile
1295 1300 1305

Ile Glu Gln Ile Ser Glu Phe Ser Lys Arg Val Ile Leu Ala Asp
1310 1315 1320

Ala Asn Leu Asp Lys Val Leu Ser Ala Tyr Asn Lys His Arg Asp
1325 1330 1335

Lys Pro Ile Arg Glu Gln Ala Glu Asn Ile Ile His Leu Phe Thr

1340 1345 1350

Leu Thr Asn Leu Gly Ala Pro Ala Ala Phe Lys Tyr Phe Asp Thr
1355 1360 1365

Thr Ile Asp Arg Lys Arg Tyr Thr Ser Thr Lys Glu Val Leu Asp
1370 1375 1380

Ala Thr Leu Ile His Gln Ser Ile Thr Gly Leu Tyr Glu Thr Arg
1385 1390 1395

Ile Asp Leu Ser Gln Leu Gly Gly Asp Lys Arg Pro Ala Ala Thr
1400 1405 1410

Lys Lys Ala Gly Gln Ala Lys Lys Lys Lys Glu Phe Gly Gly Ser
1415 1420 1425

Gly Gly Gly Ser Gly Gly Gly Ser Gly Gly Gly Ser Gly Gly Gly
1430 1435 1440

Ser Gly Gly Gly Ser Gly Gly Gly Ser Gly Gly Gly Ser Gly Gly
1445 1450 1455

Gly Ser Gly Gly Gly Ser Gly Met Asn Ile Asn Asp Leu Ile Arg
1460 1465 1470

Glu Ile Lys Asn Lys Asp Tyr Thr Val Lys Leu Ser Gly Thr Asp
1475 1480 1485

Ser Asn Ser Ile Thr Gln Leu Ile Ile Arg Val Asn Asn Ala Ala
1490 1495 1500

Asn Glu Tyr Val Ile Ser Glu Ser Glu Asn Glu Ser Ile Val Glu
1505 1510 1515

Lys Phe Ile Ser Ala Phe Lys Asn Gly Trp Asn Gln Glu Tyr Glu
1520 1525 1530

Asp Glu Glu Glu Phe Tyr Asn Asp Met Gln Thr Ile Thr Leu Lys
1535 1540 1545

Ser Glu Leu Asn
1550

<210> 28
<211> 1552
<212> PRT
<213> Artificial Sequence

<220>
<223> CasCAID#14 (NLS-Cas9-NLS fused to AcrIIA4 bearing D14A/G38A mutation)

<400> 28

Met Asp Tyr Lys Asp His Asp Gly Asp Tyr Lys Asp His Asp Ile Asp
1 5 10 15

Tyr Lys Asp Asp Asp Asp Lys Met Ala Pro Lys Lys Lys Arg Lys Val
20 25 30

Gly Ile His Gly Val Pro Ala Ala Asp Lys Lys Tyr Ser Ile Gly Leu
35 40 45

Asp Ile Gly Thr Asn Ser Val Gly Trp Ala Val Ile Thr Asp Glu Tyr
50 55 60

Lys Val Pro Ser Lys Lys Phe Lys Val Leu Gly Asn Thr Asp Arg His
65 70 75 80

Ser Ile Lys Lys Asn Leu Ile Gly Ala Leu Leu Phe Asp Ser Gly Glu
85 90 95

Thr Ala Glu Ala Thr Arg Leu Lys Arg Thr Ala Arg Arg Arg Tyr Thr
100 105 110

Arg Arg Lys Asn Arg Ile Cys Tyr Leu Gln Glu Ile Phe Ser Asn Glu
115 120 125

Met Ala Lys Val Asp Asp Ser Phe Phe His Arg Leu Glu Glu Ser Phe
130 135 140

Leu Val Glu Glu Asp Lys Lys His Glu Arg His Pro Ile Phe Gly Asn
145 150 155 160

Ile Val Asp Glu Val Ala Tyr His Glu Lys Tyr Pro Thr Ile Tyr His
165 170 175

Leu Arg Lys Lys Leu Val Asp Ser Thr Asp Lys Ala Asp Leu Arg Leu
180 185 190

Ile Tyr Leu Ala Leu Ala His Met Ile Lys Phe Arg Gly His Phe Leu
195 200 205

Ile Glu Gly Asp Leu Asn Pro Asp Asn Ser Asp Val Asp Lys Leu Phe
210 215 220

```
Ile Gln Leu Val Gln Thr Tyr Asn Gln Leu Phe Glu Glu Asn Pro Ile
225                 230                 235                 240

Asn Ala Ser Gly Val Asp Ala Lys Ala Ile Leu Ser Ala Arg Leu Ser
                245                 250                 255

Lys Ser Arg Arg Leu Glu Asn Leu Ile Ala Gln Leu Pro Gly Glu Lys
            260                 265                 270

Lys Asn Gly Leu Phe Gly Asn Leu Ile Ala Leu Ser Leu Gly Leu Thr
        275                 280                 285

Pro Asn Phe Lys Ser Asn Phe Asp Leu Ala Glu Asp Ala Lys Leu Gln
    290                 295                 300

Leu Ser Lys Asp Thr Tyr Asp Asp Asp Leu Asp Asn Leu Leu Ala Gln
305                 310                 315                 320

Ile Gly Asp Gln Tyr Ala Asp Leu Phe Leu Ala Ala Lys Asn Leu Ser
                325                 330                 335

Asp Ala Ile Leu Leu Ser Asp Ile Leu Arg Val Asn Thr Glu Ile Thr
            340                 345                 350

Lys Ala Pro Leu Ser Ala Ser Met Ile Lys Arg Tyr Asp Glu His His
        355                 360                 365

Gln Asp Leu Thr Leu Leu Lys Ala Leu Val Arg Gln Gln Leu Pro Glu
    370                 375                 380

Lys Tyr Lys Glu Ile Phe Phe Asp Gln Ser Lys Asn Gly Tyr Ala Gly
385                 390                 395                 400

Tyr Ile Asp Gly Gly Ala Ser Gln Glu Glu Phe Tyr Lys Phe Ile Lys
                405                 410                 415

Pro Ile Leu Glu Lys Met Asp Gly Thr Glu Glu Leu Leu Val Lys Leu
            420                 425                 430

Asn Arg Glu Asp Leu Leu Arg Lys Gln Arg Thr Phe Asp Asn Gly Ser
        435                 440                 445

Ile Pro His Gln Ile His Leu Gly Glu Leu His Ala Ile Leu Arg Arg
    450                 455                 460

Gln Glu Asp Phe Tyr Pro Phe Leu Lys Asp Asn Arg Glu Lys Ile Glu
465                 470                 475                 480
```

```
Lys Ile Leu Thr Phe Arg Ile Pro Tyr Tyr Val Gly Pro Leu Ala Arg
                485                 490                 495

Gly Asn Ser Arg Phe Ala Trp Met Thr Arg Lys Ser Glu Glu Thr Ile
            500                 505                 510

Thr Pro Trp Asn Phe Glu Glu Val Val Asp Lys Gly Ala Ser Ala Gln
        515                 520                 525

Ser Phe Ile Glu Arg Met Thr Asn Phe Asp Lys Asn Leu Pro Asn Glu
    530                 535                 540

Lys Val Leu Pro Lys His Ser Leu Leu Tyr Glu Tyr Phe Thr Val Tyr
545                 550                 555                 560

Asn Glu Leu Thr Lys Val Lys Tyr Val Thr Glu Gly Met Arg Lys Pro
                565                 570                 575

Ala Phe Leu Ser Gly Glu Gln Lys Lys Ala Ile Val Asp Leu Leu Phe
            580                 585                 590

Lys Thr Asn Arg Lys Val Thr Val Lys Gln Leu Lys Glu Asp Tyr Phe
        595                 600                 605

Lys Lys Ile Glu Cys Phe Asp Ser Val Glu Ile Ser Gly Val Glu Asp
    610                 615                 620

Arg Phe Asn Ala Ser Leu Gly Thr Tyr His Asp Leu Leu Lys Ile Ile
625                 630                 635                 640

Lys Asp Lys Asp Phe Leu Asp Asn Glu Glu Asn Glu Asp Ile Leu Glu
                645                 650                 655

Asp Ile Val Leu Thr Leu Thr Leu Phe Glu Asp Arg Glu Met Ile Glu
            660                 665                 670

Glu Arg Leu Lys Thr Tyr Ala His Leu Phe Asp Asp Lys Val Met Lys
        675                 680                 685

Gln Leu Lys Arg Arg Arg Tyr Thr Gly Trp Gly Arg Leu Ser Arg Lys
    690                 695                 700

Leu Ile Asn Gly Ile Arg Asp Lys Gln Ser Gly Lys Thr Ile Leu Asp
705                 710                 715                 720

Phe Leu Lys Ser Asp Gly Phe Ala Asn Arg Asn Phe Met Gln Leu Ile
                725                 730                 735
```

His Asp Asp Ser Leu Thr Phe Lys Glu Asp Ile Gln Lys Ala Gln Val
740 745 750

Ser Gly Gln Gly Asp Ser Leu His Glu His Ile Ala Asn Leu Ala Gly
755 760 765

Ser Pro Ala Ile Lys Lys Gly Ile Leu Gln Thr Val Lys Val Val Asp
770 775 780

Glu Leu Val Lys Val Met Gly Arg His Lys Pro Glu Asn Ile Val Ile
785 790 795 800

Glu Met Ala Arg Glu Asn Gln Thr Thr Gln Lys Gly Gln Lys Asn Ser
805 810 815

Arg Glu Arg Met Lys Arg Ile Glu Glu Gly Ile Lys Glu Leu Gly Ser
820 825 830

Gln Ile Leu Lys Glu His Pro Val Glu Asn Thr Gln Leu Gln Asn Glu
835 840 845

Lys Leu Tyr Leu Tyr Tyr Leu Gln Asn Gly Arg Asp Met Tyr Val Asp
850 855 860

Gln Glu Leu Asp Ile Asn Arg Leu Ser Asp Tyr Asp Val Asp His Ile
865 870 875 880

Val Pro Gln Ser Phe Leu Lys Asp Asp Ser Ile Asp Asn Lys Val Leu
885 890 895

Thr Arg Ser Asp Lys Asn Arg Gly Lys Ser Asp Asn Val Pro Ser Glu
900 905 910

Glu Val Val Lys Lys Met Lys Asn Tyr Trp Arg Gln Leu Leu Asn Ala
915 920 925

Lys Leu Ile Thr Gln Arg Lys Phe Asp Asn Leu Thr Lys Ala Glu Arg
930 935 940

Gly Gly Leu Ser Glu Leu Asp Lys Ala Gly Phe Ile Lys Arg Gln Leu
945 950 955 960

Val Glu Thr Arg Gln Ile Thr Lys His Val Ala Gln Ile Leu Asp Ser
965 970 975

Arg Met Asn Thr Lys Tyr Asp Glu Asn Asp Lys Leu Ile Arg Glu Val

980 985 990

Lys Val Ile Thr Leu Lys Ser Lys Leu Val Ser Asp Phe Arg Lys Asp
995 1000 1005

Phe Gln Phe Tyr Lys Val Arg Glu Ile Asn Asn Tyr His His Ala
1010 1015 1020

His Asp Ala Tyr Leu Asn Ala Val Val Gly Thr Ala Leu Ile Lys
1025 1030 1035

Lys Tyr Pro Lys Leu Glu Ser Glu Phe Val Tyr Gly Asp Tyr Lys
1040 1045 1050

Val Tyr Asp Val Arg Lys Met Ile Ala Lys Ser Glu Gln Glu Ile
1055 1060 1065

Gly Lys Ala Thr Ala Lys Tyr Phe Phe Tyr Ser Asn Ile Met Asn
1070 1075 1080

Phe Phe Lys Thr Glu Ile Thr Leu Ala Asn Gly Glu Ile Arg Lys
1085 1090 1095

Arg Pro Leu Ile Glu Thr Asn Gly Glu Thr Gly Glu Ile Val Trp
1100 1105 1110

Asp Lys Gly Arg Asp Phe Ala Thr Val Arg Lys Val Leu Ser Met
1115 1120 1125

Pro Gln Val Asn Ile Val Lys Lys Thr Glu Val Gln Thr Gly Gly
1130 1135 1140

Phe Ser Lys Glu Ser Ile Leu Pro Lys Arg Asn Ser Asp Lys Leu
1145 1150 1155

Ile Ala Arg Lys Lys Asp Trp Asp Pro Lys Lys Tyr Gly Gly Phe
1160 1165 1170

Asp Ser Pro Thr Val Ala Tyr Ser Val Leu Val Val Ala Lys Val
1175 1180 1185

Glu Lys Gly Lys Ser Lys Lys Leu Lys Ser Val Lys Glu Leu Leu
1190 1195 1200

Gly Ile Thr Ile Met Glu Arg Ser Ser Phe Glu Lys Asn Pro Ile
1205 1210 1215

Asp Phe Leu Glu Ala Lys Gly Tyr Lys Glu Val Lys Lys Asp Leu
1220 1225 1230

Ile Ile Lys Leu Pro Lys Tyr Ser Leu Phe Glu Leu Glu Asn Gly
1235 1240 1245

Arg Lys Arg Met Leu Ala Ser Ala Gly Glu Leu Gln Lys Gly Asn
1250 1255 1260

Glu Leu Ala Leu Pro Ser Lys Tyr Val Asn Phe Leu Tyr Leu Ala
1265 1270 1275

Ser His Tyr Glu Lys Leu Lys Gly Ser Pro Glu Asp Asn Glu Gln
1280 1285 1290

Lys Gln Leu Phe Val Glu Gln His Lys His Tyr Leu Asp Glu Ile
1295 1300 1305

Ile Glu Gln Ile Ser Glu Phe Ser Lys Arg Val Ile Leu Ala Asp
1310 1315 1320

Ala Asn Leu Asp Lys Val Leu Ser Ala Tyr Asn Lys His Arg Asp
1325 1330 1335

Lys Pro Ile Arg Glu Gln Ala Glu Asn Ile Ile His Leu Phe Thr
1340 1345 1350

Leu Thr Asn Leu Gly Ala Pro Ala Ala Phe Lys Tyr Phe Asp Thr
1355 1360 1365

Thr Ile Asp Arg Lys Arg Tyr Thr Ser Thr Lys Glu Val Leu Asp
1370 1375 1380

Ala Thr Leu Ile His Gln Ser Ile Thr Gly Leu Tyr Glu Thr Arg
1385 1390 1395

Ile Asp Leu Ser Gln Leu Gly Gly Asp Lys Arg Pro Ala Ala Thr
1400 1405 1410

Lys Lys Ala Gly Gln Ala Lys Lys Lys Lys Glu Phe Gly Gly Ser
1415 1420 1425

Gly Gly Gly Ser Gly Gly Gly Ser Gly Gly Gly Ser Gly Gly Gly
1430 1435 1440

Ser Gly Gly Gly Ser Gly Gly Gly Ser Gly Gly Gly Ser Gly Gly
1445 1450 1455

```
Gly Ser  Gly Gly Gly Ser Gly  Met Asn Ile Asn Asp  Leu Ile Arg
    1460                 1465                 1470

Glu Ile  Lys Asn Lys Ala Tyr  Thr Val Lys Leu Ser  Gly Thr Asp
    1475                 1480                 1485

Ser Asn  Ser Ile Thr Gln Leu  Ile Ile Arg Val Asn  Asn Asp Ala
    1490                 1495                 1500

Asn Glu  Tyr Val Ile Ser Glu  Ser Glu Asn Glu Ser  Ile Val Glu
    1505                 1510                 1515

Lys Phe  Ile Ser Ala Phe Lys  Asn Gly Trp Asn Gln  Glu Tyr Glu
    1520                 1525                 1530

Asp Glu  Glu Glu Phe Tyr Asn  Asp Met Gln Thr Ile  Thr Leu Lys
    1535                 1540                 1545

Ser Glu  Leu Asn
    1550
```

<210> 29
<211> 23
<212> DNA
<213> Artificial Sequence

<220>
<223> AAVS1 ON

<400> 29
gggagggaga gcttggcagg ggg 23

<210> 30
<211> 23
<212> DNA
<213> Artificial Sequence

<220>
<223> AAVS1 OFF1

<400> 30
gggaagggga gcttggcagg tgg 23

<210> 31
<211> 23
<212> DNA
<213> Artificial Sequence

<220>
<223> AAVS1 OFF2

<400> 31
gggaaggtga gcttggcagg tgg 23

<210> 32
<211> 23
<212> DNA
<213> Artificial Sequence

<220>
<223> HBB ON

<400> 32
ccacgttcac cttgccccac agg 23

<210> 33
<211> 23
<212> DNA
<213> Artificial Sequence

<220>
<223> HBB OFF

<400> 33
ccacattcac cttgccccac agg 23

<210> 34
<211> 23
<212> DNA
<213> Artificial Sequence

<220>
<223> EMX ON

<400> 34
gagtccgagc agaagaagaa ggg 23

<210> 35
<211> 23
<212> DNA
<213> Artificial Sequence

<220>
<223> EMX OFF

<400> 35
gagttagagc agaagaagaa agg 23

<210> 36
<211> 23
<212> DNA
<213> Artificial Sequence

<220>
<223> HEK ON

<400> 36
ggcactgcgg ctggaggtgg ggg 23

<210> 37

<211> 23
<212> DNA
<213> Artificial Sequence

<220>
<223> HEK OFF

<400> 37
tgcactgcgg ccggaggagg tgg 23

<210> 38
<211> 23
<212> DNA
<213> Artificial Sequence

<220>
<223> RUNX ON

<400> 38
gcattttcag gaggaagcga tgg 23

<210> 39
<211> 23
<212> DNA
<213> Artificial Sequence

<220>
<223> RUNX OFF

<400> 39
gcattttcag aaggaagcaa ggg 23

<210> 40
<211> 24
<212> DNA
<213> Artificial Sequence

<220>
<223> AAVS1_ON_fw

<400> 40
tggctactgg ccttatctca cagg 24

<210> 41
<211> 27
<212> DNA
<213> Artificial Sequence

<220>
<223> AAVS1_ON_re

<400> 41
ctctctagtc tgtgctagct cttccag 27

<210> 42
<211> 25
<212> DNA
<213> Artificial Sequence

<220>
<223> AAVS1_OFF1_fw

<400> 42
actcttctac cttgcacgcc tttgc 25

<210> 43
<211> 24
<212> DNA
<213> Artificial Sequence

<220>
<223> AAVS1_OFF1_re

<400> 43
cctgcctccc atgcaaacag tgtc 24

<210> 44
<211> 25
<212> DNA
<213> Artificial Sequence

<220>
<223> AAVS1_OFF2_fw

<400> 44
gagctggttt gcttatgtgt gcagg 25

<210> 45
<211> 24
<212> DNA
<213> Artificial Sequence

<220>
<223> AAVS1_OFF2_re

<400> 45
gcacttctcg ctggccactt acag 24

<210> 46
<211> 54
<212> DNA
<213> Artificial Sequence

<220>
<223> AAVS1_ON_seq_fw

<400> 46
tcgtcggcag cgtcagatgt gtataagaga caggggatca gtgaaacgca ccag 54

<210> 47
<211> 54
<212> DNA
<213> Artificial Sequence

<220>
<223> AAVS1_ON_seq_re

<400> 47
gtctcgtggg ctcggagatg tgtataagag acaggacaca cccccatttc ctgg 54

<210> 48
<211> 55
<212> DNA
<213> Artificial Sequence

<220>
<223> AAVS1_OFF1_seq_fw

<400> 48
tcgtcggcag cgtcagatgt gtataagaga caggactaag gcagagagac cgagg 55

<210> 49
<211> 58
<212> DNA
<213> Artificial Sequence

<220>
<223> AAVS1_OFF1_seq_re

<400> 49
gtctcgtggg ctcggagatg tgtataagag acagatgtgt gagccactta catagcac 58

<210> 50
<211> 27
<212> DNA
<213> Artificial Sequence

<220>
<223> HBB_ON_fw

<400> 50
caagcgtccc atagactcac cctgaag 27

<210> 51
<211> 26
<212> DNA
<213> Artificial Sequence

<220>
<223> HBB_ON_re

<400> 51
gtgccagaag agccaaggac aggtac 26

<210> 52
<211> 26
<212> DNA
<213> Artificial Sequence

<220>
<223> HBB_OFF_fw

<400> 52
atgatctctg ccccatctat gcttgg 26

<210> 53
<211> 27
<212> DNA
<213> Artificial Sequence

<220>
<223> HBB_OFF_re

<400> 53
ttgacccact gcatcagaat catttgg 27

<210> 54
<211> 53
<212> DNA
<213> Artificial Sequence

<220>
<223> EMX_ON_fw

<400> 54
tcgtcggcag cgtcagatgt gtataagaga cagaggtgaa ggtgtggttc cag 53

<210> 55
<211> 53
<212> DNA
<213> Artificial Sequence

<220>
<223> EMX_ON_re

<400> 55
gtctcgtggg ctcggagatg tgtataagag acagagtggc cagagtccag ctt 53

<210> 56
<211> 59
<212> DNA
<213> Artificial Sequence

<220>
<223> EMX_OFF_fw

<400> 56
tcgtcggcag cgtcagatgt gtataagaga caggacacct tttaagatct gacagagaa 59

<210> 57
<211> 58
<212> DNA
<213> Artificial Sequence

<220>
<223> EMX_OFF_re

<400> 57
gtctcgtggg ctcggagatg tgtataagag acagtgcaca tgtatgtaca ggagtcat 58

<210> 58

<211> 53
<212> DNA
<213> Artificial Sequence

<220>
<223> HEK_ON_fw

<400> 58
tcgtcggcag cgtcagatgt gtataagaga caggccctcc cctcccttca aga 53

<210> 59
<211> 54
<212> DNA
<213> Artificial Sequence

<220>
<223> HEK_ON_re

<400> 59
gtctcgtggg ctcggagatg tgtataagag acagccagac tccttctggg gcct 54

<210> 60
<211> 53
<212> DNA
<213> Artificial Sequence

<220>
<223> HEK_OFF_fw

<400> 60
tcgtcggcag cgtcagatgt gtataagaga cagcctgggg catggcttct gag 53

<210> 61
<211> 54
<212> DNA
<213> Artificial Sequence

<220>
<223> HEK_OFF_re

<400> 61
gtctcgtggg ctcggagatg tgtataagag acagctcaac ccaggtgttg gccc 54

<210> 62
<211> 53
<212> DNA
<213> Artificial Sequence

<220>
<223> RUNX_ON_fw

<400> 62
tcgtcggcag cgtcagatgt gtataagaga cagtacaggc aaagctgagc aaa 53

<210> 63
<211> 54
<212> DNA
<213> Artificial Sequence

<220>
<223> RUNX_ON_re

<400> 63
gtctcgtggg ctcggagatg tgtataagag acagccagag gtatccagca gagg 54

<210> 64
<211> 53
<212> DNA
<213> Artificial Sequence

<220>
<223> RUNX_OFF_fw

<400> 64
tcgtcggcag cgtcagatgt gtataagaga caggcatgat actttggggg aga 53

<210> 65
<211> 56
<212> DNA
<213> Artificial Sequence

<220>
<223> RUNX_OFF_re

<400> 65
gtctcgtggg ctcggagatg tgtataagag acagtctgat cagcaatgtt gagatg 56

## Description

The present invention relates to a method for modifying a target site in a host cell comprising contacting said host cell with a Cas nuclease, wherein said host cell is further contacted with a low-affinity Cas inhibitor and/or a sub-inhibitory concentration of a Cas inhibitor; and to a method for improving specificity of a Cas nuclease, comprising a) providing a Cas nuclease; and b) contacting said Cas nuclease with a low-affinity Cas inhibitor or a sub-inhibitory concentration of a Cas inhibitor; and c) thereby improving specificity of said Cas enzyme. Further, the present invention relates to compositions, polypeptides, uses and methods related thereto.

The emergence of CRISPR (clustered regularly interspaced short palindromic repeats)-Cas technologies enabled detailed genomic studies and brought a targeted therapy of genetic diseases into closer reach. The fidelity of the Cas nuclease, i.e. selectivity for the single guide (sg)RNA-matching genomic target locus as compared to Off-targets sites exhibiting partial complementary to the RNA guide, is a key parameter to be considered for CRISPR applications. Previous studies employed directed evolution or structure-guided protein engineering to identify point mutations in the Cas enzyme that reduce Off-target editing (e.g. Kulcsar et al., Genome Biol 18:190 (2017), Kleinstiver et al., Nature 529:490-495 (2016)). Complementary efforts devised rules for the design of target-specific sgRNAs (e.g. Akcakayaet al., Nature 561: 416-419 (2018)). While overall powerful, these strategies typically demand users to employ specific CRISPR-Cas components.

Anti-CRISPR (Acr) proteins were originally identified as naturally occurring CRISPR-Cas inhibitors. Amongst these is AcrIIA4, a potent inhibitor of the Streptococcus pyogenes (Spy)Cas9, which binds Cas9-sgRNA complexes with sub-nanomolar affinity and impairs DNA targeting as well as nuclease function (e.g. Dong et al., Nature 546: 436-439 (2017)). Recent data show that administering AcrIIA4 shortly after SpyCas9-sgRNA delivery can reduce Off-target editing (Shin et al, Sci Adv 3, e1701620 (2017)). However, this strategy requires two separate, precisely timed delivery steps (one for Cas9-sgRNA, one for the Acr) and also markedly reduces On-target editing efficiency.

There is, thus, a need in the art to provide reliable means to improve Cas nuclease target specificity. In particular, there is a need to provide means and methods avoiding at least in part the drawbacks of the prior art as discussed above.

This problem is solved by the methods, compositions, and uses with the features of the independent claims. Preferred embodiments, which might be realized in an isolated fashion or in any arbitrary combination are listed in the dependent claims.

Accordingly, the present invention relates to a method for modifying a target site in a host cell comprising contacting said host cell with a Cas nuclease, wherein said method further comprises contacting said host cell with a low-affinity Cas inhibitor and/or a sub-inhibitory concentration of a Cas inhibitor.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

As used herein, the term "standard conditions", if not otherwise noted, relates to IUPAC standard ambient temperature and pressure (SATP) conditions, i.e. preferably, a temperature of 25°C and an absolute pressure of 100 kPa; also preferably, standard conditions include a pH of 7. Moreover, if not otherwise indicated, the term "about" relates to the indicated value with the commonly accepted technical precision in the relevant field, preferably relates to the indicated value ± 20%, more preferably ± 10%, most preferably ± 5%. Further, the term "essentially" indicates that deviations having influence on the indicated result or use are absent, i.e. potential deviations do not cause the indicated result to deviate by more than ± 20%, more preferably ± 10%, most preferably ± 5%. Thus, "consisting essentially of' means including the components specified but excluding other components except for materials present as impurities, unavoidable materials present as a result of processes used to provide the components, and components added for a purpose other than achieving the technical effect of the invention. For example, a composition defined using the phrase "consisting essentially of' encompasses any known acceptable additive, excipient, diluent, carrier, and the like. Preferably, a composition consisting essentially of a set of components will comprise less than 5% by weight, more preferably less than 3% by weight, even more preferably less than 1%, most preferably less than 0.1% by weight of non-specified component(s). In the context of nucleic acid sequences, the term "essentially identical" indicates a %identity value of at least 80%, preferably at least 90%, more preferably at least 98%, most preferably at least 99%. As will be understood, the term essentially identical includes 100% identity. The aforesaid applies to the term "essentially complementary" mutatis mutandis.

The method of the present invention may be an in vitro method or an in vivo method. Preferably, the method is an in vitro method; thus, the method preferably is a method not performed on a human or animal body, more preferably is performed on isolated cells, most preferably performed on isolated host cells which are not re-administered to a human or animal body. Also preferably, the method is an in vivo method; thus, preferably, the method is a method performed on a human or animal body. As will be appreciated, in case the method is an in vivo method, it may be a method of treating and/or preventing disease as specified elsewhere herein. The method may comprise steps in addition to those explicitly mentioned above. For example, further steps may relate, e.g., to providing a host cell for step a), or incubating the host cell after step b). Moreover, one or more of said steps may be performed by automated equipment. Preferably, the method is a method of specifically modifying a gene and/or modifying expression of a gene in a host cell, preferably of specifically modifying a gene in a host cell. Also preferably, the method further comprises contacting said host cell with at least one guide RNA (gRNA), preferably at least one gRNA specifically binding to a target site of interest. Principles of designing gRNAs for target sites of interest are known to the skilled person, e.g. from the literature cited herein above.

The term "target site", as used herein, relates to a nucleic acid sequence in a polynucleotide comprised in a host cell, preferably in a genomic DNA, an organellar DNA, or a plasmid comprised in said host cell. Preferably, the target site is a unique site in said host cell, i.e. is a nucleic acid sequence occurring only once in the host cell. Preferably, the target site has a length of at least 12 nucleotides, more preferably at least 15 nucleotide, still more preferably at least 18 nucleotides, most preferably at least 20 nucleotides. Preferably, the target site comprises, more preferably is, a sequence recognizable by a gRNA as specified elsewhere herein; thus, preferably, the target site comprises a sequence of at least 15 nucleotides, preferably at least 18 nucleotides, more preferably at least 20 nucleotides complementary to a gRNA. As will be understood, a target site may be comprised within a gene; as will also be understood, a gene usually comprises a multitude, i.e. two or more, preferably non-identical, target sites. Thus, by modifying one or more target sites, a target gene may be modified.

The term "off-target site" is understood by the skilled person to relate to any site in a polynucleotide having a nucleic acid sequence which is non-identical to the target site as specified herein above. Thus, the nucleic acid sequence of the off-target site differs from the nucleic acid sequence of the target site by at least one, preferably at least two, more preferably at least three nucleotides. Preferably, the off-target site has been experimentally shown to be edited in a host cell when a Cas nuclease and a gRNA for a given target sequence are administered; i.e., preferably, the off-target sequence is a known off-target sequence or a predicted off-target sequence; means and methods for predicting off-target sites are known in the art and include e.g. the services offered by CRISPR OFFinder (www.rgenome.net/cas-offinder/) As will be understood by the skilled person, the meaning of the term off-target site depends on the selected target site, i.e., preferably, an off-target site is an off-target site of a specific target site.

The terms "modifying a target site" and "modifying a target gene" relate to a modification comprising at least introduction of a double strand break into a polynucleotide at the target site and/or a modification of binding state of a target site or a target gene. Preferably, in the methods specified herein, the frequency of off-target modification is decreased by at least 2-fold, preferably at least 3-fold, more preferably at least 10-fold, most preferably at least 15-fold, compared to the frequency of off-target modification in the absence of contacting said Cas nuclease with a low-affinity Cas inhibitor or a sub-inhibitory concentration of a Cas inhibitor.

Preferably, modifying a target site and modifying a target gene comprise modification of binding state of a target site or a target gene. The term "binding state", as used herein in connection with a target site or a target gene, relates to the kind and number of molecules, in particular polypeptides, bound to said target site or target gene, preferably in a host cell. Preferably, modification of binding state of a target site or a target gene comprises binding of a Cas nuclease, preferably specific binding of a Cas nuclease, preferably mediated by a gRNA, to said target site or target gene; preferably, the Cas nuclease is a non-catalytic mutein in such case, i.e., preferably, is a variant of a Cas nuclease binding to a target site but not cleaving the polynucleotide comprising said target site; more preferably, in such case, the Cas nuclease is a non-catalytic mutein fused to or non-covalently associated with a transcription activation domain, a transcription repression domain, a DNA methyltransferase, a histone acetyltransferase, a histone deacetylase, a histone methyltransferase, a histone demethylase and/or a DNA base-modifying enzyme, in particular a cytidine deaminase or an adenine base modifying enzyme. Transcription activation domains and transcription repression domains are well-known in the art. Thus, preferably, modification of binding state of a target site or a target gene is sequestering a regulatory polypeptide, preferably an activator domain or a repressor domain, to a target site or a target gene.

More preferably, modification of a target site or of a target gene comprises at least introduction of a double strand break into a polynucleotide at the target site. Preferably, said modifying further comprises introduction of a mutation, in particular an insertion or deletion. Also preferably, the term modifying a target gene further comprises exchange of a partial or of the complete nucleic acid sequence of said target gene for a non-identical nucleic acid sequence, preferably for the sequence of a different allele, more preferably an allele not causing disease. Preferably, the ratio of an editing frequency (i.e. frequency of modification by introduction of at least a double strand break into a polynucleotide at the target site) of the target site to an editing frequency of any off-target site is at least 2, preferably at least 5, more preferably at least 7, still more preferably at least 10, still more preferably at least 15, even more preferably at least 18, most preferably at least 20.

As used herein, the term "host cell" relates to any cell comprising a DNA polynucleotide, preferably as a genome. Preferably, the term relates to a host cell comprising all components required for modification of a target site by a Cas nuclease to occur being general for the pathway; i.e., preferably, in a host cell RNA interference is expected to occur in case a Cas nuclease and a gRNA comprising a sequence complementary to a target site are provided. Preferably, the cell is a bacterial cell, more preferably a cell of a common laboratory bacterial strain known in the art, most preferably an Escherichia strain, in particular an E. coli strain. Also preferably, the host cell is a eukaryotic cell, preferably a plant or yeast cell, e.g. a cell of a strain of baker's yeast, or is an animal cell. More preferably, the host cell is an insect cell or a mammalian cell. Even more preferably, the host cell is a mammalian cell, in particular a human, mouse, or rat cell, most preferably is a human cell. It is, however, also envisaged that the host cell is a plant cell, preferably of a dicot or monocot plant more preferably of a crop plant, in particular corn, rice, wheat, rye, or soybean.

The terms "CRISPR-associated endonuclease" and "Cas nuclease", as used herein, both equally relate to an endonuclease, preferably an endo-DNase or endo-RNase, more preferably an endo-DNase, recognizing a gRNA as specified herein, which is, in principle, known in the art. Preferably, the Cas nuclease is a type II CRISPR endonuclease. Preferably, the Cas nuclease is a CRISPR endonuclease from Prevotella and Francisella endonuclease, i.e. a Cpf1 endonuclease. More preferably, the CRISPR endonuclease is a Cas9 endonuclease. Preferably, the Cas9 nuclease is a Cas9 endonuclease from Staphylococcus aureus or is a Cas9 endonuclease from Streptococcus pyogenes, more preferably is a Cas9 endonuclease from Streptococcus pyogenes. Preferably, the Cas nuclease has an amino acid sequence as shown in SEQ ID NO:1, preferably encoded by a nucleic acid sequence as shown in SEQ ID NO:2.

The term "Cas inhibitor", as used herein, relates to a compound, preferably a polypeptide, causing Cas nuclease activity to decrease compared to the activity of said Cas nuclease in the absence of said Cas inhibitor. Thus, the skilled person is able to establish for a candidate compound that it is a Cas inhibitor by comparing the activity of a Cas nuclease in the presence and the absence of said candidate compound. Preferably, the Cas inhibitor is an Acr polypeptide. The terms "anti-CRISPR polypeptide" and "Acr polypeptide" are known to the skilled person and relate equally to a polypeptide having the aforesaid activity of inhibiting at least one Cas nuclease, preferably a Cas9 nuclease. Acr polypeptides and methods for their identification are known in the art e.g. from Pawluk et al. (2016), Cell 167(7): 1829, and from Rauch et al. (2017), Cell 168(1-2): 150. As will also be understood, preferably, the Acr polypeptide of the present invention is selected such that it inhibits the Cas nuclease used. Thus, e.g. in case the Cas9 endonuclease from Streptococcus pyogenes is used for target site modification, an Acr polypeptide of a Listeria monocytogenes prophage or a Streptococcus thermophilus virulent phage may be used. Thus, preferably, the Acr polypeptide is an Acr polypeptide of a Listeria monocytogenes prophage, more preferably is an AcrIIA2 or AcrIIA4 polypeptide, most preferably an AcrIIA4 polypeptide. Preferably, the Acr polypeptide comprises, preferably consists of, an amino acid sequence as shown in SEQ ID NO:3, more preferably encoded by a nucleic sequence comprising, preferably consisting of, the sequence as shown in SEQ ID NO:4.

In accordance with the above, the term "low-affinity Cas inhibitor" relates to a Cas inhibitor as specified above with a decreased affinity to the Cas nuclease. Preferably, the low-affinity Cas inhibitor, when present in equimolar amounts to a Cas nuclease in a Cas activity assay, inhibits target site modification by at most 50%, preferably at most 40%, more preferably at most 30%, even more preferably at most 20%, most preferably at most 10%. Cas activity assays are known in the art and include in particular those shown in the Examples provided herein below. Preferably, the low-affinity Cas inhibitor is a derivative of a Cas inhibitor as specified herein above, the term "derivative" in the context of a low-affinity Cas inhibitor relating to a polypeptide having at least 50%, preferably at least 75%, more preferably at least 90%, most preferably at least 95% sequence identity with the Cas inhibitor it is derived from. Thus, the derivative being a low-affinity Cas inhibitor preferably is a mutein or a fusion polypeptide of a Cas inhibitor. Preferably, the low-affinity Cas inhibitor has an affinity of from 0.01% to 90%, preferably of from 0.1 to 50%, more preferably of from 0.5% to 25%, still more preferably of from 1% to 10%, of the corresponding Cas inhibitor to the Cas nuclease.

Preferably, the Cas inhibitor is an Acr polypeptide, preferably is an AcrIIA4 polypeptide. Thus, preferably, the low-affinity Cas inhibitor is a derivative of an Acr polypeptide, preferably of an AcrIIA4 polypeptide, as specified above. Thus, preferably, the low-affinity Cas inhibitor is a derivative of an Acr polypeptide, preferably of an AcrIIA4 polypeptide, as specified herein above having an affinity for Cas nuclease, preferably a Cas9 nuclease, of from 0.01% to 90%, preferably of from 0.1 to 50%, more preferably of from 0.5% to 25%, still more preferably of from 1% to 10%, of the corresponding Acr polypeptide, preferably the corresponding AcrIIA4 polypeptide. Preferably, the low-affinity Cas inhibitor is a mutein of the AcrIIA4 polypeptide comprising at least one mutation selected from the list consisting of (i) M77A; (ii) D76A/M77A; (iii); (iv) D14A; (v) D14A/Y15A; (vi) D14A/G38A; (vii) G38A; (viii) D37A/G38A; (ix) D14A/G38A, (x) a deletion of amino acids N64/Q65/E66 and an insertion of a LOV-domain; (xi) a deletion of amino acids N64/Q65/E66; (xii) an insertion of a LOV-domain comprising T406A, T407A, G528A, and N538E mutations (positions relate to the full-length phototropin-1 protein from *Avena sativa*); and (xiii) any combination of (i) to (xii). As the skilled person understands, the indicated amino acid numbering corresponds to the numbering of amino acids in the AcrIIA4 polypeptide, preferably as shown in SEQ ID NO:3. More preferably, the low-affinity Cas inhibitor is a mutein of the AcrIIA4 polypeptide comprising at least one mutation selected from the list consisting of (i) N39A; (ii) D14A/G38A; (iii) a deletion of amino acids N64/Q65/E66 and an insertion of a LOV-domain comprising T406A, T407A, G528A, and N538E mutations; and (iv) any combination of (i) to (iii). Thus, preferably, the low-affinity Cas inhibitor comprises, preferably consists of, the amino acid sequence of SEQ ID NO:5, preferably encoded by a nucleic acid sequence as shown in SEQ ID NO:6; comprises, preferably consists of, the amino acid sequence of SEQ ID NO:7, preferably encoded by a nucleic acid sequence as shown in SEQ ID NO:8; or comprises, preferably consists of, the amino acid sequence of SEQ ID NO:9, preferably encoded by a nucleic acid sequence as shown in SEQ ID NO:10.

The term "LOV-domain", as used herein, relates to a light-oxygen-or-voltage (LOV) domain, which is in principle known to the skilled person; preferably, the LOV-domain is a LOV2 domain, preferably from Avena sativa or Arabidopsis thaliana phototropin-1, more preferably from Avena sativa. Preferably, the LOV-domain has an amino acid sequence as shown in SEQ ID NO:11 or a sequence at least 70%, preferably at least 80%, more preferably at least 90%, most preferably at least 95% identical to said sequence; more preferably, the LOV-domain has an amino acid sequence as shown in SEQ ID NO:11, preferably encoded by a nucleic acid sequence as shown in SEQ ID NO:12. Preferably, the LOV-domain inserted into a surface-exposed loop of the Acr, preferably at an insertion site corresponding to one of amino acids 62 to 69 of an AcrIIA4 polypeptide, i.e. preferably, corresponding to one of amino acids 62 to 69 of SEQ ID NO:3. As used herein, the expression "insertion site corresponding to amino acid X" relates to an insertion after amino acid X in the conventional N-terminus to C-terminus notation; thus, e.g. an insertion site corresponding to amino acid 63 relates to an insertion between amino acids 63 and 64. Still more preferably, the LOV-domain is inserted into the Acr replacing at least one amino acid corresponding to one of amino acids 62 to 69 of the AcrIIA4 polypeptide. Preferably, at least one, more preferably at least two, more preferably at least three, most preferably three amino acids are replaced. More preferably, at least one, two, or three amino acids corresponding to amino acids 64 to 67 of SEQ ID NO:3 are replaced, more preferably three amino acids corresponding to amino acids 64 to 67 of SEQ ID NO:3 are replaced. Most preferably, the LOV-domain is inserted into the Acr replacing the amino acids corresponding to amino acids 64 to 66 of SEQ ID NO:3.

The term "contacting" is understood by the skilled person to relate to bringing the compounds as indicated into close physical proximity so as to allow the components to interact. Preferably, contacting comprises causing the compounds to be present admixed in a, preferably aqueous, solution. More preferably, contacting comprises causing the compounds indicated to enter or to be expressed in a host cell. Also preferably, contacting further comprises causing the compounds to be transferred to the relevant compartment within the host cell, preferably the nucleus of a eukaryotic host cell, e.g. by fusing a nuclear localization sequence (NLS) to the compound. In accordance, contacting a Cas nuclease with a (low-affinity) Cas inhibitor may be accomplished by any method allowing the Cas nuclease and the Cas inhibitor to interact, preferably in a host cell, more preferably in a relevant compartment of the host cell, most preferably in the nucleus of a host cell.

Preferably, the Cas nuclease and the Cas inhibitor are co-expressed in a host cell, i.e., preferably, at least one polynucleotide is provided comprising a nucleic acid sequence encoding a Cas nuclease and a nucleic acid sequence encoding a Cas inhibitor, both in expressible form. More preferably, the nucleic acid sequence encoding a Cas nuclease and the nucleic acid sequence encoding a Cas inhibitor are provided in expressible form on two non-identical polynucleotides. Thus, preferably, co-expression is from two different expression constructs, which may be introduced into the cell subsequently or, preferably, simultaneously. Introduction of polynucleotides into a host cell, in particular of expression constructs, may be accomplished by any method deemed appropriate by the skilled person, e.g. by transfection into a host cell or by infection of a host cell with an appropriate viral vector.

The term "polynucleotide", as used herein, refers to a linear or circular nucleic acid molecule. The term encompasses single as well as partially or completely double-stranded polynucleotides. Preferably, the polynucleotide is RNA or DNA, including cDNA. Moreover, comprised are also chemically modified polynucleotides including naturally occurring modified polynucleotides such as glycosylated or methylated polynucleotides or artificially modified derivatives such as biotinylated polynucleotides. The polynucleotide of the present invention shall be provided, preferably, either as an isolated polynucleotide (i.e. isolated from its natural context) or in genetically modified form. The polynucleotide as specified herein, preferably, comprises at least one heterologous sequence, i.e. comprises sequences from at least two different species. Preferably, said sequences from two different species are the sequence encoding an Acr polypeptide as specified elsewhere herein and the Cas nuclease. Also preferably, the polynucleotide comprises at least one heterologous sequence relative to a mammalian, preferably human, cell, i.e. comprises at least one nucleic acid sequence not known to occur or not occurring in a mammalian, preferably human, cell. Preferably, said heterologous sequence relative to a mammalian cell is at least the sequence encoding an Acr polypeptide.

The polynucleotide of the present invention has the activity of encoding a Cas nuclease and/or a Cas inhibitor, in particular a low-affinity Cas inhibitor. More preferably, the polynucleotide encodes a Cas nuclease/low-affinity Cas inhibitor fusion polypeptide as specified elsewhere herein. Preferably, the polynucleotide comprises the nucleic acid sequence of at least one of SEQ ID NOs:2, 4, 6, 8, and 10, more preferably is a polynucleotide encoding a fusion polypeptide comprising the amino acid sequence of at least one of SEQ ID NOs:15 to 28. As used herein, the term polynucleotide, preferably, includes variants of the specifically indicated polynucleotides. More preferably, the term polynucleotide relates to the specific polynucleotides indicated. The skilled person knows how to select a polynucleotide encoding a polypeptide having a specific amino acid sequence and also knows how to optimize the codons used in the polynucleotide according to the codon usage of the organism used for expressing said polynucleotide. The term "polynucleotide variant", as used herein, relates to a variant of a polynucleotide related to herein comprising a nucleic acid sequence characterized in that the sequence can be derived from the aforementioned specific nucleic acid sequence by at least one nucleotide substitution, addition and/or deletion, wherein the polynucleotide variant shall have the activities as specified for the specific polynucleotide. Thus, it is to be understood that a polynucleotide variant as referred to in accordance with the present invention shall have a nucleic acid sequence which differs due to at least one nucleotide substitution, deletion and/or addition from the original polynucleotide. Preferably, said polynucleotide variant comprises an ortholog, a paralog or another homolog of the specific polynucleotide or of a functional subsequence thereof, e.g. of the sequence encoding a Cas nuclease, a Cas inhibitor polypeptide, and/or fusion polypeptide. Also preferably, said polynucleotide variant comprises a naturally occurring allele of the specific polynucleotide or of a functional subsequence thereof. In the context of polynucleotide variants, the term "functional subsequence", as used herein, relates to a part of a sequence of the polynucleotide mediating the activity as specified elsewhere herein. Polynucleotide variants also encompass polynucleotides comprising a nucleic acid sequence which is capable of hybridizing to the aforementioned specific polynucleotides or functional subsequences thereof, preferably, under stringent hybridization conditions. These stringent conditions are known to the skilled worker and can be found in Current Protocols in Molecular Biology, John Wiley & Sons, N. Y. (1989), 6.3.1-6.3.6. A preferred example for stringent hybridization conditions are hybridization conditions in 6x sodium chloride/sodium citrate (= SSC) at approximately 45°C, followed by one or more wash steps in 0.2x SSC, 0.1% SDS at 50 to 65°C. The skilled worker knows that these hybridization conditions differ depending on the type of nucleic acid and, for example when organic solvents are present, with regard to the temperature and concentration of the buffer. For example, under "standard hybridization conditions" the temperature differs depending on the type of nucleic acid between 42°C and 58°C in aqueous buffer with a concentration of 0.1x to 5x SSC (pH 7.2). If organic solvent is present in the abovementioned buffer, for example 50% formamide, the temperature under standard conditions is approximately 42°C. The hybridization conditions for DNA:DNA hybrids are preferably, for example, 0.1x SSC and 20°C to 45°C, preferably between 30°C and 45°C. The hybridization conditions for DNA:RNA hybrids are preferably, for example, 0.1x SSC and 30°C to 55°C, preferably between 45°C and 55°C. The abovementioned hybridization temperatures are determined, for example, for a nucleic acid with approximately 100 bp (= base pairs) in length and a G + C content of 50% in the absence of formamide. The skilled worker knows how to determine the hybridization conditions required by referring to standard textbooks. Alternatively, polynucleotide variants are obtainable by PCR-based techniques such as mixed oligonucleotide primer-based amplification of DNA, i.e. using degenerated primers against conserved domains of a polypeptide of the present invention. Conserved domains of a polypeptide may be identified by a sequence comparison of the nucleic acid sequence of the polynucleotide or the amino acid sequence of the polypeptide of the present invention with sequences of other organisms. As a template, DNA or cDNA from bacteria, fungi, plants or, preferably, from animals may be used. Further, variants include polynucleotides comprising nucleic acid sequences which are at least 70%, at least 75%, at least 80%, at least 85%, at least 90%, at least 95%, at least 98% or at least 99% identical to the specifically indicated nucleic acid sequences or functional subsequence thereof. Moreover, also encompassed are polynucleotides which comprise nucleic acid sequences encoding amino acid sequences which are at least 70%, at least 75%, at least 80%, at least 85%, at least 90%, at least 95%, at least 98% or at least 99% identical to the amino acid sequences specifically indicated. The percent identity values are, preferably, calculated over the entire amino acid or nucleic acid sequence region. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (J. Mol. Evolution., 25, 351-360, 1987, Higgins et al., CABIOS, 5 1989: 151-153) or the programs Gap and BestFit (Needleman and Wunsch (J. Mol. Biol. 48; 443-453 (1970)) and Smith and Waterman (Adv. Appl. Math. 2; 482-489 (1981))), which are part of the GCG software packet (Genetics Computer Group, 575 Science Drive, Madison, Wisconsin, USA 53711 (1991)), are to be used. The sequence identity values recited above in percent (%) are to be determined, preferably, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments.

A polynucleotide comprising a fragment of any of the specifically indicated nucleic acid sequences, said polynucleotide retaining the indicated activity or activities, is also encompassed as a variant polynucleotide. A fragment as meant herein, preferably, comprises at least 100, preferably at least 200, more preferably at least 250 consecutive nucleotides of any one of the specific nucleic acid sequences or encodes an amino acid sequence comprising at least 50, preferably at least 60, more preferably at least 75 consecutive amino acids of any one of the specific amino acid sequences.

The polynucleotides of the present invention either consist of, essentially consist of, or comprise the aforementioned nucleic acid sequences. Thus, they may contain further nucleic acid sequences as well. Specifically, the polynucleotides of the present invention may encode fusion proteins comprising further fusion partners, in particular those specified elsewhere herein. Fusion proteins may comprise as additional part polypeptides for monitoring expression (e.g., green, yellow, blue or red fluorescent proteins, alkaline phosphatase and the like) or so called "tags" which may serve as a detectable marker or as an auxiliary measure for purification purposes. Tags for the different purposes are well known in the art and are described elsewhere herein. Preferably, the polynucleotide encodes a polypeptide fused to a nuclear localization sequence (NLS). Preferably, the polynucleotide further comprises a nucleic acid sequence encoding at least a fragment of a Cas nuclease, preferably as specified elsewhere herein; also preferably, the polynucleotide does not comprise a nucleic acid sequence encoding at least a fragment of a Cas nuclease.

Preferably, the polynucleotide is an RNA. More preferably, the polynucleotide is a DNA comprising a nucleic acid sequence expressible as a continuous RNA comprising said sequence encoding a polypeptide or fusion polypeptide. Preferably, in case the polynucleotide is DNA, the polynucleotide is operatively linked to expression control sequences allowing expression in prokaryotic or eukaryotic, preferably in eukaryotic host cells or isolated fractions thereof, i.e. preferably, the polynucleotide is an expression construct. Expression of said polynucleotide comprises transcription of the polynucleotide, preferably into a translatable mRNA. Regulatory elements ensuring expression in eukaryotic cells, preferably mammalian cells, are well known in the art. They, preferably, comprise regulatory sequences ensuring initiation of transcription and, optionally, poly-A signals ensuring termination of transcription and stabilization of the transcript. Additional regulatory elements may include transcriptional as well as translational enhancers. Examples for regulatory elements permitting expression in eukaryotic host cells are the AOX1 or GAL1 promoter in yeast or the SMVP-, CMV-EFS-, SV40-, or RSV-promoter (Rous sarcoma virus), CMV-enhancer, SV40-enhancer or a globin intron in mammalian and other animal cells. Moreover, inducible or cell type-specific expression control sequences may be comprised in a polynucleotide of the present invention. Inducible expression control sequences may comprise tet or lac operator sequences or sequences inducible by heat shock or other environmental factors. Suitable expression control sequences are well known in the art. Besides elements which are responsible for the initiation of transcription, such regulatory elements may also comprise transcription termination signals, such as the SV40-poly-A site or the tk-poly-A site, downstream of the polynucleotide.

Preferably, contacting a Cas nuclease with a sub-inhibitory concentration of a Cas inhibitor comprises co-expression of a Cas nuclease and a Cas inhibitor in a host cell, wherein the ratio Cas nuclease:Cas inhibitor is adjusted such that activity of the Cas nuclease is reduced significantly, but the Cas nuclease is not completely inhibited. Thus, preferably, contacting said Cas nuclease with a sub-inhibitory concentration of a Cas inhibitor comprises contacting said Cas nuclease with a Cas inhibitor at a molar ratio Cas nuclease:Cas inhibitor of 10:1 to 1:1, preferably of from 8:1 to 1.5:1, more preferably of from 6:1 to 1.75:1, even more preferably from 5:1 to 2:1, still more preferably of from 4:1 to 2.2:1, most preferably of about 3:1. Preferably, the aforesaid ratio is adjusted by providing two expression constructs with different degrees of expression, e.g. by using promoters with different activity in the host cell. Optionally, one or both of said promoters may be regulatable in such case. More preferably, the Cas nuclease and the Cas inhibitor are expressed from similar expression constructs, preferably comprising promotors having essentially the same strength, more preferably comprising the same promoters. Preferably, contacting a Cas nuclease with a sub-inhibitory concentration of a Cas inhibitor comprises contacting said Cas nuclease with a Cas inhibitor at a molar ratio of expression constructs, preferably a molar vector ratio, Cas nuclease:Cas inhibitor of 10:1 to 1:1, preferably of from 8:1 to 1.5:1, more preferably of from 6:1 to 1.75:1, even more preferably from 5:1 to 2:1, still more preferably of from 4:1 to 2.2:1, most preferably of about 3:1. Contacting a Cas nuclease with a sub-inhibitory concentration of a Cas inhibitor may also comprise contacting said Cas nuclease with a low-affinity Cas inhibitor; as will be understood by the skilled person, the aforesaid molar ratios may require adjustment, preferably decrease, to compensate for the lower activity of the low-affinity Cas inhibitor compared to a Cas inhibitor, in such case.

Preferably, contacting a Cas nuclease with a low-affinity Cas inhibitor comprises providing a complex or fusion polypeptide between a Cas nuclease and a low-affinity Cas inhibitor. Preferably, in case a complex comprising a Cas nuclease and a low-affinity Cas inhibitor is provided, the dissociation constant ($K_d$) for the complex Cas nuclease/low-affinity Cas inhibitor is at most $10^{-6}$ M, preferably at most $10^{-7}$ M, more preferably at most $10^{-8}$ M, most preferably at most $10^{-9}$ M; appropriate $K_d$ values can e.g. be achieved by fusing the Cas nuclease and the low-affinity Cas inhibitor to the respective components of a high-affinity pair of molecules, such as in the strep-tag system or in an antibody/antigen complex. More preferably, contacting a Cas nuclease with a low-affinity Cas inhibitor comprises providing a fusion polypeptide of said Cas nuclease with said low-affinity Cas inhibitor, preferably via a linker peptide, more preferably via a GS and/or GG-comprising linker, preferably a GGSG (SEQ ID NO:13)-comprising linker, more preferably a

$(GGSG)_n$-linker, with n being an integer of from 1 to 100, preferably of from 2 to 50, more preferably of from 3 to 25, even more preferably being about 10, most preferably being 10. Most preferably, the linker has the amino acid sequence GGSGGGSGGGSGGGSGGGSGGGSGGGSGGGSGGGSGGGSG (SEQ ID NO:14). Thus, preferably, contacting a Cas nuclease with a low-affinity Cas inhibitor comprises providing the Cas nuclease and the low-affinity Cas inhibitor at a 1:1 molar ratio in a fusion polypeptide. Preferably, the Cas nuclease is fused N-terminal to the low-affinity Cas inhibitor, i.e., preferably, the sequence in the fusion polypeptide is Cas nuclease-low-affinity Cas inhibitor. Preferably, the Cas nuclease and/or the low-affinity Cas inhibitor in the fusion polypeptide are those as specified herein above. More preferably, the Cas nuclease and the low-affinity Cas inhibitor in the fusion polypeptide are those as specified herein above.Most preferably, in such case, the fusion polypeptide is the polypeptide comprising (i) a Cas nuclease and (ii) a low-affinity Cas inhibitor as specified herein below.

Advantageously, it was found in the work underlying the present invention that off-site activity of Cas nucleases can be kinetically separated from target activity (i.e. On-target activity) and that by reducing Cas activity, in particular by contacting the Cas nuclease with a sub-inhibitory activity of a Cas inhibitor or by contacting the Cas nuclease with a low-affinity Cas inhibitor, off-target activity can be reduced by more than one order of magnitude, while only moderately reducing target activity.

The definitions made above apply mutatis mutandis to the following. Additional definitions and explanations made further below also apply for all embodiments described in this specification mutatis mutandis.

The present invention also relates to a composition comprising or causing expression in a host cell of a Cas nuclease and (i) a low-affinity Cas inhibitor and/or (ii) a sub-inhibitory concentration of a Cas inhibitor.

The term "composition" is understood by the skilled person. Preferably, the term relates to a composition of matter comprising, preferably consisting of, at least the indicated components. Preferably, the composition comprises a Cas nuclease and a low-affinity Cas inhibitor, preferably as a complex or fusion polypeptide as specified herein above, more preferably as a fusion polypeptide. Also preferably, the composition comprises a Cas nuclease and a sub-inhibitory concentration of a Cas inhibitor; thus, in such case, the relative concentrations of the compounds are adjusted accordingly, preferably as specified herein above. The composition preferably comprises the indicated polypeptides. More preferably, the composition comprises polynucleotides, preferably expression constructs, causing expression of the indicated components in a host cell. Preferably, the composition comprises the components in a pre-adjusted, preferably ready-to-use, manner, in particular with the relative amounts of Cas nuclease and Cas inhibitor or polynucleotides causing their expression pre-adjusted. The composition may further comprise additional compounds, in particular solubilization means such as a solvent like water, salts, transfection agents, at least one gRNA, and the like. Preferably, additional components are selected such as to not interfere with the activity of the indicated components. Preferably, the composition is a pharmaceutical composition.

The term "pharmaceutical composition", as used herein, relates to a composition comprising the compounds as specified and optionally one or more pharmaceutically acceptable carrier, preferably produced and admixed to make the composition suitable for pharmaceutical use. Thus, the compounds are, preferably, produced and/or admixed under conditions of good manufacturing practice (GMP). The compounds of the present invention can be formulated as pharmaceutically acceptable salts. Acceptable salts are known in the art and comprise preferably acetate, sulfate, chloride salts and the like. The pharmaceutical composition is, preferably, administered topically or systemically. Suitable routes of administration conventionally used for drug administration are oral, intravenous, or parenteral administration as well as inhalation. However, depending on the nature and mode of action of a compound, the pharmaceutical compositions may be administered by other routes as well. For example, polynucleotide compounds may be administered in a gene therapy approach by using viral vectors or viruses or liposomes, as specified elsewhere herein. Moreover, the compounds can be administered in combination with other drugs either in a common pharmaceutical composition or as separated pharmaceutical compositions wherein said separated pharmaceutical compositions may be provided in form of a kit of parts. The compounds are, preferably, administered in conventional dosage forms prepared by combining the drugs with standard pharmaceutical carriers according to conventional procedures. These procedures may involve mixing, granulating and compressing or dissolving the ingredients as appropriate to the desired preparation. It will be appreciated that the form and character of the pharmaceutically acceptable carrier or diluent is dictated by the amount of active ingredient with which it is to be combined, the route of administration and other well-known variables.

The carrier(s) must be acceptable in the sense of being compatible with the other ingredients of the formulation and being not deleterious to the recipient thereof. The pharmaceutical carrier employed may be, for example, either a solid, a gel or a liquid. Exemplary of solid carriers are lactose, terra alba, sucrose, talc, gelatin, agar, pectin, acacia, magnesium stearate, stearic acid and the like. Exemplary of liquid carriers are phosphate-buffered saline solution, syrup, oil such as peanut oil and olive oil, water, emulsions, various types of wetting agents, sterile solutions and the like. Similarly, the carrier or diluent may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pennsylvania. The diluent(s) is/are selected so as not to affect the biological activity of the combination. Examples of such diluents are distilled water, physiological saline, Ringer's solutions, dextrose solution, and Hank's solution. In addition, the pharmaceutical composition or formulation may also include other carriers, adjuvants, or nontoxic, nontherapeutic, nonimmunogenic stabilizers and the like.

A therapeutically effective dose refers to an amount of the compounds to be used in a pharmaceutical composition of the present invention which prevents, ameliorates or treats the symptoms accompanying a disease or condition referred to in this specification. Therapeutic efficacy and toxicity of such compounds can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50. The dosage regimen will be determined by the attending physician and other clinical factors; preferably in accordance with any one of the above described methods. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment. A typical dose can be, for example, in the range of 1 to 1000 $\mu$g; however, doses below or above this exemplary range are envisioned, especially considering the aforementioned factors. Generally, the regimen as a regular administration of the pharmaceutical composition should be in the range of 1 $\mu$g to 10 mg units per day. If the regimen is a continuous infusion, it should also be in the range of 1 $\mu$g to 10 mg units per kilogram of body weight per minute, respectively. Progress can be monitored by periodic assessment. However, depending on the subject and the mode of administration, the quantity of substance administration may vary over a wide range to provide from about 0.01 mg per kg body mass to about 10 mg per kg body mass. In case a viral vector, in particular adeno-associated viral vector, is administered, preferred doses are from 5 x $10^{11}$, to 2 x $10^{13}$ viral particles or viral genomes / kg body weight; as will be understood, these exemplary doses may be modified depending, in addition to the factors described above, on additional factors like type of virus, target organ, and the like. Preferably, the dose is adjusted such that the components, in particular the Cas nuclease and the Cas inhibitor, are provided to at least 25%, more preferably at least 50%, most preferably at least 75% of host cells for which administration is intended. The pharmaceutical compositions and formulations referred to herein are administered at least once in order to treat or ameliorate or prevent a disease or condition recited in this specification. However, the said pharmaceutical compositions may be administered more than one time, for example from one to four times daily up to a non-limited number of days.

Specific pharmaceutical compositions are prepared in a manner well known in the pharmaceutical art and comprise at least the active compound or compounds referred to herein above in admixture or otherwise associated with a pharmaceutically acceptable carrier or diluent. For making those specific pharmaceutical compositions, the active compound(s) will usually be mixed with a carrier or the diluent, or enclosed or encapsulated in a capsule, sachet, cachet, paper or other suitable containers or vehicles. The resulting formulations are to be adopted to the mode of administration, i.e. in the forms of tablets, capsules, suppositories, solutions, suspensions or the like. Dosage recommendations shall be indicated in the prescriber's or user's instructions in order to anticipate dose adjustments depending on the considered recipient.

The present invention further relates to a polypeptide or polypeptide complex comprising (i) a Cas nuclease and (ii) a low-affinity Cas inhibitor.

As indicated herein above, the polypeptide comprising (i) a Cas nuclease and (ii) a low-affinity Cas inhibitor, preferably, is a fusion polypeptide. The terms "polypeptide" and "fusion polypeptide", as used herein, preferably encompass variants of said polypeptides and fusion polypeptides, the terms "polypeptide variant" and "fusion polypeptide variant" relating to any chemical molecule comprising at least one polypeptide or fusion polypeptide as specified elsewhere herein, having the indicated activity, but differing in primary structure from said polypeptide or fusion polypeptide indicated above. Thus, the polypeptide variant, preferably, is a mutein having the indicated activity. Preferably, the polypeptide variant comprises a peptide having an amino acid sequence corresponding to an amino acid sequence of 20 to 1000, more preferably 50 to 500, even more preferably 100 to 250 consecutive amino acids comprised in a polypeptide as specified above. Moreover, also encompassed are further (fusion) polypeptide variants of the aforementioned polypeptides. Such (fusion) polypeptide variants have at least essentially the same biological activity as the specific polypeptides. Moreover, it is to be understood that a (fusion) polypeptide variant as referred to in accordance with the present invention shall have an amino acid sequence which differs due to at least one amino acid substitution, deletion and/or addition, wherein the amino acid sequence of the variant is still, preferably, at least 50%, 60%, 70%, 80%, 85%, 90%, 92%, 95%, 97%, 98%, or 99% identical with the amino acid sequence of the specific (fusion) polypeptide. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the sequence it is compared to for optimal alignment. The percentage is calculated by determining, preferably over the whole length of the polypeptide, the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman (1981), by the homology alignment algorithm of Needleman and Wunsch (1970), by the search for similarity method of Pearson and Lipman (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. (Fusion) polypeptide variants referred to herein may be allelic variants or any other species specific homologs, paralogs, or orthologs. Moreover, the (fusion) polypeptide variants referred to herein include fragments of the specific polypeptides or the aforementioned types of (fusion) polypeptide variants as long as these fragments and/or variants have the biological activity as referred to above. Such fragments may be or may be derived from, e.g., degradation products or splice variants of the polypeptides. Further included are variants which differ due to posttranslational modifications such as phosphorylation, glycosylation, ubiquitinylation, sumoylation, or myristylation, by including non-natural amino acids, and/or by being peptidomimetics. Preferably, the polypeptide or polypeptide complex comprising (i) a Cas nuclease and (ii) a low-affinity Cas inhibitor comprises a Cas nuclease as specified herein above and/or a low-affinity Cas inhibitor as specified herein above. More preferably, the polypeptide comprising (i) a Cas nuclease and (ii) a low-affinity Cas inhibitor is a fusion polypeptide comprising, preferably consisting of, a Cas nuclease as specified herein above and/or a low-affinity Cas inhibitor as specified herein above. More preferably, the polypeptide comprises the amino acid sequence of at least one of SEQ ID NOs:15 to 28.

The present invention also relates to a composition according to the present invention or a polypeptide or polypeptide complex according to the present invention for use in medicine; and/or for use in treating and/or preventing genetic disease, neurodegenerative disease, cancer, and/or infectious disease.

The means and methods of the present invention are, in principle, usable in treatment and/or prevention of each and every disease for which genetic or epigenetic modification of a cell preferably a specific type of cell, or modification of expression of a target gene, is considered beneficial. Such is the case in particular in genetic disease, neurodegenerative disease, cancer, and infectious disease. As used herein, the term "genetic modification", preferably, includes modification of any kind of nucleic acid comprised in a host cell at a given time, including nuclear DNA, organelle DNA (mitochondrial DNA or plastid DNA), but also nucleic acid from an infectious agent, either as free nucleic acid or covalently connected to the DNA of the host cell. Preferably, genetic modification is modification of nucleic acid, preferably DNA, present in the nucleus of a host cell. More preferably, genetic modification is modification of the nucleic acid sequence of at least one gene present in the nucleus of a host cell.

The term "treatment" refers to an amelioration of the diseases or disorders referred to herein or the symptoms accompanied therewith to a significant extent. Said treating as used herein also includes an entire restoration of the health with respect to the diseases or disorders referred to herein. It is to be understood that treating as used in accordance with the present invention may not be effective in all subjects to be treated. However, the term shall require that, preferably, a statistically significant portion of subjects suffering from a disease or disorder referred to herein can be successfully treated. Whether a portion is statistically significant can be determined without further ado by the person skilled in the art using various well known statistic evaluation tools, e.g., determination of confidence intervals, p-value determination, Student's t-test, Mann-Whitney test etc. Preferred confidence intervals are at least 90%, at least 95%, at least 97%, at least 98% or at least 99 %. The p-values are, preferably, 0.1, 0.05, 0.01, 0.005, or 0.001. Preferably, the treatment shall be effective for at least 10%, at least 20%, at least 50%, at least 60%, at least 70%, at least 80%, or at least 90% of the subjects of a given cohort or population.

The term "preventing" refers to retaining health with respect to the diseases or disorders referred to herein for a certain period of time in a subject. It will be understood that said period of time is dependent on a variety of individual factors of the subject and the specific preventive treatment. It is to be understood that prevention may not be effective in all subjects treated with the compound according to the present invention. However, the term requires that, preferably, a statistically significant portion of subjects of a cohort or population are effectively prevented from suffering from a disease or disorder referred to herein or its accompanying symptoms. Preferably, a cohort or population of subjects is envisaged in this context which normally, i.e. without preventive measures according to the present invention, would develop a disease or disorder as referred to herein. Whether a portion is statistically significant can be determined without further ado by the person skilled in the art using various well known statistic evaluation tools discussed elsewhere in this specification.

The term "genetic disease", as used herein, relates to a disease causally linked to one or more modifications, preferably mutations, in the genome of an individual. Thus, preferably, the genetic disease is causally linked to one or more epigenetic changes, more preferably is causally linked to one or more genetic mutations. Preferably, the genetic disease is a monogenic disease, i.e., the disorder and its symptoms are essentially caused by a genetic change in one gene. As will be understood, symptoms of a genetic disease often are caused by expression of a mutated gene and/or lack of expression of a gene providing normal function of the gene product in one or more specific tissue(s) and/or cell type(s). Thus, it may be preferable to genetically modify by Cas activity only those cells in which the mutation contributes to disease. Preferably, the genetic disease is Duchenne muscular dystrophy, Huntington's disease, Hemophilia A/B, cystic fibrosis, myotubular myopathy, a glycogen storage disorder, or sickle cell anemia, the causes and symptoms of which are known to the skilled person from textbooks of medicine.

The term "neurodegenerative disease" relates to a disease caused by progressive loss of structure and/or function of neurons in the peripheral and/or central nervous system of an individual. Preferably, the neurodegenerative disease is a neurodegenerative disease of motoneurons and/or a neurodegenerative disease of the central nervous system. Preferably, the neurodegenerative disease is Alzheimer's disease, amyotrophic lateral sclerosis (ALS), Parkinson's disease, or a spinocerebellar ataxia, preferably spinocerebellar ataxia type 1 (SCA1). As will be understood, many neurodegenerative diseases are genetic diseases.

The term "cancer" is, in principle, understood by the skilled person and relates to a disease of an animal, including man, characterized by uncontrolled growth by a group of body cells ("cancer cells"). This uncontrolled growth may be accompanied by intrusion into and destruction of surrounding tissue and possibly spread of cancer cells to other locations in the body. Preferably, also included by the term cancer is a relapse. Thus, preferably, the cancer is a non-solid cancer, e.g. a leukemia, or is a tumor of a solid cancer, a metastasis, or a relapse thereof, in particular is hepatocellular carcinoma, pancreatic cancer, osteosarcoma, leukemia or colorectal cancer. As is known to the skilled person, cancer cells accumulate mutations in particular in oncogenes or in tumor-suppressor genes, which may be amenable to correction by genetic modification. Moreover, the means and methods of the present invention may be used to induce cell death, e.g. via apoptosis, specifically in cancer cells. Preferably, treating cancer is reducing tumor and/or cancer cell burden in a subject. As will be understood by the skilled person, effectiveness of treatment of e.g. cancer is dependent on a variety of factors including, e.g. cancer stage and cancer type. As will also be understood by the skilled person, in case the disease is cancer, the disease preferably is treated.

The term "infectious disease" is, in principle, understood by the skilled person. Preferably, the term as used herein relates to an infectious disease in which the replicative cycle of the infectious agent comprises at least one stage in which the genome of the infectious agent is present in a permissive host cell. Thus the infectious disease, preferably, is a viral infection, preferably is immunodeficiency virus infection, herpes virus infection, papillomavirus infection, or hepatitis B virus infection.

The present invention further relates to a kit comprising a composition according to the present invention or a polypeptide or polypeptide complex according to the present invention; comprised in a housing.

The term "kit", as used herein, refers to a collection of the aforementioned compounds, means or reagents of the present invention which may or may not be packaged together. The components of the kit may be comprised by separate vials (i.e. as a kit of separate parts) or provided in a single vial. Moreover, it is to be understood that the kit of the present invention, preferably, is to be used for practicing the methods referred to elsewhere herein. It is, in an embodiment, envisaged that all components are provided in a ready-to-use manner for practicing the methods referred to above. Further, the kit, preferably, contains instructions for carrying out said methods. The instructions can be provided by a user's manual in paper or electronic form. In addition, the manual may comprise instructions for interpreting the results obtained when carrying out the aforementioned methods using the kit of the present invention. Preferably, the kit comprises further components. Preferably, the kit further comprises a polynucleotide encoding at least one guide RNA (gRNA).

Also preferably, the kit further comprises at least one delivery means for at least one component it comprises, the term "delivery means" relating to any means suitable to mediate entry of a polynucleotide, polypeptide, and/or host cell of the kit to enter the relevant site in the body of a subject. Preferably, in case the kit comprises a host cell of the invention, the relevant site, preferably, is the blood stream, a tumor mass, or a body cavity. Preferably, in case the kit comprises a polynucleotide or a polypeptide of the invention, the relevant site preferably is the interior of a host cell. Suitable delivery means are known in the art and include in particular transfection reagents, packaging means, and the like. Preferably, the polynucleotides of the present invention are pre-packaged in a delivery means, e.g. in viral particles.

The present invention also relates to a device for modifying a target site in a host cell, comprising (i) a composition according to the present invention or a polypeptide or polypeptide complex according to the present invention; and (ii) a contacting unit adapted for contacting said host cell with said composition, polypeptide, or polypeptide complex.

The term "device", as used herein relates to a system of means comprising at least the means operatively linked to each other as to allow administration of the compound or of the composition of the present invention. Preferred means for contacting host cells with compounds are well known in the art. How to link the means in an operating manner will depend on the type of means included into the device and on the kind of administration envisaged. Preferably, the means are comprised by a single device in such a case. Said device may accordingly include a delivery unit for the administration of the compound or composition to the host cells and an incubation unit for performing the contacting step. However, it is also contemplated that the means of the current invention may appear as separate devices in such an embodiment and are, preferably, packaged together as a kit. The person skilled in the art will realize how to link the means without further ado. Preferred devices are those which can be applied without the particular knowledge of a specialized technician.

The present invention further relates to a use of (i) composition according to the present invention or (ii) a polypeptide or polypeptide complex according to the present invention; for modifying a target site in a host cell.

The present invention also relates to a method for improving specificity of a Cas nuclease, comprising

a) providing a Cas nuclease; and
b) contacting said Cas nuclease with a low-affinity Cas inhibitor or a sub-inhibitory concentration of a Cas inhibitor; and
c) thereby improving specificity of said Cas enzyme.

The method for improving specificity of a Cas nuclease of the present invention, preferably, is an in vitro method. The method may, however, also be an in vivo method. Moreover, it may comprise steps in addition to those explicitly mentioned above. Moreover, one or more of said steps may be performed by automated equipment.

Furthermore, the present invention relates to a method for identifying a low-affinity Cas nuclease inhibitor comprising

a) providing a representation of editing efficiency in dependence of Cas inhibitor strength for at least one target site and for at least one off-target site;
b) determining editing efficiency in the presence of at least one candidate low-affinity Cas inhibitor for said least one target site and for said at least one off-target site;
c) comparing the editing efficiency obtained in step b) to the representation obtained in step a); and, thereby,
d) identifying a low-affinity Cas nuclease inhibitor.

The method for identifying a low-affinity Cas nuclease inhibitor of the present invention, preferably, is an in vitro method. Moreover, it may comprise steps in addition to those explicitly mentioned above. Moreover, one or more of said steps may be performed by automated equipment.

The term "representation of editing efficiency in dependence of Cas inhibitor strength" relates to a, preferably graphical, representation indicating the relationship between Cas inhibitor strength and editing efficiency for a given target site or off-target site. Thus, providing said representation preferably comprises determining editing frequency at a target site or off-target site for a Cas nuclease in the absence of a Cas inhibitor and in the presence of at least one Cas-inhibitor; more preferably, said representation is provided for at least two, more preferably at least three, non-identical Cas inhibitors, wherein said Cas inhibitors preferably have different activity in inhibiting said Cas nuclease, i.e., preferably, have different inhibitor strength. Thus, preferably, at least one Cas inhibitor is a low-affinity Cas inhibitor. Preferably, step a) providing a representation of editing efficiency in dependence of Cas inhibitor strength for at least one target site and for at least one off-target site; and/or step b) determining editing efficiency in the presence of at least one candidate low-affinity Cas inhibitor for said least one target site and for said at least one off-target site; are performed as shown herein in the Examples. Preferably, the representation of editing efficiency is provided by model simulations, more preferably model simulations as specified herein in the Examples.

Preferably, a low-affinity Cas nuclease inhibitor is identified if it is found in comparing step c) that the value determined for the editing efficiency of the candidate low-affinity Cas inhibitor lies between the value of the editing efficiency determined in the absence of a Cas inhibitor and the value determined for the editing efficiency in the presence of the Cas inhibitor with the highest inhibitory activity. More preferably, a low-affinity Cas nuclease inhibitor is identified if it is found in comparing step c) that the value determined for the editing efficiency of the candidate low-affinity Cas inhibitor is of from 0.01% to 90%, preferably of from 0.1 to 50%, more preferably of from 0.5% to 25%, still more preferably of from 1% to 10% of the value of the editing efficiency determined in the absence of a Cas inhibitor. More preferably, a low-affinity Cas nuclease inhibitor is identified in case the off-target editing efficiency determined in step b) is at most 10%, preferably at most 5%, more preferably at most 2%, still more preferably at most 1% of the value of the editing efficiency determined in the absence of a Cas inhibitor. Most preferably, a low-affinity Cas nuclease inhibitor is identified in case the editing efficiency determined in step b) lies within the region of the maximal slope of the target editing efficiency curve of step a). As will be understood by the skilled person, the shape of the representation of the editing efficiency is dependent on Cas inhibitor strength, target site selection, and other factors.

Preferably, the method for identifying a low-affinity Cas nuclease inhibitor further comprises determining inhibitor strength of said candidate low-affinity Cas inhibitor. More preferably, in such case a low-affinity Cas inhibitor is identified in case the inhibitor strength determined in step b) is in the range of from 50% to 0.1%, preferably of from 20% to 1%, more preferably of from 10% to 2%, most preferably about 5% of the corresponding wildtype Cas inhibitor. Also preferably, determining inhibitor strength of said candidate low-affinity Cas inhibitor comprises comparing target editing efficiency and/or off-target editing efficiency obtained in step b) to the representation of editing efficiency obtained in step a).

In view of the above, the following embodiments are particularly envisaged:

1. A method for modifying a target site in a host cell comprising contacting said host cell with a Cas nuclease, wherein said host cell is further contacted with a low-affinity Cas inhibitor and/or a sub-inhibitory concentration of a Cas inhibitor.

2. The method of embodiment 1, wherein the ratio of an editing frequency of the target site to an editing frequency of any off-target site is at least 2, preferably at least 5, more preferably at least 7, still more preferably at least 10, still more preferably at least 15, even more preferably at least 18, most preferably at least 20.

3. The method of embodiment 1 or 2, wherein the frequency of off-target modification is decreased by at least 2-fold, preferably at least 3-fold, more preferably at least 10-fold, most preferably at least 15-fold, compared to the frequency of off-target modification in the absence of contacting said Cas nuclease with a low-affinity Cas inhibitor or a sub-inhibitory concentration of a Cas inhibitor.

4. The method of any one of embodiments 1 to 3, wherein said low-affinity Cas inhibitor has an affinity of from 0.01% to 90%, preferably of from 0.1 to 50%, more preferably of from 0.5% to 25%, still more preferably of from 1% to 10%, of the corresponding Cas inhibitor.

5. The method of any one of embodiments 1 to 4, wherein said Cas nuclease is a Cas9 endonuclease.

6. The method of any one of embodiments 1 to 5, wherein said Cas inhibitor is an Acr polypeptide, preferably is an AcrIIA4 polypeptide.

7. The method of any one of embodiments 1 to 6, wherein said low-affinity Cas inhibitor is a mutein of the AcrIIA4 polypeptide comprising at least one mutation selected from the list consisting of (i) M77A; (ii) D76A/M77A; (iii); (iv) D14A; (v) D14A/Y15A; (vi) D14A/G38A; (vii) G38A; (viii) D37A/G38A; (ix) D14A/G38A, (x) a deletion of amino acids N64/Q65/E66 and an insertion of a LOV-domain; (xi) a deletion of amino acids N64/Q65/E66; (xii) an insertion of a LOV-domain comprising T406A, T407A, G528A, and N538E mutations; and (xiii) any combination of (i) to (xii).

8. The method of any one of embodiments 1 to 7, wherein said low-affinity Cas inhibitor is a mutein of the AcrIIA4 polypeptide comprising at least one mutation selected from the list consisting of (i) N39A; (ii) D14A/G38A; (iii) a deletion of amino acids N64/Q65/E66 and an insertion of a LOV-domain comprising T406A, T407A, G528A, and N538E mutations; and (iv) any combination of (i) to (iii).

9. The method of any one of embodiments 1 to 8, wherein said contacting said Cas nuclease with a low-affinity Cas inhibitor comprises providing a fusion polypeptide of said Cas nuclease with said low-affinity Cas inhibitor, preferably via a linker peptide.

10. The method of any one of embodiments 1 to 9, wherein said contacting said Cas nuclease with a low-affinity Cas inhibitor comprises providing a fusion polypeptide of said Cas nuclease with said low-affinity Cas inhibitor via a GS and/or GG-comprising linker, preferably a GGSG (SEQ ID NO:13)-comprising linker, more preferably a $(GGSG)_n$ linker, with n being an integer of from 1 to 100, preferably of from 2 to 50, more preferably of from 3 to 25, even more preferably being about 10, most preferably being 10.

11. The method of any one of embodiments 1 to 10, wherein contacting said Cas nuclease with a sub-inhibitory concentration of a Cas inhibitor comprises contacting said Cas nuclease with a Cas inhibitor at a molar ratio Cas nuclease:Cas inhibitor of 10:1 to 1:1, preferably of from 8:1 to 1.5:1, more preferably of from 6:1 to 1.75:1, even more preferably from 5:1 to 2:1, still more preferably of from 4:1 to 2.2:1, most preferably of about 3:1.

12. The method of any one of embodiments 1 to 11, wherein said target site is a target gene.

13. The method of any one of embodiments 1 to 12, wherein said method is a method of specifically modifying a gene and/or modifying expression of a gene in a host cell, preferably of specifically modifying a gene in a host cell.

14. The method of any one of embodiments 1 to 13, wherein said method is an in vitro method.

15. The method of any one of embodiments 1 to 14, wherein said method further comprises contacting said host cell with at least one guide RNA.

16. A composition comprising or causing expression in a host cell of a Cas nuclease and (i) a low-affinity Cas inhibitor and/or (ii) a sub-inhibitory concentration of a Cas inhibitor.

17. A polypeptide or polypeptide complex comprising (i) a Cas nuclease and (ii) a low-affinity Cas inhibitor.

18. The composition according to embodiment 16 or the polypeptide or polypeptide complex according to embodiment 17, wherein said low-affinity Cas inhibitor has an affinity for said Cas nuclease of from 0.1% to 90% of the corresponding Cas inhibitor.

19. The composition according to embodiment 16 or 18 or the polypeptide or polypeptide complex according to embodiment 17 or 18, wherein said Cas nuclease and said low-affinity Cas inhibitor are comprised as a fusion polypeptide.

20. The composition according to embodiment 16, 18, or 19 or the polypeptide or polypeptide complex according to any one of embodiments 17 to 19, wherein said low-affinity Cas inhibitor has an affinity of from 0.01% to 90%, preferably of from 0.1 to 50%, more preferably of from 0.5% to 25%, still more preferably of from 1% to 10%, of the corresponding Cas inhibitor.

21. The composition according to embodiment 16 or any one of embodiments 17 to 20 or the polypeptide or polypeptide complex according to any one of embodiments 17 to 20, wherein said Cas nuclease is a Cas9 endonuclease.

22. The composition according to embodiment 16 or any one of embodiments 17 to 21 or the polypeptide or polypeptide complex according to any one of embodiments 17 to 21, wherein said Cas inhibitor is an Acr polypeptide, preferably is an AcrIIA4 polypeptide.
23. The composition according to embodiment 16 or any one of embodiments 17 to 22 or the polypeptide or polypeptide complex according to any one of embodiments 17 to 22, wherein said low-affinity Cas inhibitor is a mutein of the AcrIIA4 polypeptide comprising at least one mutation selected from the list consisting of (i) M77A; (ii) D76A/M77A; (iii); (iv) D14A; (v) D14A/Y15A; (vi) D14A/G38A; (vii) G38A; (viii) D37A/G38A; (ix) D14A/G38A, (x) a deletion of amino acids N64/Q65/E66 and an insertion of a LOV-domain; (xi) a deletion of amino acids N64/Q65/E66; (xii) an insertion of a LOV-domain comprising T406A, T407A, G528A, and N538E mutations; and (xiii) any combination of (i) to (xii).
24. The composition according to embodiment 16 or any one of embodiments 17 to 23 or the polypeptide or polypeptide complex according to any one of embodiments 17 to 23, wherein said low-affinity Cas inhibitor is a mutein of the AcrIIA4 polypeptide comprising at least one mutation selected from the list consisting of (i) N39A; (ii) D14A/G38A; (iii) a deletion of amino acids N64/Q65/E66 and an insertion of a LOV-domain comprising T406A, T407A, G528A, and N538E mutations; and (iv) any combination of (i) to (iii).
25. A composition according to embodiment 16 or any one of embodiments 17 to 24 or a polypeptide or polypeptide complex according to any one of embodiments 17 to 24 for use in medicine.
26. A composition according to embodiment 16 or any one of embodiments 17 to 24 or a polypeptide or polypeptide complex according to any one of embodiments 17 to 24 for use in treating and/or preventing genetic disease, neurodegenerative disease, cancer, and/or infectious disease.
27. A kit comprising composition according to embodiment 16 or any one of embodiments 17 to 24 or a polypeptide or polypeptide complex according to any one of embodiments 17 to 24; comprised in a housing.
28. A device for modifying a target site in a host cell, comprising

(i) composition according to embodiment 16 or any one of embodiments 17 to 24 or a polypeptide or polypeptide complex according to any one of embodiments 17 to 24; and
(ii) a contacting unit adapted for contacting said host cell with said composition, polypeptide, or polypeptide complex.

29. Use of (i) composition according to embodiment 16 or any one of embodiments 17 to 24 or (ii) a polypeptide or polypeptide complex according to any one of embodiments 17 to 24; for modifying a target site in a host cell.
30. A method for improving specificity of a Cas nuclease, comprising

a) providing a Cas nuclease; and
b) contacting said Cas nuclease with a low-affinity Cas inhibitor or a sub-inhibitory concentration of a Cas inhibitor; and
c) thereby improving specificity of said Cas enzyme.

31. The method of embodiment 30, wherein improving specificity of said Cas enzyme comprises reducing off-target activity by at least 2-fold, preferably at least 3-fold, more preferably at least 10-fold, most preferably at least 15-fold, compared to off-target modification in the absence of contacting said Cas nuclease with a low-affinity Cas inhibitor or a sub-inhibitory concentration of a Cas inhibitor.
32. The method of embodiment 30 or 31, wherein contacting said Cas nuclease with a sub-inhibitory concentration of a Cas inhibitor comprises contacting said Cas nuclease with a Cas inhibitor at a molar ratio Cas nuclease:Cas inhibitor of 10:1 to 1:1, preferably of from 8:1 to 1.5:1, more preferably of from 6:1 to 1.75:1, even more preferably from 5:1 to 2:1, still more preferably of from 4:1 to 2.2:1, most preferably of about 3:1.
33. The method of any one of embodiments 30 to 32, wherein said low-affinity Cas inhibitor has an affinity of from 0.01% to 90%, preferably of from 0.1 to 50%, more preferably of from 0.5% to 25%, still more preferably of from 1% to 10%, of the corresponding Cas inhibitor.
34. The method of any one of embodiments 30 to 33, wherein said Cas nuclease is a Cas9 endonuclease.
35. The method of any one of embodiments 30 to 34, wherein said Cas inhibitor is an Acr polypeptide, preferably is an AcrIIA4 polypeptide.
36. The method of any one of embodiments 30 to 35, wherein said low-affinity Cas inhibitor is a mutein of the AcrIIA4 polypeptide comprising at least one mutation selected from the list consisting of (i) M77A; (ii) D76A/M77A; (iii); (iv) D14A; (v) D14A/Y15A; (vi) D14A/G38A; (vii) G38A; (viii) D37A/G38A; (ix) D14A/G38A, (x) a deletion of amino acids N64/Q65/E66 and an insertion of a LOV-domain; (xi) a deletion of amino acids N64/Q65/E66; (xii) an insertion of a LOV-domain comprising T406A, T407A, G528A, and N538E mutations; and (xiii) any combination of (i) to (xii).
37. The method of any one of embodiments 30 to 36, wherein said low-affinity Cas inhibitor is a mutein of the AcrIIA4 polypeptide comprising at least one mutation selected from the list consisting of (i) N39A; (ii) D14A/G38A; (iii) a deletion of amino acids N64/Q65/E66 and an insertion of a LOV-domain comprising T406A, T407A, G528A, and N538E mutations; and (iv) any combination of (i) to (iii).

38. The method of any one of embodiments 30 to 37, wherein said contacting said Cas nuclease with a low-affinity Cas inhibitor comprises providing a fusion polypeptide of said Cas nuclease with said low-affinity Cas inhibitor, preferably via a linker peptide.

39. The method of any one of embodiments 30 to 38, wherein said contacting said Cas nuclease with a low-affinity Cas inhibitor comprises providing a fusion polypeptide of said Cas nuclease with said low-affinity Cas inhibitor via a GS and/or GG-comprising linker, preferably a GGSG (SEQ ID NO:13)-comprising linker, more preferably a $(GGSG)_n$-linker, with n being an integer of from 1 to 100, preferably of from 2 to 50, more preferably of from 3 to 25, even more preferably being about 10, most preferably being 10.

40. A method for identifying a low-affinity Cas nuclease inhibitor comprising

a) providing a representation of editing efficiency in dependence of Cas inhibitor strength for at least one target site and for at least one off-target site;
b) determining editing efficiency in the presence of at least one candidate low-affinity Cas inhibitor for said least one target site and for said at least one off-target site;
c) comparing the editing efficiency obtained in step b) to the representation obtained in step a); and, thereby,
d) identifying a low-affinity Cas nuclease inhibitor.

41. The method of embodiment 40, wherein a low-affinity Cas nuclease inhibitor is identified in case the editing efficiency determined in step b) lies within the region of the maximal slope of the target editing efficiency curve of step a).

42. The method of embodiment 40 or 41, wherein a low-affinity Cas nuclease inhibitor is identified in case the off-target editing efficiency determined in step b) is at most 50%, preferably at most 20%, more preferably at most 10%, still more preferably at most 5%, even more preferably at most 2%, most preferably at most 1%, of the corresponding target editing efficiency.

43. The method of any one of embodiments 40 to 42, wherein said method further comprises determining inhibitor strength of said candidate low-affinity Cas inhibitor and wherein a low-affinity Cas inhibitor is identified in case the inhibitor strength determined in step b) is in the range of from 50% to 0.1%, preferably of from 20% to 1%, more preferably of from 10% to 2%, most preferably about 5% of the corresponding wt.

44. The method of embodiment 43, wherein said determining inhibitor strength of said candidate low-affinity Cas inhibitor comprises comparing target editing efficiency and/or off-target editing efficiency obtained in step b) to the representation of editing efficiency obtained in step a).

45. The method of any one of embodiments 40 to 44, wherein said representation of editing efficiency is provided by model simulations.

All references cited in this specification are herewith incorporated by reference with respect to their entire disclosure content and the disclosure content specifically mentioned in this specification.

Figure Legends

**Fig. 1. Kinetic insulation of CRISPR ON- and OFF-target effects by co-expression of anti-CRISPR proteins.** (**A**) Schematic of a model for Cas9 genome editing. After co-transfection with plasmids encoding Cas9 and sgRNA, plasmids are transcribed to Cas9-mRNA and sgRNA or degraded. Furthermore, the model describes the turnover of mRNAs, sgRNA, Cas9 protein, binding of sgRNA and Cas9, association of Cas9:sgRNA with the target gene as well as gene editing. $DNA_{site}$, un-edited target locus. $DNA_{edited}$, edited target locus. D:sgR:C, trimeric complex of DNA, sgRNA and Cas9. (**B**) Modeling of editing kinetics at high affinity (ON-target) and low affinity (OFF-target) sites. Left: The model relates transient sgRNA and Cas9 expression to a gene-modified fraction of cells. The final gene-edited fraction depends on the integral of Cas9:sgRNA complex expression (shaded areas in upper left panel). Right: Relation between editing efficiency and Cas9 activity (time integral of Cas9:sgRNA complex). The target affinity of a sgRNA determines the editing efficiency at a respective locus. At very large Cas9:sgRNA integrals, gene-edited fractions reach saturation, irrespective of the target affinity. (**C**) Schematic of constructs used for expression of Cas9, AcrIIA4 and sgRNAs. (**D**, **E**) Co-expressing mild doses of AcrIIA4 improves genome editing specificity. Cells were co-transfected with plasmids encoding AcrIIA4, Cas9 and a sgRNA targeting the AAVS1 locus and incubated for 72 h followed by T7 endonuclease assay. The AcrIIA4 vector dose used during transfection is indicated. 22 ng thereby corresponds to a 3-fold excess of Cas9/sgRNA vectors. (**D**) Representative gel image and (**E**) quantification of InDel frequencies. (**F**) AAV-mediated co-expression of AcrIIA4, Cas9 and a sgRNA targeting the HBB locus were incubated for 72 h followed by T7 endonuclease assay. (**E**, **F**). Bars indicate mean editing frequencies, dots are individual data points from n = 2 independent experiments.

**Fig. 2. CascAID design improves genome editing specificity.** (**A**) Schematic of CascAID constructs comprising Cas9 fused to an artificially weakened AcrIIA4 variant functioning as auto-inhibitory domain (AID). (**B-G**) Cells were co-transfected with plasmids encoding the indicated CascAID variant and a sgRNA targeting the AAVS1 (**B, C**), EMX1 (**D, E**) and HEK (**F**, **G**) locus and incubated for 72 h followed by T7 endonuclease assay. Representative gel images (**B**, **D**, **F**) and corresponding quantification of InDel frequencies (**C**, **E**, **G**). Data are means $\pm$ SD, dots are individual data points from n = 3 independent experiments. Ins. 5, insertion variant 5 (see table S2). wt, Cas9 fused to wild-type AcrIIA4.

**Fig. 3. A mathematical model of CascAID action explains improved specificity and informs experimental planning.** (**A**) Overview of the mathematical model of gene editing with CascAID constructs. The model accounts for turnover of plasmids, sgRNA, CascAID mRNA and protein, transition between the active and inhibited states ($CascAID_{inh}$), sgRNA-binding (CascAID:sgRNA, $CascAID_{inh}$:sgRNA), association with a target gene as well as gene editing. (**B**) Exemplary model fits to time-resolved T7 endonuclease assay measurements using the AAVS1 -targeting sgRNA and either wild-type Cas9 or the CascAID variant 'Ins. 5'. See fig. S8 for the full set of fits. (**C**) ON- and OFF target editing efficiencies for sgRNAs targeting the AAVS1, EMX1, RUNX1 or HEK locus, are shown together with model simulations of editing efficiencies for either wild-type Cas9 or the indicated CascAID variants. The model was calibrated with ON- and OFF-target editing efficiencies for AAVS1 and ON-target efficiencies for EMX1, RUNX1 and HEK. OFF-target editing measurements for EMX1, RUNX1 and HEK were used for model validation. (**D**, **E**) Kinetic insulation of ON- and OFF-target editing by CascAID variants. (**D**) Data points are shown together with inhibitor strengths as estimated by model fitting. Kinetic insulation is achieved for inhibitor strengths that fall between sigmoidal curves for ON- and OFF-target editing. (**E**) The calibrated model can be used to predict the ratio between ON- and OFF-target editing efficiencies resulting from CascAID variants. The inhibitor strength was defined as the fold-change relative to the inhibition rate of CascAID wt, i.e. Cas9 fused to wild-type AcrIIA4. (**F**) Model simulations of the ratio between ON- and OFF-target editing efficiencies relative to ON-target editing efficiency illustrate the trade-off between Cas9 fidelity and ON-target editing efficiency. CascAID variants can be selected based on highest tolerated OFF-target editing efficiencies.

**Fig. 4. Estimation of Cas9, AcrIIA4 and CascAID protein levels and corresponding model fits.** (**A**) To estimate the concentration of GFP-Cas9 and mCherry-AcrIIA4 after transient transfection, the scaling factors between measured GFP and mCherry fluorescence intensities and fluorophore concentrations were calculated by determining fluorescence intensity sums of image stacks of calibration cells (HeLa) stably expressing GFP and mCherry with known average fluorophore concentrations and cell volumes (n=50 cells) *(39)*. (**B**, **C**) The mathematical model describing the turnover of CascAID variants and editing of ON- as well as OFF targets was simultaneously fitted to time-resolved estimated protein concentrations and T7 assay measurements of gene editing efficiencies. (**B**) HEK cells were co-transfected with an AAVS1-targeting sgRNA and either (i) Cas9-GFP (dark green) or (ii) Cas9-GFP and mCherry-Acr (pink, Cas9+Acr, mCherry-Acr; light green, Cas9+Acr, Cas9-GFP). After transient transfection, average fluorescence intensities were determined for n = 50 cells per condition. From these, average protein concentrations were estimated by applying the scaling factor obtained from measurements in calibration cells (see panel **A** and Materials and Methods for details). (**C**) T7 assay measurements and corresponding model fits for Cas9 or CascAID variants (wt, wild-type; Ins. 5; N39A; D14A/G38A). The following measurements were used for model fitting (indicated by circles): (i) Time-resolved measurements of ON- and OFF-target editing efficiencies for AAVS1 after transfection with wild-type Cas9 or the indicated CascAID variants, (ii) measurements of ON- and OFF-target editing efficiencies by wild-type Cas9 for all four target genes, (iii) measurements of ON-target editing efficiencies for EMX1, RUNX1 and HEK. Based on estimates of model parameters, CascAID OFF-target efficiencies were predicted for EMX1, RUNX1 and HEK sgRNAs. Model predictions were consistent with measurements of OFF-target efficiencies (indicated by triangles).

**Fig. 5. Kinetic insulation of ON- and OFF-target editing events by CascAID variants.** The calibrated model was used to simulate editing efficiencies for ON- and OFF-targets for sgRNAs RUNX1 and HEK in relation to the strength of the Cas9-fused inhibitor. Gray lines indicate values for CascAID variants, ordered from weak to strong inhibition: CascAID D14A/G38A, CascAID Ins. 5, CascAID N39A, and CascAID wild-type. Editing efficiencies for ON- and OFF-targets follow sigmoidal curves dependent on the inhibitor strength. Inhibitor strengths of the CascAID variants 'D14A/G38A', 'Ins. 5' and 'N39A' are in the region of the maximal slope of the ON-target editing efficiency curve but below the region of maximal slope of the OFF-target editing efficiency curve indicating that these variants are well suited for insulation of ON- and OFF-target editing.

The following Examples shall merely illustrate the invention. They shall not be construed, whatsoever, to limit the scope of the invention.

Example 1

**Materials and Methods**

**Plasmids**

All vectors were created by classical restriction enzyme cloning. Oligonucleotides as well as synthetic, double-stranded DNA fragments (gBlocks) were obtained from Integrated DNA Technologies (IDT). Vectors expressing Cas9, Cas9 fused to GFP (Cas9-GFP), wild-type AcrIIA4, different AcrIIA4-LOV2 hybrids or a U6 promoter driven sgRNA bearing the improved F+E scaffold have been previously reported (Bubeck et al., Nat Methods 15, 924-927 (2018); Hoffmann et al., Nucleic Acids Res 271, (2019)); (see Addgene # 113033-113039). The mCherry-AcrIIA4 vector was created by fusing a mCherry coding sequence to the N-terminus of wild-type AcrIIA4 using overlap extension PCR. A construct expressing Cas9 fused to wild-type AcrIIA4 via a 40 residue GS linker was created by cloning a synthetic DNA fragment encoding the GS linker-AcrIIA4 fragment into vector CMV-SpyCas9 (Addgene # 103033) via EcoRI/HindIII. The AcrIIA4 fragment in the resulting construct was subsequently replaced by PCR fragments encoding AcrIIA4-LOV2 fusions or AcrIIA4 point mutants via BamHI/HindIII. To generate the AcrIIA4-LOV2 PCR fragments, our previously reported AcrIIA4-LOV2 vectors were employed as template (Bubeck et al., loc. cit.). The AcrIIA4 point mutants were created by first amplifying a vector encoding wild-type AcrIIA4 (Addgene # 113037) with 5'-phosphorylated primers introducing the point mutation(s). The resulting vectors were then used as template to generate PCR fragments encoding AcrIIA4 mutants. SgRNA expression vectors were created by inserting target-complementary sequences into vector pAAV-RSV-GFP-U6-sgRNA scaffold (Addgene # 113039) by oligo cloning via BbsI.

In all cloning procedures, PCRs were performed using Q5 Hot Start High-Fidelity DNA Polymerase (New England Biolabs, NEB) followed by agarose gel electrophoresis to analyze PCR products. Bands of the expected size were cut out from the gel and the DNA was purified by using a QIAquick Gel Extraction Kit (Qiagen). Restriction digests and ligations were performed with corresponding enzymes from New England Biolabs applying to the manufacturer's protocols. Following ligation, plasmids were transformed into chemically-competent Top10 cells and plasmids were extracted and purified using the QIAamp DNA Mini or Plasmid Plus Midi Kit (all from Qiagen).

**Cell Culture**

Prior to use, all cell lines were authenticated and tested negative for mycoplasma contamination via a commercial service (Multiplexion, Heidelberg). Cells were maintained at 5 % $CO_2$ and at 37 °C in a humidified incubator and passaged every 2-4 days, i.e. when reaching 70 - 90 % confluency. HEK 293T and HeLa cells were cultivated in 1 × DMEM supplemented with 2 mM L-glutamine, 100 U per mL penicillin, 100 μg per mL streptomycin (all ThermoFisher) and 10 % (v/v) fetal calf serum (Biochrom AG). The Huh-7 medium was additionally supplemented with 1 mM non-essential amino-acids (ThermoFisher).

**AAV lysate production**

Low-passage HEK 293T cells were employed for production of AAV-containing cell lysates. Cells were seeded into 6-well plates (CytoOne) at a density of 350,000 cells per well. The following day, cells were triple-transfected with (i) an AAV helper plasmid carrying AAV serotype 2 (AAV2) rep and cap genes, (ii) an adenoviral plasmid providing helper functions for AAV production, and (iii) the AAV vector plasmid using 1.33 μg of each construct and 8 μL of TurboFect Transfection Reagent (ThermoFisher) per well. The AAV vector plasmid encoded either (i) a U6-promoter driven sgRNA targeting the AAVS1 locus as well as an RSV promoter-driven GFP (used as transduction reporter), (ii) Cas9 or (iii) AcrIIA4. Three days post transfection, cells were collected in 300 μL PBS and subjected to five freeze-thaw cycles by alternating between snap freezing in liquid nitrogen and 37 °C in a water bath. Centrifugation at 18,000 × g was applied for 10 min to remove cell debris and the supernatant containing AAV particles were stored at 4 °C until use.

**T7 endonuclease assay and targeted amplicon sequencing**

Table 1 shows the genomic target/off-target sites relevant to this study:

For transfection-based T7 assays, HEK 293T cells were seeded at a density of 12,500 cells per well and a culture volume of 100 μl per well into 96 well-plates (Eppendorf). The following day, cells were transfected with jetPRIME® (Polyplus-transfection) using 0.3 μL jetPrime reagent per well and as detailed in the following. For experiments shown in Figs. 1E, S1, S2 and S3, cells were co-transfected with (i) 66 ng Cas9 expression construct, (ii) 66 ng of sgRNA constructs and (iii) different doses of Acr construct as indicated in the figures. To keep the total amount of DNA transfected constant between all samples, DNA was topped up to 200 ng per well using an irrelevant vector. For experiments shown in Fig. 2, cells were co-transfected with (i) 66 ng Cas9 or CascAID vector, (ii) 66 ng of sgRNA expression construct and (iii) 66 ng of an irrelevant stuffer DNA.

For transduction-based T7 assays, HEK 293T were seeded at a density of 3,500 cells per well and HeLa and Huh-7 cells were seeded at a density of 3,000 cells per well into 96 well-plates. The following day, cells were co-transduced with 33 μl Cas9 AAV lysate, 33 μl sgRNA AAV lysate and the indicated volume of AcrIIA4 AAV lysate. The transduction volume was always topped up with PBS to a total volume of 100 μl. The transduction was repeated 24 h after the first transduction. Seventy-two hours post transfection or post (first) transduction, the medium was aspirated and cells were lysed using DirectPCR lysis reagent (VIAGEN Biotech) supplemented with proteinase K (Sigma-Aldrich).

For T7 assays, the genomic target locus and relevant off target loci were PCR-amplified with primers flanking the corresponding ON-target/OFF-target sites, shown in Table 2, using Q5 Hot Start High-Fidelity DNA Polymerase (New England Biolabs). Five μl of PCR amplicon were diluted 1:4 in buffer 2 (NEB), and then heated up to 95 °C and slowly cooled down to room temperature to allow heteroduplex formation using nexus GSX1 Mastercycler (Eppendorf) and the following temperature steps: 95 °C/5 min, 95-85 °C at -2 °C per second, 85-25 °C at -0.1 °C per second. Then, 0.5 μl T7 endonuclease (NEB) was added, samples were mixed and incubated for 15 min at 37 °C. Next, gel loading dye (NEB) supplemented with 1 % GelRed (Biotium) was added and samples were then loaded onto 2 % Tris-borate-EDTA agarose gels. Voltage (100 volts) was applied for 40 min to resolve DNA fragments. The Gel iX20 system equipped with a 2.8 megapixel/14 bit scientific-grade CCD camera (INTAS) was used for gel documentation. To calculate the indel percentages from the gel images, T7 bands were quantified using the ImageJ gel analysis tool. Peak areas were measured and percentages of insertions and deletions (indel(%)) were calculated using the formula indel (%) = 100×(1-(1-fraction cleaved)^(1/2)), whereas the fraction cleaved = ∑](Cleavage product bands)/∑(Cleavage product bands+PCR input band).

For targeted amplicon sequencing, a 1st step PCR was performed by PCR amplifying the genomic ON-target/OFF-target loci with primers carrying 5' Illumina Nextera sequencing adaptors. The $2^{nd}$ step PCR for introducing barcodes, sequencing on a Illumina MiSeq machine and downstream bioinformatics for quality control and calling of CRISPR-induced InDels was performed via the CRISPR/Cas9 commercial sequencing service (Microsynth) via their in-house pipelines.

**Fluorescence microscopy and image analysis**

Cells were seeded into 8-well Glass Bottom μ-Slides (Ibidi) at a density of 9,000 cells per well for HeLa and 10,000 cells per well for HEK 293T and a volume of 300 μl medium per well. The following day, cells were co-transfected with (i) 25 ng of Cas9-GFP, 25 ng sgRNA AAVS1 construct and 25 ng of stuffer DNA (pBluescript) or (ii) 25 ng of Cas9-GFP, 25 ng of mCherry-AcrIIA4 and 25 ng sgRNA AAVS1 construct using 0.2 μl JetPrime per well. Imaging was performed at 12 h, 18 h, 24 h, 48 h and 72 h post transfection using a Leica SP8 confocal laser scanning microscope equipped with automated CO2 and temperature control, a UV, argon, and a solid state laser, as well as a HCX PL APO 40x oil objective (N/A = 0.7). The identical imaging settings were applied to all samples as detailed in the following. GFP fluorescence was recorded using the 488 nm laser line for excitation and the detection wavelength was set to 493-578 nm. mCherry fluorescence was recorded using the 552 nm laser line for excitation and the detection wavelength was set to 578-789 nm. Laser power was 0.25 %, gain set to 800 V. For each field-of-view, a 40 μm Z-stack (40 slices) was recorded and five field-of-views were recorded per sample and time point. A single-plane bright field image was recorded in parallel. A previously reported HeLa reference cell line expressing known GFP and mCherry molecule numbers per cell (Kallenberger et al, Sci Signal 7, ra23 (2014)) was subjected to the identical imaging conditions.

For image analysis, cells were manually segmented using the freehand selection tool in ImageJ using the bright field channel and the area of each cell was measured. The segments were then applied to measure mean fluorescence in z-projections of the GFP and mCherry stacks. The number of fluorescent molecules per cell was then calculated using the following formula:

$$\mathrm{FM(sample)} = \frac{A(sample)\cdot I(sample)}{A(ref)\cdot I(ref)} \cdot \mathrm{FM(ref)},$$

whereby FM(sample) and FM(ref) represent the number of fluorescent molecules, A(sample) and A(ref) the cell area and I(sample) and I(ref) the fluorescence intensity, after background subtraction, in a particular cell in the sample cell or reference (ref) cell line, respectively.

**Mathematical modeling and parameter estimation**

To quantitatively describe gene editing dynamics by Cas9 or CascAID variants, an ODE model was developed. The model describes the transient expression of sgRNAs, Cas9 or CascAID mRNAs and Cas9 or CascAID proteins, binding of sgRNAs to Cas9 or CascAID variants, activation and inhibition of CascAID variants as well as gene editing by active complexes of Cas9 or CascAID variants and sgRNAs. A model without CascAID species was used for initial simulations that consisted of 9 equations containing a total of 11 parameters. Three types of models were defined for simultaneous model fitting to experimental data: (i) a model describing turnover of plasmids, mRNAs and proteins, consisting of 7 equations, (ii) a Cas9 model consisting of 39 equations, (iii) CascAID models containing 47 equations. A total of 32 parameters was estimated by model fitting to 75 data points. In the following, model assumptions and steps to iteratively refine the model shall be described.

The experimental dataset comprised measurements related to protein turnover and gene editing. However, several reactions in between were experimentally inaccessible. For this reason, we tried to limit the problem of parameter unidentifiability by parsimoniously defining model parameters. Taking into account that the sizes of plasmids for expressing sgRNAs, Cas9 or CascAID variants were of the same order of magnitude, the same degradation rate was assumed for all plasmids. Furthermore, the model assumes the same degradation rate $k_{deg,C}$ for Cas9 and all CascAID species (CascAID, $CascAID_{inh}$, CascAID:sgRNA, $CascAID_{inh}$:sgRNA) independent on their activation state and sgRNA binding. Similarly, degradation of different sgRNAs was described by one parameter $k_{deg,gRNA}$. If CascAID as part of complexes with sgRNA is degraded, it is assumed that sgRNA remains within the cell. Thereby, the model pertains flexibility regarding a potential sgRNA-rescuing effect of Cas9 in consistence with the observation that otherwise very short-lived sgRNA is protected from degradation after binding to Cas9 (Ma et al., J Cell Biol 214, 529-537 (2016)).

We assumed that binding of sgRNA to Cas9 or CascAID variants was fast compared to other processes such as translation or gene editing. A quasi-steady state was enforced by fixing the binding parameter $k_{gRNA,on}$ to a large value and effectively only estimating the dissociation constant $K_{d,gRNA} = k_{gRNA,off} / k_{gRNA,on}$. At first, we tried to fit the model with equal $K_{d,gRNA}$ values for the sgRNAs targeting four different genes. We realized that estimating $K_{d,gRNA}$ individually for sgRNAs resulted in a significantly improved model fit, indicated by a difference in the Akaike information criterion of $\Delta$AIC=53. This implies that affinities to Cas9 or CascAID variants varied between sgRNAs.

Similarly, we first assumed the same parameter for the maximal editing efficiency for experiments in all targeted genes. In the model, this parameter served as initial value $D_{tot}$ for the fraction of unedited genes. In case that all target sites in transfected cells can be edited, this parameter equals the percentage of transfected cells expressing sgRNA and Cas9 or CascAID variants. Estimating this parameter individually for the four edited genes significantly improved the model fit ($\Delta$AIC=42).

For CascAID variants, a constant activation parameter but individual inactivation parameters were defined. To directly estimate ratios of inhibitor strengths for CascAID variants with mutated AcrIIA4 ('Ins. 5', 'N39A', 'D14A/G38A'), inhibition parameters for these CascAID variants were defined as a product between the inhibition parameter for CascAID wt ($k_{inh,CascAID}$ ) and parameters $factor_{CascAID,Ins.\ 5}$, $factor_{CascAID,N39\ A}$ or $factor_{CascAID,D14\ A/G38\ A}$.

Furthermore, individual parameters were estimated for the unbinding of active Cas9:sgRNA or CascAID:sgRNA complexes to target sites of the four different genes. The same binding parameter $k_{on,target}$ was defined for all genes, in consistence with the observation that mismatches between sgRNAs and target sites rather take influence on the unbinding than on the binding kinetics (Ma et al. (2016) loc. cit.). To explain differences between ON-target and OFF-target editing, factors between unbinding rates of Cas9:sgRNA or CascAID:sgRNA complexes for ON- and OFF-targets were estimated separately for the four genes. For example, the unbinding rate from the OFF-target of EMX1 defined as the product of the unbinding rate $k_{EMX1,off}$ and $factor_{EMX1,OFF\ target}$.

Residuals between model observables and experimental measurements were weighted by the standard error of the mean if replicates for data points were available. Protein expression measurements were determined from averages of n = 50 cells. Quadruplicates were measured for AAVS1 editing efficiencies in case of Cas9 and all CascAID variants at 72 h. Triplicates were measured for EMX1, RUNX1 and HEK editing efficiencies. Single measurements were available for AAVS1 editing efficiencies at 24 h and 48 h. In this case, residuals were weighted using an error model. To this end, the linear error model $\varepsilon = m_1 y + m_2$ was fitted to all SEM values and editing efficiencies $y$ measured with replicates resulting in parameter estimates $m_1$ = 0.054 and $m_2$ =0.681.

Model simulations were performed with custom scripts in MATLAB (The Mathworks, Natick, MA, USA). For parameter estimations the MATLAB toolbox PottersWheel (www.potterswheel.de) was used (Maiwald et al., Bioinformatics 24, 2037-2043 (2008)). A total of 500 multi-start local optimizations were conducted followed by profile likelihood estimation to determine parameter confidence intervals. Parameter estimates, parameter bounds and parameter confidence intervals are listed in Table 5 below.

Example 2: **Results**

To investigate the possibility of insulating ON- and OFF-target editing events by fine-tuning Cas9 activity, we first devised a simple mathematical model consisting of coupled ordinary differential equations (ODEs). Our model captures the major molecular steps underlying Cas9-mediated editing in cells, namely (i) transient expression of sgRNA and Cas9 after transfection, (ii) formation of Cas9-sgRNA complexes, (iii) binding of the complex to a genomic target locus and (iv) target locus cleavage (Fig. 1A, Table 3). In this simple model, the level of Cas9-mediated target cleavage mainly depends on two parameters: The time integral of active Cas9-sgRNA complexes present in a cell and the affinity of the Cas9-sgRNA complex to a given genomic locus. High affinity of Cas9-sgRNA to a given locus as would be expected for perfect target sites would thereby result in a high percentage of gene-edited cells (Fig. 1B). In contrast, lower affinities as would be expected for OFF-target loci require larger time integrals of active Cas9-sgRNA complexes (Fig. 1B). Notably, at sufficiently large time frames, the editing of ON-target as well as OFF-target loci will both reach saturation. Importantly, our model qualitatively predicts that by fine-tuning the amount of (active) Cas9-sgRNA complex to specific levels, editing at "high-affinity" loci (ON-targets) can be insulated from editing at "low-affinity" loci (OFF-targets) (Fig. 1B).

We hypothesized that anti-CRISPR proteins such as AcrIIA4 could provide a simple means to fine-tune Cas9 activity to selected levels and, therefore, to test our model prediction. We thus co-transfected HEK 293T cells with vectors encoding AcrIIA4, Cas9 and a previously reported sgRNA targeting the human AAVS1 locus, which exhibits particularly strong OFF-target editing at two additional loci (Fig. 1C). During transfection, we used relatively low AcrIIA4 vector doses (~3- to 130-fold excess of Cas9 and sgRNA vector), as ON-target editing would be completely blocked at higher Acr doses. Seventy-two hours post transfection, we measured the frequency of insertions and deletions (InDels) at the AAVS1 locus and the two OFF-target loci using a T7 endonuclease assay. In line with the model prediction, we observed a potent reduction in OFF-target editing, but only mild reduction in ON-target editing at selected Acr vector doses (Fig. 1D and E).

To test whether this effect was independent of the specific cellular context and compatible with different modes of delivery, we packaged the different component of our system (Cas9, sgRNA and AcrIIA4) into Adeno-associated virus (AAV) vectors (Fig. 1C), which are prime vector candidates for gene therapy applications. For our experiments we chose AAV serotype-2, which is able to efficiently transduce various cell lines (Grimm et al. (2008), J Virol 82(12):5887). We infected HEK 293T (human embryonic kidney), HeLa (cervix carcinoma) and Huh-7 (hepatocellular carcinoma) cells with AAV2 particles encoding Cas9, a sgRNA targeting the AAVS1 locus as well as different doses of AcrIIA4 vector and measured InDel frequencies at the ON- and OFF-target loci. Again, at selected, low Acr doses, we observed potent ON-target editing, while OFF-target editing was effectively suppressed.

While co-expression of Acrs as shown above offers a highly flexible strategy to improve Cas9 specificity without the necessity of altering Cas9 or the sgRNA itself, this approach is rather sensitive with respect to the Acr dose. Moreover, the amounts of Cas9, the sgRNA and the Acr will be somewhat stochastic after co-delivery into a population of cells, i.e. the ratio of Cas9-sgRNA complex to Acr will vary between individual cells. We hypothesized, that a more robust fine-tuning of Cas9 activity could be achieved by covalently linking Acrs to Cas9 via genetic fusion, an approach we termed Cas9 coupling to artificial, inhibitory domains (CascAID). In the CascAID configuration, every Cas9 molecule carries its own, inhibitory domain and therefore exists in an equilibrium between an active or inactive state. The likelihood by which the active or inactive states are populated thereby depend on the strength of the fused Acr, i.e. by modulating the Acr strength Cas9 activity can be fine-tuned to desired levels (Fig. 2A). As one might expect, simply fusing wild-type AcrIIA4 to Cas9 blocks Cas9 activity (almost) entirely (Fig. 2B-G, wt samples). Thus, to enable the envisaged fine-tuning, we employed a previously reported set of AcrIIA4 domain insertion mutants (Bubeck et al., Nat Methods 15, 924-927 (2018)) as well as AcrIIA4 point mutants (Basgall et al., Microbiology 164, 464-474 (2018)) which display various Cas9 inhibition potencies when co-expressed with Cas9. We then fused these attenuated AcrIIA4 variants to the Cas9 C-terminus via long (40 residue) glycine-serine linkers and pre-screened the resulting, ten CascAID variants using the previously employed AAVS1 and HBB locus targeting sgRNAs. A number of CascAID variants not only showed ON-target editing efficiencies comparable to wild-type Cas9, but at the same time displayed strongly reduced OFF-target editing. We then selected three CascAID variants that showed different levels of specificity gain and compared their ON- and OFF-target editing frequencies with that of wild-type Cas9 using five different sgRNAs. Remarkably, genome editing specificity was markedly improved for all CascAID variants as we observed in T7 endonuclease experiments (Fig. 2B-G) and further confirmed by targeted amplicon sequencing. In several cases this improvement came at the cost of some reduction in ON-target editing, therefore suggesting a trade-off between the gain in specificity and reduction in off-target editing (Fig. 2B-G), as predicted by our model (Fig. 1A and B).

To quantitatively characterize the relation between ON-target editing efficiency and specificity in detail in context of our CascAID approach, we extended our initial model by including the inhibitory states of CascAID variants (Fig. 3A). In particular, the model explains the transient expression of sgRNA, Cas9 or CascAID mRNA, and Cas9 or CascAID protein in transfected cells. Cas9 and active as well as inactive CascAID proteins can be reversibly bound to sgRNA molecules. CascAID is reversibly inactivated by the fused Acr. Thereby, the model effectively describes how the ratio between activation and inactivation rates of CascAID variants is related to time integral of active complexes of CascAID:sgRNA.

The model was calibrated with experimental data on editing frequencies after transient expression of Cas9, CascAID containing unmodified AcrIIA4 as well as CascAID variants 'Ins. 5', 'N39A' and D14A/G38A' (Fig. 3B; see Fig.4 for the complete set of model fits and data). Moreover, the model was calibrated with time-lapse measurements of Cas9-GFP and mCherry-AcrIIA4 expression recorded by life-cell fluorescence microscopy. For parameter estimation, the model was fitted to time-resolved ON- and OFF-target editing efficiencies as observed for the AAVS1-targeting sgRNA in combination with different CascAID variants as well as wild-type Cas9. The model was further fitted to ON and OFF-target efficiencies measured for the EMX1, RUNX1 and HEK locus when using wild-type Cas9 as well as ON-target editing frequencies measured for the different CascAID variants. Corresponding OFF-target editing data for the CascAID variants were used for model validation. Remarkably, simulations using the calibrated model predicted the experimentally measured OFF-target rates in the CascAID validation dataset with high precision (Fig. 3C, 4).

Finally, the calibrated model was used to quantitatively dissect the kinetic insulation of ON- and OFF-target editing events by CascAID. Simulated editing efficiencies at ON- and OFF-target sites followed sigmoidal curves dependent on the strength of the Cas9-fused inhibitor (Fig. 3D and E and 5). Notably, inhibitor strengths of the CascAID variants 'D14A/G38A', 'Ins. 5' and 'N39A' are all in the regions of the maximal slope of the ON-target editing efficiency curves but below the region of maximal slopes of the OFF-target editing efficiency curves for the studied sgRNAs, explaining why these variants are well suited for insulation of ON- and OFF-target editing events. According to parameter estimates (Tables 4 and 5), transition rates to the inhibited state of the CascAID variants carrying AcrIIA4 mutants were decreased by factors between 16 and 19 relative to the CascAID variant containing wild-type AcrIIA4, explaining why these variants still enable potent ON-target editing. To further characterize the capability of tuning the activity of Cas9, we simulated the ratio between ON- and OFF-target editing efficiencies (Fig. 3E and F). The dependence of this ratio on the strength of the Cas9-fused inhibitor was reflected by sigmoidal curves for each of the edited genes (Fig. 3E). Notably, inhibitor strengths of the variants 'Ins. 5', 'N39A' and 'D14A/G38A' were in the upper-to-medium part of the sigmoidal curves (Fig. 3E). Taken together, these data show that the CascAID variants hit the desired "sweet spot" in the Cas9 activity profile characterized by efficient insulation of ON- and OFF-target events (Fig. 3F).

Literature cited:

Akcakayaet al., Nature 561: 416-419 (2018)
Basgall et al., Microbiology 164, 464-474 (2018)
Bubeck et al., Nat Methods 15, 924-927 (2018)
Dong et al., Nature 546: 436-439 (2017)
Grimm et al. (2008), J Virol 82(12):5887
Hoffmann et al., Nucleic Acids Res 271, (2019)
Kallenberger et al, Sci Signal 7, ra23 (2014)
Kleinstiver et al., Nature 529:490-495 (2016)
Kulcsar et al., Genome Biol 18:190 (2017),
Ma et al., J Cell Biol 214, 529-537 (2016)
Pawluk et al. (2016), Cell 167(7): 1829
Rauch et al. (2017), Cell 168(1-2): 150
Shin et al, Sci Adv 3, e1701620 (2017)

Table 1: target sites and Off-target sites

| **sgRNA** | **ON/OFF target** | **Target sequence (5' to 3')** | SEQ ID NO: |
|---|---|---|---|
| AAVS1 | ON | GGGAGGGAGAGCTTGGCAGG**GGG** | 29 |
| | OFF1 | GGGAAGGGGAGCTTGGCAGG**TGG** | 30 |
| | OFF2 | GGGAAGGTGAGCTTGGCAGG**TGG** | 31 |
| HBB | ON | CCACGTTCACCTTGCCCCAC**AGG** | 32 |
| | OFF | CCACATTCACCTTGCCCCAC**AGG** | 33 |
| EMX | ON | GAGTCCGAGCAGAAGAAGAA**GGG** | 34 |
| | OFF | GAGTTAGAGCAGAAGAAGAA**AGG** | 35 |
| HEK | ON | GGCACTGCGGCTGGAGGTGG**GGG** | 36 |
| | OFF | TGCACTGCGGCCGGAGGAGG**TGG** | 37 |
| RUNX | ON | GCATTTTCAGGAGGAAGCGA**TGG** | 38 |
| | OFF | GCATTTTCAGAAGGAAGCAA**GGG** | 39 |

Table 2: Primers

| Primer Name | Assay | Sequence (5' to 3') | SEQ ID NO: |
|---|---|---|---|
| AAVS1_ON_fw | T7 | TGGCTACTGGCCTTATCTCACAGG | 40 |
| AAVS1_ON_re | T7 | CTCTCTAGTCTGTGCTAGCTCTTCCAG | 41 |
| AAVS1_OFF1_fw | T7 | ACTCTTCTACCTTGCACGCCTTTGC | 42 |
| AAVS1_OFF1_re | T7 | CCTGCCTCCCATGCAAACAGTGTC | 43 |
| AAVS1_OFF2_fw | T7 | GAGCTGGTTTGCTTATGTGTGCAGG | 44 |
| AAVS1_OFF2_re | T7 | GCACTTCTCGCTGGCCACTTACAG | 45 |
| AAVS1_ON_seq_fw | Seq | TCGTCGGCAGCGTCAGATGTGTATAAGAGACAGGGGATCAGTGAAACGCACCAG | 46 |
| AAVS1_ON_seq_re | Seq | GTCTCGTGGGCTCGGAGATGTGTATAAGAGACAGGACACACCCCCATTTCCTGG | 47 |
| AAVS1_OFF1_seq_fw | Seq | TCGTCGGCAGCGTCAGATGTGTATAAGAGACAGGACTAAGGCAGAGAGACCGAGG | 48 |
| AAVS1_OFF1_seq_re | Seq | GTCTCGTGGGCTCGGAGATGTGTATAAGAGACAGATGTGTGAGCCACTTACATAGCAC | 49 |
| HBB_ON_fw | T7 | CAAGCGTCCCATAGACTCACCCTGAAG | 50 |
| HBB_ON_re | T7 | GTGCCAGAAGAGCCAAGGACAGGTAC | 51 |
| HBB_OFF_fw | T7 | ATGATCTCTGCCCCATCTATGCTTGG | 52 |

| | | | |
|---|---|---|---|
| HBB_OFF_re | T7 | TTGACCCACTGCATCAGAATCATTTGG | 53 |
| EMX_ON_fw | T7 | TCGTCGGCAGCGTCAGATGTGTATAAGAGACAGAGGTGAAGGTGTGGTTCCAG | 54 |
| EMX_ON_re | T7 | GTCTCGTGGGCTCGGAGATGTGTATAAGAGACAGAGTGGCCAGAGTCCAGCTT | 55 |
| EMX_OFF_fw | T7 | TCGTCGGCAGCGTCAGATGTGTATAAGAGACAGGACACCTTTTAAGATCTGACAGAGAA | 56 |
| EMX_OFF_re | T7 | GTCTCGTGGGCTCGGAGATGTGTATAAGAGACAGTGCACATGTATGTACAGGAGTCAT | 57 |
| HEK_ON_fw | T7 and Seq | TCGTCGGCAGCGTCAGATGTGTATAAGAGACAGGCCCTCCCCTCCCTTCAAGA | 58 |
| HEK_ON_re | T7 and Seq | GTCTCGTGGGCTCGGAGATGTGTATAAGAGACAGCCAGACTCCTTCTGGGGCCT | 59 |
| HEK_OFF_fw | T7 and Seq | TCGTCGGCAGCGTCAGATGTGTATAAGAGACAGCCTGGGGCATGGCTTCTGAG | 60 |
| HEK_OFF_re | T7 and Seq | GTCTCGTGGGCTCGGAGATGTGTATAAGAGACAGCTCAACCCAGGTGTTGGCCC | 61 |
| RUNX_ON_fw | T7 | TCGTCGGCAGCGTCAGATGTGTATAAGAGACAGTACAGGCAAAGCTGAGCAAA | 62 |
| RUNX_ON_re | T7 | GTCTCGTGGGCTCGGAGATGTGTATAAGAGACAGCCAGAGGTATCCAGCAGAGG | 63 |
| RUNX_OFF_fw | T7 | TCGTCGGCAGCGTCAGATGTGTATAAGAGACAGGCATGATACTTTGGGGGAGA | 64 |

| | | | |
|---|---|---|---|
| RUNX_OFF_rc | T7 | GTCTCGTGGGCTCGGAGATGTGTATAAGAGACAGTCTGATCAGCAATGTTGAGATG | 65 |

Table 3: Equations of the Cas9 gene editing model visualized in Fig. 1A.

| **Model equations** | **Description** |
|---|---|
| $\frac{d[P_{gRNA}]}{dt} = -k_{\deg,Pl}[P_{gRNA}]$ | Plasmid for gRNA expression |
| $\frac{d[gRNA]}{dt} = k_{syn,gRNA}[P_{gRNA}] - k_{deg,gRNA}[gRNA] - k_{gRNA,on}[Cas9][gRNA] + k_{gRNA,off}[Cas9:gRNA] + k_{deg,C}[Cas9:gRNA]$ | gRNA turnover and binding to Cas9 |
| $\frac{d[P_{Cas9}]}{dt} = -k_{deg,Pl}[P_{Cas9}]$ | Plasmid for Cas9 expression |
| $\frac{d[mRNA_{Cas9}]}{dt} = k_{syn,mRNA}[P_{Cas9}] - k_{deg,mRNA}[mRNA_{Cas9}]$ | Cas9 mRNA turnover |
| $\frac{d[Cas9]}{dt} = k_{syn,C}[mRNA_{Cas9}] - k_{deg,C}[Cas9] - k_{gRNA,on}[gRNA][Cas9] + k_{gRNA,off}[Cas9:gRNA]$ | Cas9 turnover and binding to gRNA |
| $\frac{d[Cas9:gRNA]}{dt} = k_{gRNA,on}[gRNA][Cas9] - k_{gRNA,off}[Cas9:gRNA] - k_{deg,C}[Cas9:gRNA]$ | Formation and degradation of active Cas9:sgRNA complexes |
| $\frac{d[D]}{dt} = -k_{on,target}[Cas9:gRNA][D] + k_{off,target}[D:g:C]$ | Reversible binding to target gene |
| $\frac{d[D:g:C]}{dt} = k_{on,target}[Cas9:gRNA][D] - k_{off,target}[D:g:C] - k_{editing}[D:g:C]$ | Reversible formation of complexes between target gene, Cas9 and gRNA, gene editing |
| $\frac{d[D_{ed}]}{dt} = k_{editing}[D:g:C]$ | Gene editing |

(continued)

| Model equations | Description |
|---|---|
| $\frac{d[D_{off}]}{dt} = -k_{on,target}[Cas9:gRNA][D_{off}] + k_{off,target}\phi_{OFF\ target}[D_{off}:g:C]$ | Reversible binding to OFF-target |
| $\frac{d[D_{off}:g:C]}{dt} = k_{on,target}[Cas9:gRNA][D_{off}] - k_{off,target}\phi_{OFF\ target}[D_{off}:g:C]$ $-k_{editing}[D_{off}:g:C]$ | Reversible formation of complexes between OFF-target, Cas9 and gRNA, OFF-target editing |
| $\frac{d[D_{ed,OFF}]}{dt} = k_{editing}[D_{off}:g:C]$ | OFF-target editing |

Table 4: Equations of the CascAID gene editing model visualized in Fig. 3A.

| **Model equations, turnover model** | **Description** |
|---|---|
| $\frac{d[P_C]}{dt} = -k_{deg,Pl}[P_C]$ | Plasmid for Cas9/CascAID expression |
| $\frac{d[P_{Cas9+Acr}]}{dt} = -k_{deg,Pl}[P_{Cas9+Acr}]$ | Plasmids for co-expression of Cas9 and Acr |
| $\frac{d[mRNA_C]}{dt} = k_{syn,mRNA}[P_C] - k_{deg,mRNA}[mRNA_C]$ | Cas9/CascAID mRNA turnover |
| $\frac{d[mRNA_{Cas9+Acr}]}{dt} = k_{syn,mRNA}[P_{Cas9+Acr}] - k_{deg,mRNA}[mRNA_{Cas9+Acr}]$ | mRNAs for co-expression of Cas9 and Acr |
| $\frac{d[Cas9]}{dt} = k_{syn,C}[mRNA_C] - k_{deg,C}[Cas9]$ | Cas9 turnover |
| $\frac{d[Cas9_{CA}]}{dt} = k_{syn,C}[mRNA_{Cas9+Acr}] - k_{deg,C}[Cas9_{CA}]$ | Cas9 turnover, co-expression with Acr |
| $\frac{d[Acr_{CA}]}{dt} = k_{syn,Acr}[mRNA_{Cas9+Acr}] - k_{deg,Acr}[Acr_{CA}]$ | Cas9 turnover, co-expression with Acr |
| **Model equations, Cas9 model**<br>*i* = 1...4 for genes AAVS1, EMX1, RUNX1 and HEK | **Description** |
| $\frac{d[P_{gRNA}]}{dt} = -k_{\deg,Pl}[P_{gRNA}]$ | Plasmid for gRNA expression |
| $\frac{d[gRNA_i]}{dt} = k_{syn,gRNA}[P_{gRNA}] - k_{deg,gRNA}[gRNA_i] - k_{gRNA,on}[Cas9][gRNA_i]$ $+ k_{gRNA,off,i}[Cas9:gRNA_i] + k_{deg,C}[Cas9:gRNA_i]$ | gRNA turnover and binding to Cas9 |

(continued)

| **Model equations, Cas9 model**<br>*i* = 1...4 for genes AAVS1, EMX1, RUNX1 and HEK | **Description** |
|---|---|
| $\frac{d[P_C]}{dt} = -k_{deg,Pl}[P_C]$ | Plasmid for Cas9/CascAID expression |
| $\frac{d[mRNA_C]}{dt} = k_{syn,mRNA}[P_C] - k_{deg,mRNA}[mRNA_C]$ | Cas9/CascAID mRNA turnover |
| $\frac{d[Cas9]}{dt} = k_{syn,C}[mRNA_C] - k_{deg,C}[Cas9] - k_{gRNA,on}[gRNA_i][Cas9] + k_{gRNA,off,i}[Cas9 : gRNA_i]$ | Cas9 turnover and binding to gRNAs |
| $\frac{d[Cas9 : gRNA_i]}{dt} = k_{gRNA,on}[gRNA_i][Cas9] - k_{gRNA,off,i}[Cas9 : gRNA_i] - k_{deg,C}[Cas9 : gRNA_i]$ | Formation and degradation of active Cas9:sgRNA complexes |
| $\frac{d[D_i]}{dt} = -k_{on,target}[Cas9 : gRNA_i][D_i] + k_{off,target,i}[D_i : g_i : C]$ | Reversible binding to target gene |
| $\frac{d[D_i : g_i : C]}{dt} = k_{on,target}[Cas9 : gRNA_i][D_i] - k_{off,target,i}[D_i : g_i : C] - k_{editing}[D_i : g_i : C]$ | Reversible formation of complexes between target genes, Cas9 and gRNAs, gene editing |
| $\frac{d[D_{ed,i}]}{dt} = k_{editing}[D_i : g_i : C]$ | Gene editing |
| $\frac{d[D_{off,i}]}{dt} = -k_{on,target}[Cas9 : gRNA_i][D_{off,i}] + k_{off,target,i}\phi_{OFF\ target,i}[D_{off,i} : g_i : C]$ | Reversible binding to OFF-target |

(continued)

| **Model equations, Cas9 model**<br>*i* = 1...4 for genes AAVS1, EMX1, RUNX1 and HEK | **Description** |
|---|---|
| $$\frac{d[D_{off,i}:g_i:C]}{dt}=k_{on,target}[Cas9:gRNA_i][D_{off,i}]-k_{off,target,i}\phi_{OFF\,target,i}[D_{off,i}:g_i:C]-k_{editing}[D_{off,i}:g_i:C]$$ | Reversible formation of complexes between OFF-target, Cas9 and gRNA, OFF-target editing |
| $$\frac{d[D_{ed,OFF,i}]}{dt}=k_{editing}[D_{off,i}:g_i:C]$$ | OFF-target editing |
| **Model equations, CascAID model**<br>*i* = 1...4 for genes AAVS1, EMX1, RUNX1 and HEK; *j* = 1...4 for variants CascAID wt, Ins. 5, N39A, and D14A/G38A | Description |
| $$\frac{d[P_{gRNA}]}{dt}=-k_{\deg,Pl}[P_{gRNA}]$$ | Plasmid for gRNA expression |
| $$\frac{d[gRNA_i]}{dt}=k_{syn,gRNA}[P_{gRNA}]-k_{deg,gRNA}[gRNA_i]-k_{gRNA,on}[CascAID_j][gRNA_i]-k_{gRNA,on}[CascAID_{inh,j}][gRNA_i]+k_{gRNA,off,i}[CascAID_j:gRNA_i]+k_{gRNA,off,i}[CascAID_{inh,j}:gRNA_i]+k_{deg,C}[CascAID_j:gRNA_i]+k_{deg,C}[CascAID_j:gRNA_i]$$ | gRNA turnover and binding to CascAID variants |
| $$\frac{d[P_C]}{dt}=-k_{deg,Pl}[P_C]$$ | Plasmid for Cas9/CascAID expression |
| $$\frac{d[mRNA_C]}{dt}=k_{syn,mRNA}[P_C]-k_{deg,mRNA}[mRNA_C]$$ | Cas9/CascAID mRNA turnover |

(continued)

| **Model equations, CascAID model**<br>$i$ = 1...4 for genes AAVS1, EMX1, RUNX1 and HEK; $j$ = 1...4 for variants CascAID wt, Ins. 5, N39A, and D14A/G38A | Description |
|---|---|
| $\frac{d[CascAID_j]}{dt} = k_{syn,C}[mRNA_C] - k_{deg,C}[CascAID_j] - k_{inh,CascAID}\gamma_j[CascAID_j] + k_{act}[CascAID_{inh,j}] - k_{gRNA,on}[gRNA_i][CascAID_j] + k_{gRNA,off,i}[CascAID_j : gRNA_i]$ | CascAID turnover, transition between inactive and active states ($\gamma_1 \equiv 1$ for CascAID wt), formation of active CascAID:sgRNA complexes |
| $\frac{d[CascAID_{inh,j}]}{dt} = k_{inh,CascAID}\gamma_j[CascAID_j] - k_{act}[CascAID_{inh,j}] - k_{gRNA,on}[gRNA_i][CascAID_{inh,j}] + k_{gRNA,off,i}[CascAID_{inh,j} : gRNA_i] - k_{deg,C}[CascAID_{inh,j}]$ | Transition between inactive and active states, binding of inactive CascAID variants to gRNAs, degradation |
| $\frac{d[CascAID_j : gRNA_i]}{dt} = -k_{inh,CascAID}\gamma_j[CascAID_j : gRNA_i] + k_{act}[CascAID_{inh,j} : gRNA_i] + k_{gRNA,on}[gRNA_i][CascAID_j] - k_{gRNA,off,i}[CascAID_j : gRNA_i] - k_{deg,C}[CascAID_j : gRNA_i]$ | Transition between inactive and active states, formation of active CascAID:sgRNA complexes, degradation |
| $\frac{d[CascAID_{inh,j} : gRNA_i]}{dt} = k_{inh,CascAID}\gamma_j[CascAID_j : gRNA_i] - k_{act}[CascAID_{inh,j} : gRNA_i] + k_{gRNA,on}[gRNA_i][CascAID_{inh,j}] - k_{gRNA,off,i}[CascAID_{inh,j} : gRNA_i] - k_{deg,C}[CascAID_{inh,j} : gRNA_i]$ | Transition between inactive and active states, binding of inactive CascAID variants to gRNAs, degradation |
| $\frac{d[D_i]}{dt} = -k_{on,target}[CascAID_j : gRNA_i][D_i] + k_{off,target,i}[D_i : g_i : C_j]$ | Reversible binding to target gene |
| $\frac{d[D_i : g_i : C_j]}{dt} = k_{on,target}[CascAID_j : gRNA_i][D_i] - k_{off,target,i}[D_i : g_i : C_j] - k_{editing}[D_i : g_i :$ | Reversible formation of complexes between target genes, CascAID variants and gRNAs, gene editing |

(continued)

| **Model equations, CascAID model** <br> *i* = 1...4 for genes AAVS1, EMX1, RUNX1 and HEK; *j* = 1...4 for variants CascAID wt, Ins. 5, N39A, and D14A/G38A | Description |
|---|---|
| $\frac{d[D_{ed,i}]}{dt} = k_{editing}[D_i : g_i : C_j]$ | Gene editing |
| $\frac{d[D_{off,i}]}{dt} = -k_{on,target}[CascAID_j : gRNA_i][D_{off,i}] + k_{off,target,i}\phi_{OFF\ target,i}[D_{off,i} : g_i : C_j]$ | Reversible binding to OFF-target |
| $\frac{d[D_{off,i} : g_i : C_j]}{dt} = k_{on,target}[C_j : gRNA_i][D_{off,i}] - k_{off,target,i}\phi_{OFF\ target,i}[D_{off,i} : g_i : C_j]$ $-k_{editing}[D_{off,i} : g_i : C]$ | Reversible formation of complexes between OFF-target, CascAID variants and gRNAs, OFF-target editing |
| $\frac{d[D_{ed,OFF,i}]}{dt} = k_{editing}[D_{off,i} : g_i : C_j]$ | OFF-target editing |

Table 5: Parameter estimates and confidence intervals.

| Parameter | Unit | Best fit value | Confidence interval obtained from profile likelihood estimation | | identifiable | Allowed parameter interval | |
|---|---|---|---|---|---|---|---|
| | | | Lower bound | Upper bound | | Lower bound | Upper bound |
| $k_{deg,PI}$ | $h^{-1}$ | 0.0317 | 0.0209 | 0.0494 | yes | $10^{-5}$ | $10^5$ |
| $k_{syn,mRNA}$ | $h^{-1}$ | 0.0343 | 0 | Inf | no | $10^{-5}$ | $10^5$ |
| $k_{deg,mRNA}$ | $h^{-1}$ | 0.0517 | 0.0224 | 0.0941 | yes | $10^{-5}$ | $10^5$ |
| $k_{syn,C}$ | $h^{-1}$ | 282 | 0 | Inf | no | $10^{-5}$ | $10^5$ |
| $k_{deg,C}$ | $h^{-1}$ | 0.0501 | 0.0235 | 0.103 | yes | $10^{-5}$ | $10^5$ |
| $k_{syn,Acr}$ | $h^{-1}$ | 398 | 0.216 | Inf | no | $10^{-5}$ | $10^5$ |
| $k_{deg,Acr}$ | $h^{-1}$ | 0.0381 | 0.0184 | 0.0825 | yes | $10^{-5}$ | $10^5$ |
| $[P_C]_0$ | unitless | 2.81 | 2.36 | 3.43 | yes | $10^{-5}$ | $10^5$ |
| $[D_1]_0$ | unitless | 0.488 | 0.475 | 0.509 | yes | 0.1 | 1 |
| $[D_2]_0$ | unitless | 0.178 | 0.16 | 0.201 | yes | 0.1 | 1 |
| $[D_3]_0$ | unitless | 0.275 | 0.251 | 0.299 | yes | 0.1 | 1 |
| $[D_4]_0$ | unitless | 0.231 | 0.211 | 0.254 | yes | 0.1 | 1 |
| $k_{deg,gRNA}$ | $h^{-1}$ | 99995 | 66980 | Inf | no | $10^{-5}$ | $10^5$ |
| $K_{d,gRNA,1} = K_{gRNA,off,1}/k_{gRNA,on}$ | $h^{-1}$ | 0.00394 | 0.00157 | 0.00907 | yes | $10^{-5}$ | $10^5$ |
| $K_{d,gRNA,2} = k_{gRNA,off,2}/k_{gRNA,on}$ | $h^{-1}$ | 0.0100 | 0.000324 | 0.0282 | yes | $10^{-5}$ | $10^5$ |
| $K_{d,gRNA,3} = k_{gRNA,off,3}/k_{gRNA,on}$ | $h^{-1}$ | 0.0208 | 0 | 0.0484 | no | $10^{-5}$ | $10^5$ |
| $K_{d,gRNA,4} = k_{gRNA,off,4}/k_{gRNA,on}$ | $h^{-1}$ | 0.00132 | 0 | 0.0105 | no | $10^{-5}$ | $10^5$ |
| $k_{on,target}$ | $h^{-1}$ | 11.9 | 0 | Inf | no | $10^{-5}$ | $10^5$ |
| $k_{off,target,1}$ | $h^{-1}$ | $9.7 \cdot 10^{-5}$ | $6.25 \cdot 10^{-5}$ | 0.000148 | yes | $10^{-5}$ | $10^5$ |
| $\phi_{OFF\ target,1}$ | unitless | 87204 | 610 | Inf | no | $10^{-5}$ | $10^5$ |
| $k_{off,target,2}$ | $h^{-1}$ | 0.0381 | $5.85 \cdot 10^{-5}$ | 2.16 | yes | $10^{-5}$ | $10^5$ |
| $\phi_{OFF\ target,2}$ | unitless | 44.0 | 6.19 | 388 | yes | $10^{-5}$ | $10^5$ |
| $k_{off,target,3}$ | $h^{-1}$ | $6.39 \cdot 10^{5}$ | $1.19 \cdot 10^{-5}$ | Inf | no | $10^{-5}$ | $10^5$ |
| $\phi_{OFF\ target,3}$ | unitless | 27884 | 10.7 | Inf | no | $10^{-5}$ | $10^5$ |
| $k_{off,target,4}$ | $h^{-1}$ | 0.402 | $2.15 \cdot 10^{-5}$ | 1.24 | yes | $10^{-5}$ | $10^5$ |
| $\phi_{OFF\ target,4}$ | unitless | 33.5 | 20.6 | Inf | no | $10^{-5}$ | $10^5$ |
| $k_{editing}$ | $h^{-1}$ | 0.144 | 0.116 | 0.179 | yes | $10^{-5}$ | $10^5$ |
| $k_{inh,CascAID}$ | $h^{-1}$ | 618 | 115 | Inf | no | $10^{-5}$ | $10^5$ |
| $k_{act}$ | $h^{-1}$ | 1.13 | 0.213 | 190 | yes | $10^{-5}$ | $10^5$ |

(continued)

| Parameter | Unit | Best fit value | Confidence interval obtained from profile likelihood estimation | | identifiable | Allowed parameter interval | |
|---|---|---|---|---|---|---|---|
| | | | Lower bound | Upper bound | | Lower bound | Upper bound |
| $\gamma_2$ | unitless | 0.058 | 0.0452 | 0.0726 | yes | $10^{-5}$ | $10^{5}$ |
| $\gamma_3$ | unitless | 0.0609 | 0.0471 | 0.0762 | yes | $10^{-5}$ | $10^{5}$ |
| $\gamma_4$ | unitless | 0.0519 | 0.0404 | 0.0622 | yes | $10^{-5}$ | $10^{5}$ |

## Claims

1. A polypeptide or polypeptide complex comprising (i) a Cas nuclease and (ii) a low-affinity Cas inhibitor.

2. The polypeptide of claim 1, wherein said Cas nuclease and said low-affinity Cas inhibitor are comprised as a fusion polypeptide.

3. The polypeptide of claim 2, wherein said fusion polypeptide comprises said Cas nuclease and said low-affinity Cas inhibitor connected via a linker peptide.

4. The polypeptide of claim 3, wherein said linker peptide is a GS and/or GG-comprising linker, preferably a GGSG (SEQ ID NO:13)-comprising linker, more preferably a $(GGSG)_n$ linker, with n being an integer of from 1 to 100, preferably of from 2 to 50, more preferably of from 3 to 25, even more preferably being about 10, most preferably being 10.

5. The polypeptide of any one of claims 1 to 4, wherein said Cas nuclease is a Cas9 endonuclease.

6. The polypeptide of any one of claims 1 to 5, wherein said Cas inhibitor is an Acr polypeptide, preferably is an AcrIIA4 polypeptide.

7. The polypeptide of any one of claims 1 to 6,, wherein said low-affinity Cas inhibitor is a mutein of the AcrIIA4 polypeptide comprising at least one mutation selected from the list consisting of (i) M77A; (ii) D76A/M77A; (iii); (iv) D14A; (v) D14A/Y15A; (vi) D14A/G38A; (vii) G38A; (viii) D37A/G38A; (ix) D14A/G38A, (x) a deletion of amino acids N64/Q65/E66 and an insertion of a LOV-domain; (xi) a deletion of amino acids N64/Q65/E66; (xii) an insertion of a LOV-domain comprising T406A, T407A, G528A, and N538E mutations; and (xiii) any combination of (i) to (xii).

8. The polypeptide of any one of claims 1 to 7, wherein the ratio of an editing frequency of the target site to an editing frequency of any off-target site of said polypeptide is at least 2, preferably at least 5, more preferably at least 7, still more preferably at least 10, still more preferably at least 15, even more preferably at least 18, most preferably at least 20.

**9.** The polypeptide of any one of claims 1 to 8, wherein said low-affinity Cas inhibitor has an affinity to said Cas nuclease of from 0.01% to 90%, preferably of from 0.1 to 50%, more preferably of from 0.5% to 25%, still more preferably of from 1% to 10%, of the corresponding Cas inhibitor.

**10.** A composition comprising or causing expression in a host cell of a Cas nuclease and (i) a low-affinity Cas inhibitor and/or (ii) a sub-inhibitory concentration of a Cas inhibitor.

**11.** A polypeptide or polypeptide complex according to any one of claims 1 to 9 or a composition according to claim 10 for use in medicine, in particular for use in treating and/or preventing genetic disease, neurodegenerative disease, cancer, and/or infectious disease.

**12.** A method for improving specificity of a Cas nuclease, comprising

a) providing a Cas nuclease; and
b) contacting said Cas nuclease with a low-affinity Cas inhibitor or a sub-inhibitory concentration of a Cas inhibitor; and
c) thereby improving specificity of said Cas enzyme.

**13.** Use of (i) a polypeptide or polypeptide complex according to claim 1 or 9 or (ii) a composition according to claim 10 for modifying a target site in a host cell.

**14.** A method for modifying a target site in a host cell comprising contacting said host cell with a Cas nuclease, wherein said host cell is further contacted with a low-affinity Cas inhibitor and/or a sub-inhibitory concentration of a Cas inhibitor.

**15.** A method for identifying a low-affinity Cas nuclease inhibitor comprising

a) providing a representation of editing efficiency in dependence of Cas inhibitor strength for at least one target site and for at least one off-target site;
b) determining editing efficiency in the presence of at least one candidate low-affinity Cas inhibitor for said least one target site and for said at least one off-target site;
c) comparing the editing efficiency obtained in step b) to the representation obtained in step a); and, thereby,
d) identifying a low-affinity Cas nuclease inhibitor.

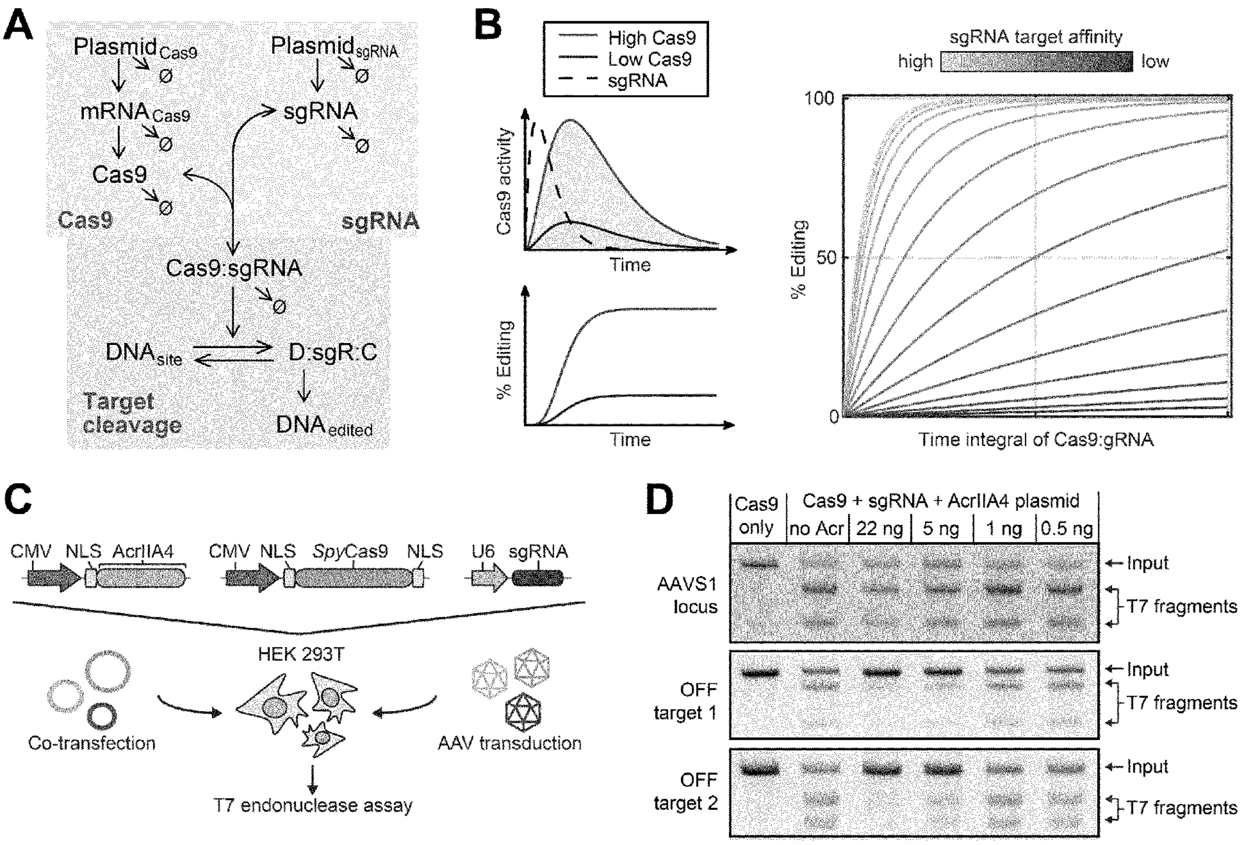
A
Plasmid Cas9
Plasmid sgRNA
mRNA Cas9
sgRNA
Cas9
Cas9
sgRNA
Cas9:sgRNA
DNA site
D:sgR:C
Target cleavage
DNA edited
B
High Cas9
Low Cas9
sgRNA
Cas9 activity
Time
% Editing
Time
sgRNA target affinity
high
low
100
50
0
% Editing
Time integral of Cas9:gRNA
C
CMV NLS AcrIIA4
CMV NLS SpyCas9 NLS
U6 sgRNA
HEK 293T
Co-transfection
AAV transduction
T7 endonuclease assay
D
Cas9 only
Cas9 + sgRNA + AcrIIA4 plasmid
no Acr
22 ng
5 ng
1 ng
0.5 ng
AAVS1 locus
OFF target 1
OFF target 2
Input
T7 fragments

Fig. 1

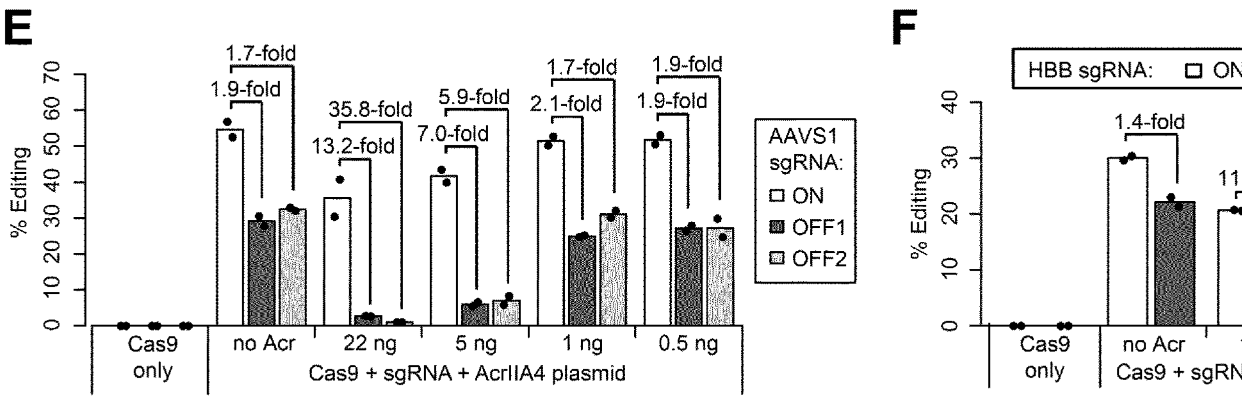


Fig. 1 (continued)

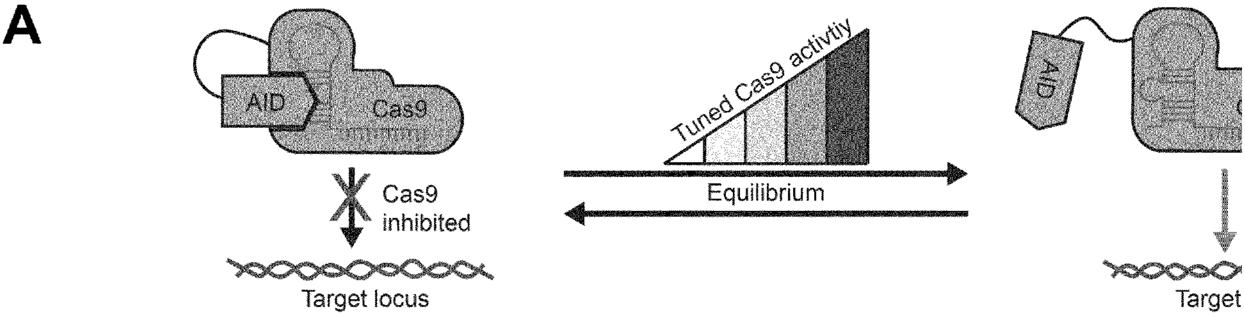


Fig. 2

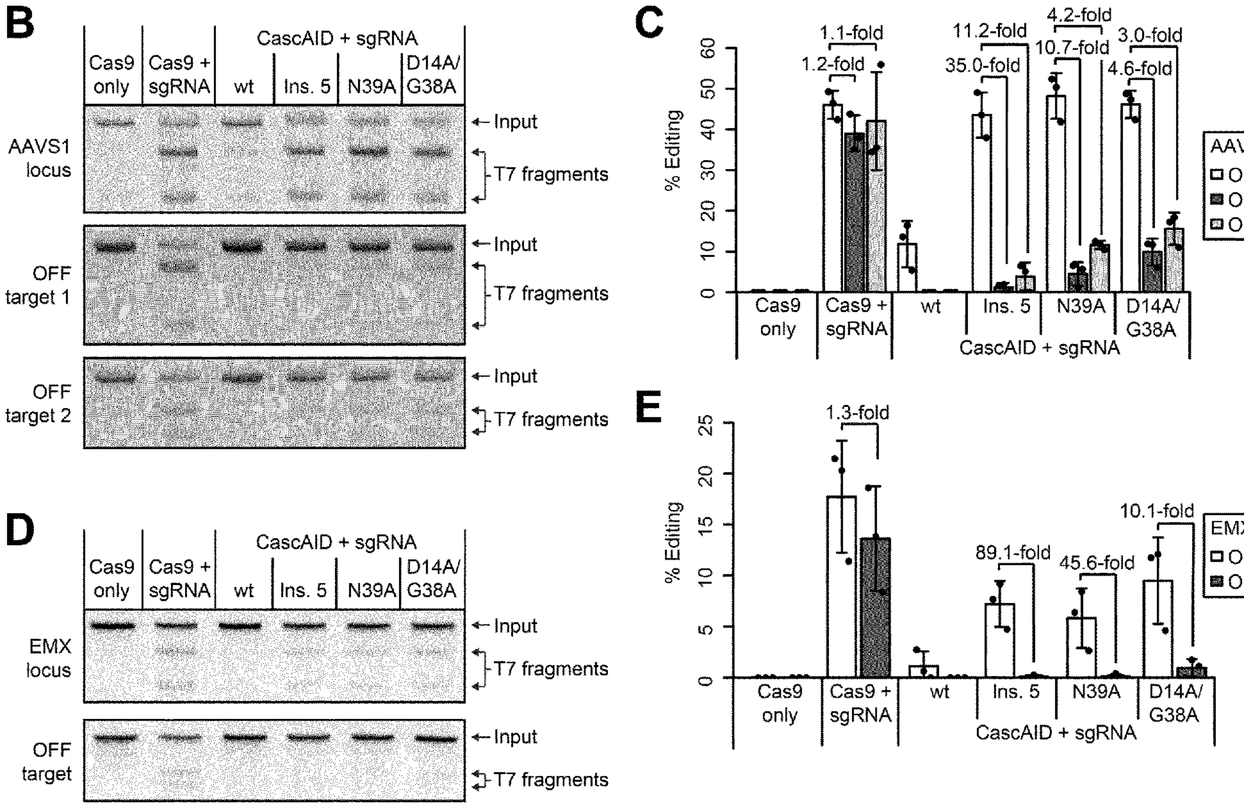


Fig. 2 (continued)

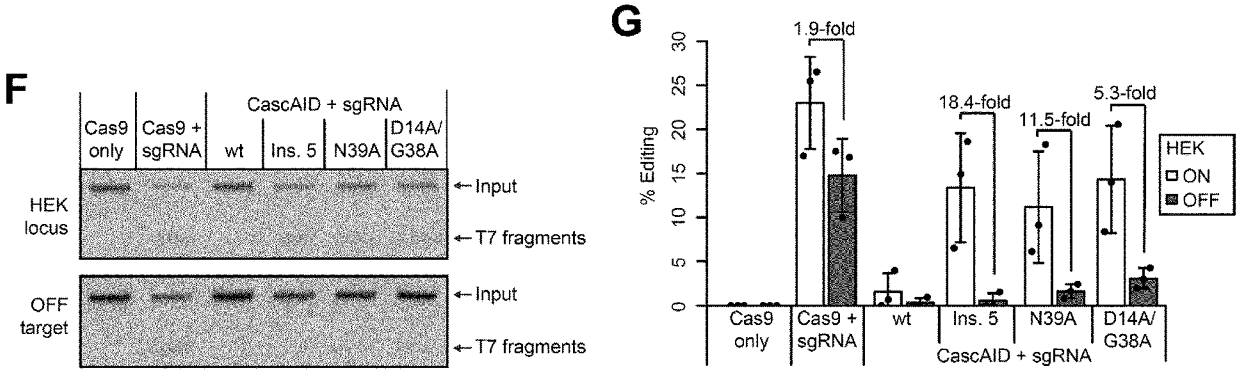


Fig. 2 (continued)

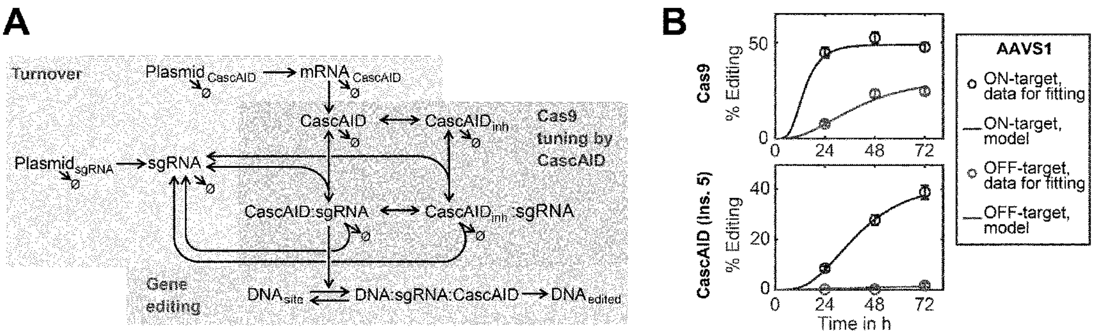


Fig. 3

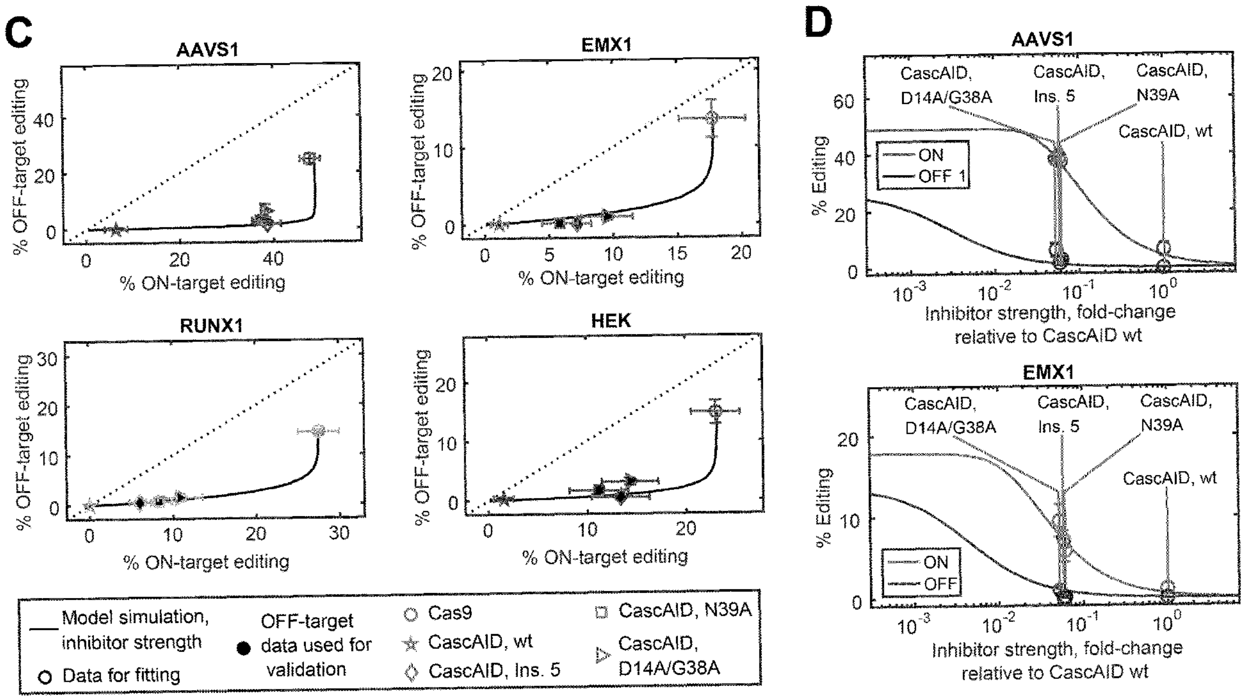


Fig. 3 (continued)

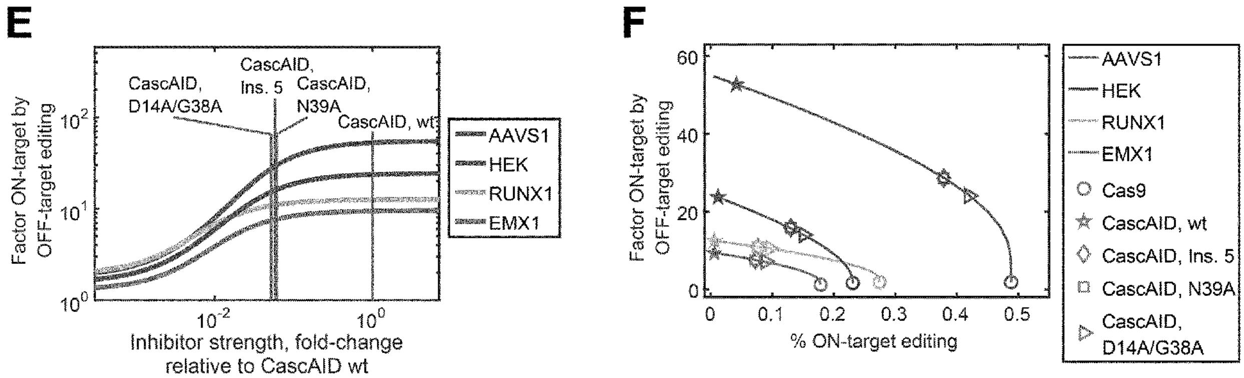


Fig. 3 (continued)

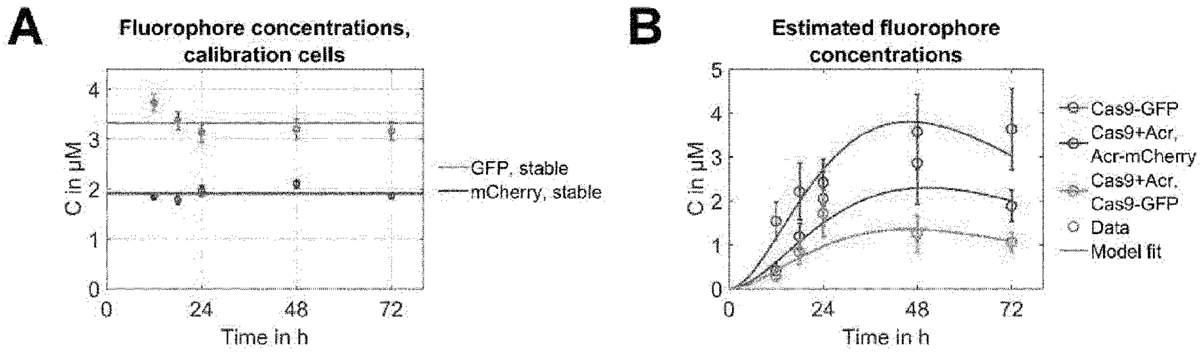


Fig. 4

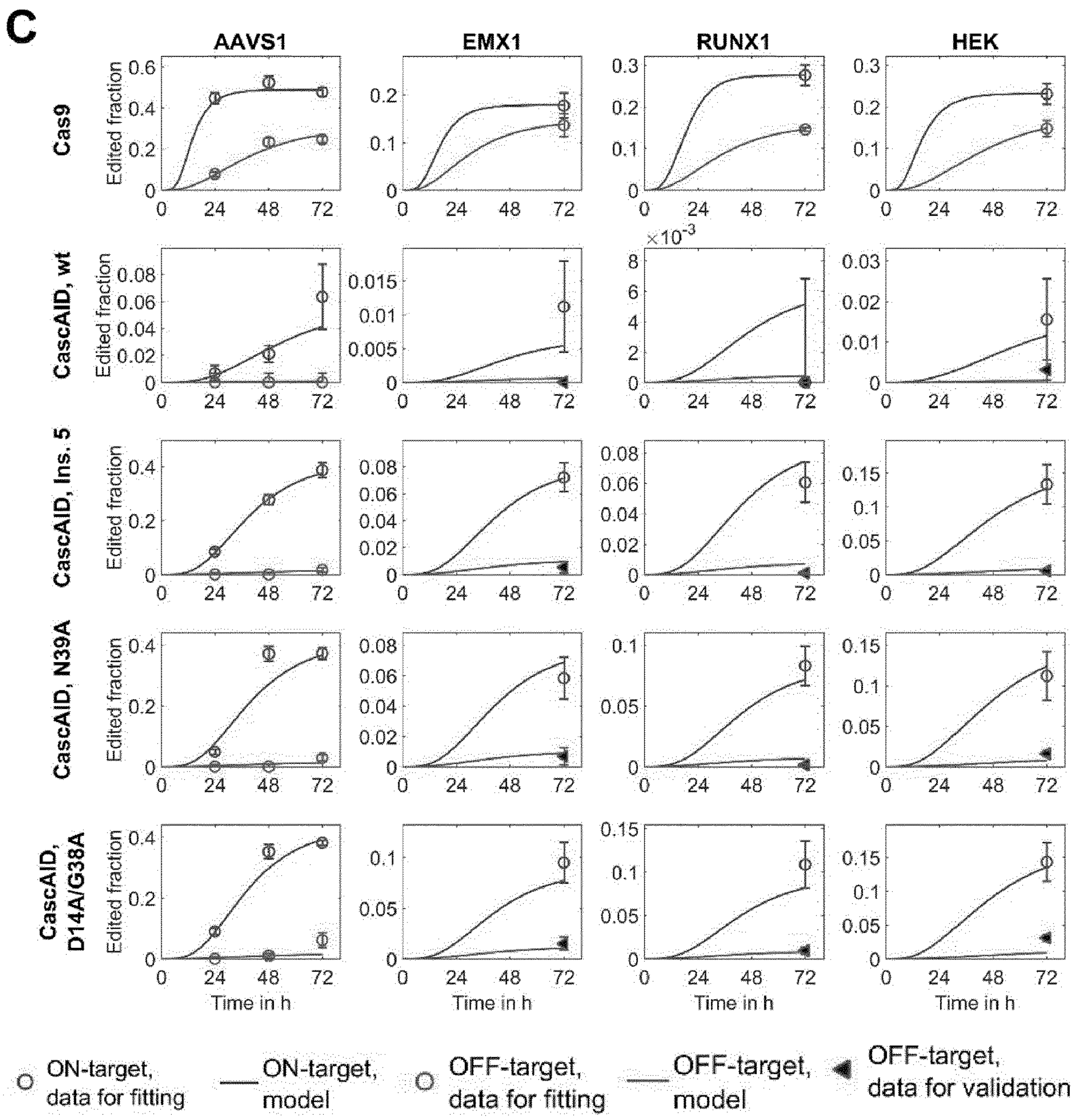


Fig. 4 (continued)

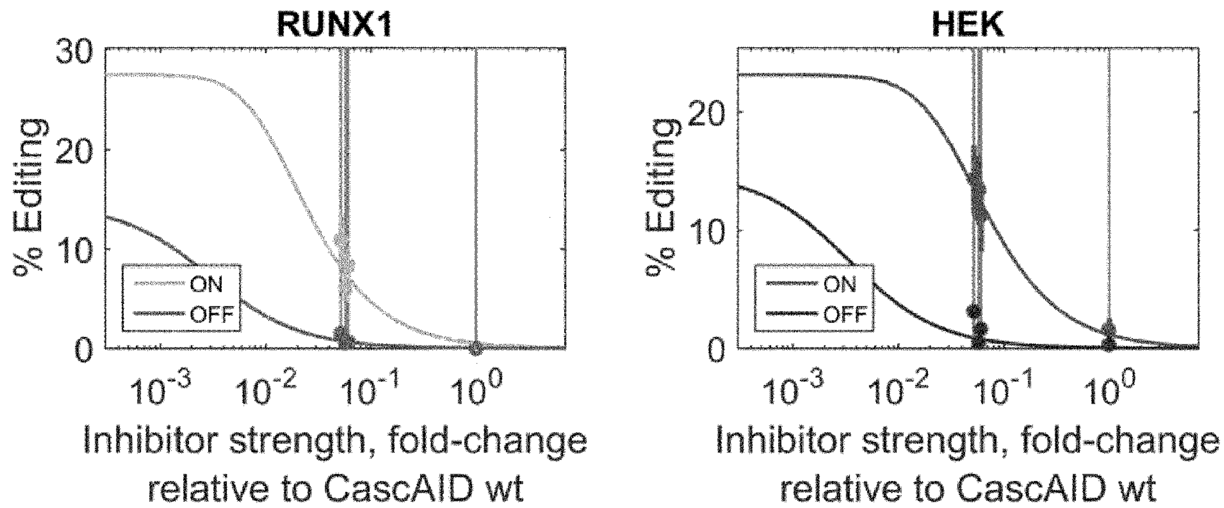
RUNX1
30
20
10
0
% Editing
ON
OFF
$10^{-3}$
$10^{-2}$
$10^{-1}$
$10^{0}$
Inhibitor strength, fold-change relative to CascAID wt
HEK
20
10
0
% Editing
ON
OFF
$10^{-3}$
$10^{-2}$
$10^{-1}$
$10^{0}$
Inhibitor strength, fold-change relative to CascAID wt

Fig. 5

Europäisches Patentamt
European Patent Office
Office européen des brevets

# EUROPEAN SEARCH REPORT

Application Number
EP 19 18 6590

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JIYUNG SHIN ET AL: "Disabling Cas9 by an anti-CRISPR DNA mimic", SCIENCE, vol. 3, no. 7, 12 July 2017 (2017-07-12), page e1701620, XP055444909, ISSN: 0036-8075, DOI: 10.1126/sciadv.1701620 | 1,5,6, 10-14 | INV. C12N9/22 C07K14/005 C12N15/63 A61K38/46 |
| A | * abstract * * page 3, right-hand column, paragraph 2 - page 4, right-hand column, paragraph 1; figure 2 * ----- | 2-4,7-9, 15 | |
| X | ERIANNA M. BASGALL ET AL: "Gene drive inhibition by the anti-CRISPR proteins AcrIIA2 and AcrIIA4 in Saccharomyces cerevisiae", JOURNAL OF GENERAL MICROBIOLOGY, vol. 164, no. 4, 1 April 2018 (2018-04-01), pages 464-474, XP055646561, ISSN: 1350-0872, DOI: 10.1099/mic.0.000635 | 1,5-7,9, 10,13-15 | |
| A | * page 468; figure 3 * * page 470; figure 5 * ----- | 2-4,8, 11,12 | TECHNICAL FIELDS SEARCHED (IPC) |
| X | WO 2019/067011 A1 (KANSAS STATE UNIV RESEARCH FOUNDATION [US]) 4 April 2019 (2019-04-04) | 1,5-7,9, 10,13-15 | C12N C07K C40B A61K |
| A | * figures 22,23 * ----- | 2-4,8, 11,12 | |
| X | WO 2019/076651 A1 (UNIV HEIDELBERG [DE]; DEUTSCHES KREBSFORSCH [DE]) 25 April 2019 (2019-04-25) * figure 2 * * figure 4 * * claims 3,4 * ----- -/-- | 1,5,6, 10,13,14 | |

The present search report has been drawn up for all claims

1

| Place of search | Date of completion of the search | Examiner |
|---|---|---|
| Munich | 28 November 2019 | Niebuhr-Ebel, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 19 18 6590

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BUBECK FELIX ET AL: "Engineered anti-CRISPR proteins for optogenetic control of CRISPR-Cas9", NATURE METHODS, NATURE PUB. GROUP, NEW YORK, vol. 15, no. 11, 30 October 2018 (2018-10-30), pages 924-927, XP036624660, ISSN: 1548-7091, DOI: 10.1038/S41592-018-0178-9 [retrieved on 2018-10-30] * abstract * * page 925; figure 1 * ----- | 1,5,6, 10,13,14 | |
| | | | TECHNICAL FIELDS SEARCHED (IPC) |

The present search report has been drawn up for all claims

1

| Place of search | Date of completion of the search | Examiner |
|---|---|---|
| Munich | 28 November 2019 | Niebuhr-Ebel, K |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
........................................
& : member of the same patent family, corresponding document

page 2 of 2

## ANNEX TO THE EUROPEAN SEARCH REPORT ON EUROPEAN PATENT APPLICATION NO.

EP 19 18 6590

This annex lists the patent family members relating to the patent documents cited in the above-mentioned European search report. The members are as contained in the European Patent Office EDP file on
The European Patent Office is in no way liable for these particulars which are merely given for the purpose of information.

28-11-2019

| Patent document cited in search report | Publication date | Patent family member(s) | Publication date |
|---|---|---|---|
| WO 2019067011 A1 | 04-04-2019 | NONE | |
| WO 2019076651 A1 | 25-04-2019 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **KULCSAR et al.** *Genome Biol,* 2017, vol. 18, 190
- **KLEINSTIVER et al.** *Nature,* 2016, vol. 529, 490-495
- **AKCAKAYA et al.** *Nature,* 2018, vol. 561, 416-419
- **DONG et al.** *Nature,* 2017, vol. 546, 436-439
- **SHIN et al.** *Sci Adv,* 2017, vol. 3, e1701620
- **PAWLUK et al.** *Cell,* 2016, vol. 167 (7), 1829
- **RAUCH et al.** *Cell,* 2017, vol. 168 (1-2), 150
- Current Protocols in Molecular Biology. John Wiley & Sons, 1989, 6.3.1-6.3.6
- **PILEUP.** *J. Mol. Evolution.,* 1987, vol. 25, 351-360
- **HIGGINS et al.** *CABIOS,* 1989, vol. 5, 151-153
- **GAP ; BESTFIT ; NEEDLEMAN ; WUNSCH.** *J. Mol. Biol.,* 1970, vol. 48, 443-453
- **SMITH ; WATERMAN.** *Adv. Appl. Math.,* 1981, vol. 2, 482-489
- Remington's Pharmaceutical Sciences. Mack Publishing Company
- **BUBECK et al.** *Nat Methods,* 2018, vol. 15, 924-927
- **HOFFMANN et al.** *Nucleic Acids Res,* 2019, vol. 271
- **KALLENBERGER et al.** *Sci Signal,* 2014, vol. 7, ra23
- **MA et al.** *J Cell Biol,* 2016, vol. 214, 529-537
- **MAIWALD et al.** *Bioinformatics,* 2008, vol. 24, 2037-2043
- **GRIMM et al.** *J Virol,* 2008, vol. 82 (12), 5887
- **BASGALL et al.** *Microbiology,* 2018, vol. 164, 464-474
- **AKCAKAYAET.** *Nature,* 2018, vol. 561, 416-419